# EUROPEAN PATENT APPLICATION

(11) **EP 4 431 151 A1**
(43) Date of publication of application: **18.09.2024**
(21) Application number: 22892890.9
(22) Date of filing: 11.11.2022
(51) Int. Cl.: A61P 31/14, C07D 403/12, C07D 405/12, C07D 239/94, C07D 239/95, A61K 31/517

(54) **ANTI-SARS-COV-2 DRUG**

(30) Priority: 12.11.2021 JP 2021184512
(71) Applicant: Kagoshima University, Kagoshima-shi, Kagoshima 890-8580 (JP); Tokyo University of Science Foundation, Tokyo 162-8601 (JP)
(72) Inventor: BABA Masanori, Kagoshima-shi, Kagoshima 890-8580 (JP); OKAMOTO Mika, Kagoshima-shi, Kagoshima 890-8580 (JP); TOYAMA Masaaki, Kagoshima-shi, Kagoshima 890-8580 (JP); AOKI Shin, Tokyo 162-8601 (JP); TANAKA Tomohiro, Tokyo 162-8601 (JP); YOKOI Kenta, Tokyo 162-8601 (JP)
(74) Representative: J A Kemp LLP
(86) International application number: PCT/JP2022/042029
(87) International publication number: WO 2023/085392

(57) **Abstract**

The present invention relates to a compound represented by formula (I), a salt thereof, a solvate thereof, or a prodrug thereof, and an anti-SARS-CoV-2 drug comprising the same: 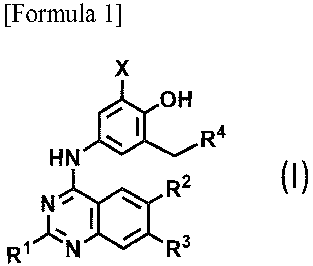 wherein R¹, R², and R³ are the same or different, and each of them is a hydrogen atom, a halogen atom, a substituted or unsubstituted C₁₋₆-alkyl group, a substituted or unsubstituted C₁₋₆-alkoxy group, or -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
R⁴ is -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
X is a hydrogen atom, a fluorine atom, a chlorine atom, a bromine atom, or an iodine atom, and
the hydroxyl group in the formula may be substituted with a protecting group.

## Description

### Technical Field

The present invention relates to an anti-SARS-CoV-2 drug.

### Background Art

Coronaviruses are viruses that essentially cause cold symptoms in humans. Four types of coronaviruses are known, and these viruses cause 10 to 15% of cases of cold. In addition, coronaviruses causing severe acute respiratory syndrome (SARS) and Middle East respiratory syndrome (MERS) which are highly fatal are known so far. The number of SARS patients is about 8,000 with a fatality rate of about 10%. The number of MERS patients is about 2,500 with a fatality rate of about 35%.

The coronaviruses are positive-stranded RNA viruses having an envelope of about 100 nm in diameter. SARS-CoV is classified as a Class II pathogen, and MERS-CoV is classified as a Class III pathogen.

A variety of antibody drugs targeting spike proteins on the surface of SARS-CoV-2 and Remdesivir diverted from an anti-Ebola drug are currently used as curative medicines. Besides, Molnupiravir developed as an anti-Influenza drug and newly developed Paxlovid (Paxlovid) have been approved as anti-SARS-CoV-2 drugs which can be orally administrated. Molnupiravir has an action mechanism of inhibiting the RNA-dependent RNA polymerase enzyme of the virus and causing errors during virus RNA replication. Paxlovid is a combination drug of Nirmatrelvir and Ritonavir where Nirmatrelvir is a low-molecular-weight compound which inhibits the function of the main protease needed for virus replication and Ritonavir functions as a booster for maintaining the blood concentration of Nirmatrelvir. However, these drugs have some deficits, for example, side effects such as teratogenicity and problems on combination use with other drugs. Accordingly, it is very important to identify and develop a novel drug having a selective and strong anti-virus effects against SARS-CoV-2.

On the other hand, Amodiaquine has already been approved for clinical use as an anti-Malaria drug. In addition, it is known that 7-chloro-4-aminoquinoline compounds such as Amodiaquine are effective against Parkinson's disease (Patent Literature 1). Furthermore, the present inventors have found that Amodiaquine or derivatives thereof are effective against severe fever with thrombocytopenia syndrome virus (SFTSV) and Ebola virus, and filed patent applications (Patent Literatures 2 and 3).

However, Patent Literature 1 to Patent Literature 3 have no description suggesting the relation between Amodiaquine or a derivative thereof and anti-SARS-CoV-2 activity thereof.

### Citation List

### Patent Literature

Patent Literature 1: JP Patent Publication (Kohyo) No. 2009-527478 A
Patent Literature 2: WO 2018/135449
Patent Literature 3: WO 2018/191642

### Summary of Invention

### Technical Problem

An object of the present invention is to provide an antiviral drug which is effective against SARS-CoV-2.

### Solution to Problem

To solve the problems above, the present inventors have established an anti-SARS-CoV-2 assay system for drugs and performed screening of various drugs, found selective anti-SARS-CoV-2 effects of Amodiaquine and specific derivatives thereof, and filed a patent application (PCT/JP2021/18602).

The present inventors have further conducted research and found that by replacing the quinoline skeleton of the compound disclosed in PCT/JP2021/18602 with a quinazoline skeleton, toxicity is reduced while anti-virus activity is maintained, thus achieving the present invention.

Specifically, the present invention is summarized as follows.
(1) A compound represented by the following formula (I), a salt thereof, a solvate thereof, or a prodrug thereof 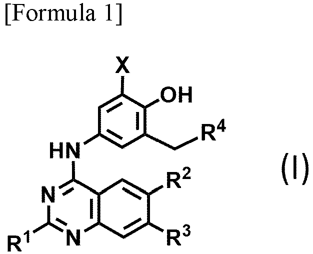 wherein R¹, R², and R³ are the same or different, and each of them is a hydrogen atom, a halogen atom, a substituted or unsubstituted C₁₋₆-alkyl group, a substituted or unsubstituted C₁₋₆-alkoxy group, or -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
   R⁴ is -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
   X is a hydrogen atom, a fluorine atom, a chlorine atom, a bromine atom, or an iodine atom, and the hydroxyl group in the formula may be substituted with a protecting group.
(2) The compound, a salt thereof, a solvate thereof, or a prodrug thereof according to (1) above, wherein in the formula (I), R⁴ is an ethyl(isopropyl)amino group, a diisopropylamino group, a cyclohexyl(ethyl)amino group, an ethyl(pentane-3-yl)amino group, a tert-butyl(ethyl)amino group, or a 3,5-dimethylpiperidino group.
(3) The compound, a salt thereof, a solvate thereof, or a prodrug thereof according to (1) or (2) above, wherein in the formula (I), R¹, R², and R³ are the same or different, and each of them is a hydrogen atom, a halogen atom, a C₁₋₆-alkoxy group, or a dimethylamino group.
(4) The compound, a salt thereof, a solvate thereof, or a prodrug thereof according to any one of (1) to (3) above, wherein in the formula (I), X is a hydrogen atom or a chlorine atom.
(5) An anti-SARS-CoV-2 drug comprising the compound, a salt thereof, a solvate thereof, or a prodrug thereof according to any one of (1) to (4) above.
(6) Use of the compound, a salt thereof, a solvate thereof, or a prodrug thereof according to any one of (1) to (4) above in production of an anti-SARS-CoV-2 drug.

### Advantageous Effect of Invention

The present invention can provide an antiviral drug effective against SARS-CoV-2.

### Brief Description of Drawing

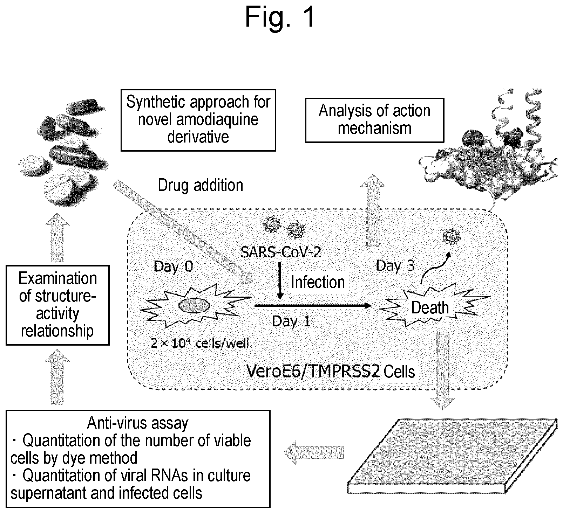

[Figure 1] Figure 1 schematically shows the anti-SARS-CoV-2 assay performed in Examples.

### Description of Embodiments

Hereinafter, the present invention will be described in detail.

Examples of the C₁₋₁₀-alkyl group in the formula (I) include a methyl group, an ethyl group, a propyl group, an isopropyl group, a butyl group, an isobutyl group, a sec-butyl group, a tert-butyl group, a pentyl group, an isopentyl group, a hexyl group, a heptyl group, an octyl group, a nonyl group, a decyl group, a cyclopropyl group, a cyclobutyl group, a cyclopentyl group, a cyclohexyl group, and a 2-cyclopropylethyl group. Examples of the C₁₋₆-alkyl group include a methyl group, an ethyl group, a propyl group, an isopropyl group, a butyl group, an isobutyl group, a sec-butyl group, a tert-butyl group, a pentyl group, an isopentyl group, a hexyl group, a cyclobutyl group, a cyclopentyl group, a cyclohexyl group, and a 2-cyclopropylethyl group.

Examples of the C₁₋₆-alkoxy group include a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopropyloxy group, a cyclobutyloxy group, a cyclopentyloxy group, and a cyclohexyloxy group.

The C₁₋₁₀-alkyl group, the C₁₋₆-alkyl group, and the C₁₋₆-alkoxy group may be substituted with one or more substituents selected from the group consisting of C₁₋₆-alkoxy groups such as a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopropyloxy group, a cyclobutyloxy group, a cyclopentyloxy group, and a cyclohexyloxy group; C₁₋₆-alkoxy-carbonyl groups such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a cyclopropyloxycarbonyl group, a cyclobutyloxycarbonyl group, and a cyclopentyloxycarbonyl group; aromatic hydrocarbon groups such as a phenyl group, a tolyl group, and a naphthyl group; heterocyclic groups such as a pyridyl group; halogen atoms such as a fluorine atom, a chlorine atom, a bromine atom, and an iodine atom; C₁₋₆-aliphatic acyl groups such as a formyl group, an acetyl group, a propionyl group (propanoyl group), a butyryl group (butanoyl group), a valeryl group (pentanoyl group), and a hexanoyl group; aromatic acyl groups (aroyl groups) such as a benzoyl group and a toluoyl group; an aralkyloxy group, a carboxyl group, a hydroxyl group, an amino group, C₁₋₆-alkylamino groups, and di-C₁₋₆-alkylamino groups.

Examples of the aryl group include aromatic hydrocarbon groups such as a phenyl group and a naphthyl group. For example, these may be substituted with one or more substituents selected from the group consisting of C₁₋₆-alkyl groups; C₁₋₆-alkoxy groups such as a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group, a cyclopropyloxy group, a cyclobutyloxy group, a cyclopentyloxy group, and a cyclohexyloxy group; C₁₋₆-alkoxy-carbonyl groups such as a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a cyclopropyloxycarbonyl group, a cyclobutyloxycarbonyl group, and a cyclopentyloxycarbonyl group; aromatic hydrocarbon groups such as a phenyl group, a tolyl group, and a naphthyl group; halogen atoms such as a fluorine atom, a chlorine atom, a bromine atom, and an iodine atom; C₁₋₆-aliphatic acyl groups such as a formyl group, an acetyl group, a propionyl group (propanoyl group), a butyryl group (butanoyl group), a valeryl group (pentanoyl group), and a hexanoyl group; aromatic acyl groups (aroyl groups) such as a benzoyl group and a toluoyl group; an aralkyloxy group, a carboxyl group, an amino group, C₁₋₆-alkylamino groups, and di-C₁₋₆-alkylamino groups.

Examples of 5 to 7-membered ring groups formed by R^{a} and R^{b} together with an adjacent nitrogen atom include a 1-pyrrolidinyl group, a 1-imidazolidinyl group, a 1-pyrazolidinyl group, a morpholino group (4-morpholinyl group), a piperidino group (1-piperidinyl group), a 1-piperazinyl group, a 4-thiamorpholinyl group, a perhydro-1,4-diazepin-1-yl group, a hexahydro-1H-azepin-1-yl group, and a perhydro-1,4-thiazepin-4-yl groups. The 5 to 7-membered ring groups may be substituted with one or more substituents selected from the group consisting of C₁₋₆-alkyl groups, C₂₋₆-alkenyl groups, C₂₋₆-alkynyl groups, aromatic groups, an acyl group, a hydroxyl group, a carboxyl group, a cyano group, halogen atoms (such as a fluorine atom), C₁₋₆-alkoxy groups, an aralkyl group, a nitro group, an amino group, C₁₋₆-alkylamino groups, and di-C₁₋₆-alkylamino groups. The 5 to 7-membered ring groups are preferably 5-membered ring groups or 6-membered ring groups, more preferably substituted or unsubstituted 1-pyrrolidinyl groups (such as a 3-fluoro-1-pyrrolidinyl group), substituted or unsubstituted 1-piperazinyl groups (such as a 4-methyl-1-piperazinyl group), substituted or unsubstituted morpholino groups (4-morpholinyl group), substituted or unsubstituted piperidino groups (1-piperidinyl groups) (such as a 3-fluoropiperidino group and a 3,5-dimethylpiperidino group).

Examples of the halogen atom include a fluorine atom, a chlorine atom, a bromine atom, and an iodine atom.

The hydroxyl group in the formula (I) may be substituted with a protecting group such as a 2-tetrahydropyranyl group (THP), a 3,4,5-trihydroxy-6-methyltetrahydropyran-2-yl group, or a methoxy methyl group.

From the viewpoint of selectivity coefficient (50% toxicity concentration (CC₅₀)/50% effective concentration (EC₅₀)), in the formula (I), preferably, R⁴ is an ethyl (isopropyl)amino group, a diisopropylamino group, a cyclohexyl(ethyl)amino group, an ethyl(pentane-3-yl)amino group, a tert-butyl(ethyl)amino group, or a 3,5-dimethylpiperidino group, R¹, R², and R³ are the same or different, and each of them is a hydrogen atom, a halogen atom (preferably a fluorine atom), a C₁₋₆-alkoxy group (preferably a methoxy group), or a dimethylamino group, and X is a hydrogen atom or a chlorine atom; and their combinations are more preferable.

Since the compound according to the present invention can have an asymmetric carbon, the compound may have optical isomers. The compound according to the present invention may also have tautomers. The compound according to the present invention may be any isolated isomer (such as an R form or an S form), or may be a mixture of two or more isomers including racemates and diastereoisomers in any proportion.

Salts of the compound represented by the formula (I) are preferably pharmaceutically acceptable salts. Examples thereof include salts thereof with inorganic acids such as hydrochloric acid, sulfuric acid, phosphoric acid, hydrobromic acid, hydroiodic acid, nitric acid, pyrosulfuric acid, and metaphosphoric acid; or salts thereof with organic acids such as citric acid, benzoic acid, acetic acid, propionic acid, fumaric acid, maleic acid, and sulfonic acids (such as methanesulfonic acid, p-toluenesulfonic acid, and naphthalenesulfonic acid).

Examples of solvates of the compound represented by the formula (I) or a salt thereof include hydrates.

The compound represented by the formula (I), a salt thereof, a solvate thereof, or a prodrug thereof may be a deuterium converter obtained by converting ¹H to ²H (D). Such a compound is also included in the present invention.

In this specification, the term "prodrug" refers to any compound which is administrated to a living organism and then generates the compound represented by the formula (I) as a result of a spontaneous chemical reaction or by a catalytic enzyme or a metabolic reaction. Preferably, the prodrug is a compound which can be administrated into a body as a medical drug. Examples of the prodrug include pharmaceutically acceptable esters or amides. Specifically, examples of the group which constitutes the prodrug and is used for the hydroxyl group or the amino group include C₂₋₇-acyl groups, C₁₋₆-alkoxy (C₂₋₇-acyl) groups, C₁₋₆-alkoxycarbonyl (C₂₋₇-acyl) groups, C₁₋₆-alkoxycarbonyl groups, C₁₋₆-alkoxy (C₂₋₇-alkoxycarbonyl) groups, (C₂₋₇-acyloxy)methyl groups, 1-(C₂₋₇-acyloxy)ethyl groups, (C₂₋₇-alkoxycarbonyl)oxymethyl groups, and 1-[(C₂₋₇-alkoxycarbonyl)oxy]ethyl groups. C₂₋₇-acyl groups and C₁₋₆-alkoxycarbonyl groups are preferred. Examples of the group which constitutes the prodrug and is used for the carboxyl group include C₁₋₆-alkyl groups, C₁₋₆-alkoxy-C₁₋₆-alkyl groups, (C₂₋₇-acyloxy)methyl groups, 1-(C₂₋₇-acyloxy)ethyl groups, (C₂₋₇-alkoxycarbonyl)oxymethyl groups, and 1-[(C₂₋₇-alkoxycarbonyl)oxy]ethyl groups. C₁₋₆-alkyl groups and C₁₋₆-alkoxy-C₁₋₆-alkyl groups are preferred.

The compound represented by the formula (I) can be produced as shown below, for example. 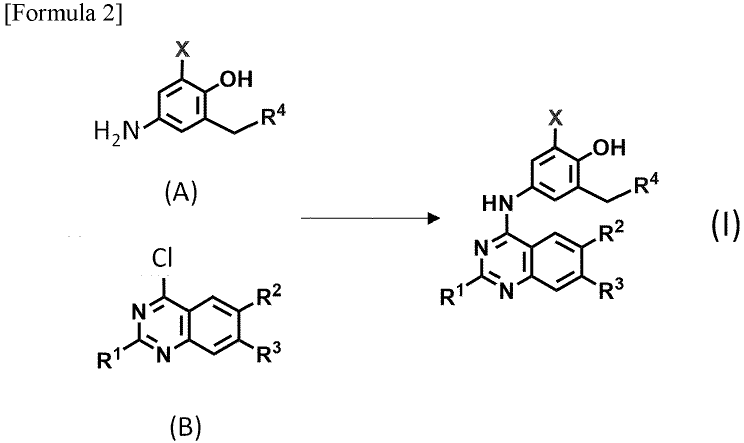 (where the symbols are as defined as in the formula (I).)

Specifically, a 4-aminophenol derivative (A) is added to an ethanol solution of a 4-chloroquinazoline compound (B), and these are reacted under heating. Thereby, the target compound (I) can be produced.

Among the compound (I), compounds where R¹, R², or R³ is a halogen atom, e.g., a chlorine atom, can be reacted with NH(R^{a})(R^{b})·HCl (where R^{a} and R^{b} are as defined as in the formula (I)), e.g., a dimethylamine hydrochloride, and converted to compounds where R¹, R², or R³ in the formula (I) is -N(R^{a})(R^{b}).

The product thus obtained may be purified by a method usually used, such as column chromatography using silica gel or the like as a carrier, or recrystallization using methanol, ethanol, chloroform, dimethyl sulfoxide, n-hexane-ethyl acetate, or water. Examples of the elution solvent for column chromatography include methanol, ethanol, chloroform, acetone, hexane, dichloromethane, ethyl acetate, and mixed solvents thereof.

The compound can be formulated in combination with a traditional pharmaceutical carrier, as an anti-SARS-CoV-2 drug. The dosage form thereof is not particularly limited, and can be appropriately selected as needed. Examples of the dosage form include oral agents such as a tablet, a capsule, a granule, a fine granule, a powder, a sustained release formulation, a liquid preparation, a suspension, an emulsion, a syrup, and an elixir, and parenteral agents such as an injection and a suppository.

The oral agents are produced by a normal method, for example, using starch, lactose, sucrose, mannite, carboxymethyl cellulose, or inorganic salts. In addition to these, a binder, a disintegrant, a surfactant, a lubricant, a glidant, a flavoring agent, a colorant, and/or a fragrance can be appropriately added.

Examples of the binder include starch, dextrin, gum arabic, gelatin, hydroxypropyl starch, methylcellulose, sodium carboxymethylcellulose, hydroxypropylcellulose, crystalline cellulose, ethylcellulose, polyvinylpyrrolidone, and macrogol.

Examples of the disintegrant include starch, hydroxypropyl starch, carboxymethylcellulose sodium, carboxymethylcellulose calcium, carboxymethylcellulose, and a low-substituted hydroxypropylcellulose.

Examples of the surfactant include sodium lauryl sulfate, soy lecithin, sucrose fatty acid ester, and polysorbate 80.

Examples of the lubricant include talc, waxes, hydrogenated vegetable oil, sucrose fatty acid esters, magnesium stearate, calcium stearate, aluminum stearate, and polyethylene glycol.

Examples of the glidant include light anhydrous silicic acid, dry aluminum hydroxide gel, synthetic aluminum silicate, and magnesium silicate.

The injection is produced by a normal method, and as a diluent, distilled water for injection, saline, a glucose aqueous solution, olive oil, sesame oil, peanut oil, soybean oil, corn oil, propylene glycol, or polyethylene glycol can be generally used. Further, a bactericide, a preservative agent, a stabilizer, an isotonizing agent, and/or a soothing agent may be added as needed. From the viewpoint of stability, the injection can be filled into a vial or the like, frozen, and then subjected to ordinary lyophilization to remove the water content. Immediately before use, a liquid preparation can be reconstituted from the lyophilized injection. The proportion of the compound represented by the formula (I) in the injection can be varied between 5% by weight and 50% by weight, but not limited thereto.

Examples of other parenteral agents include suppositories for intrarectal administration, which are produced by a normal method.

The formulated anti-SARS-CoV-2 drug can be administrated, for example, 1 to 4 times a day for one week to three months, depending on the form of the drug and the administration route.

The anti-SARS-CoV-2 drug according to the present invention is used for the treatment of COVID-19. In the present invention, the treatment encompasses prevention of severe illness.

To demonstrate a desired effect as an oral agent, although the dose varies depending on the age and the body weight of the patient and the severity of the disease thereof, usually, for an adult, it is suitable that the dose is, for example, 0.1 to 1000 mg, preferably 1 to 500 mg of the compound represented by the formula (I) per day, which is divided into portions and administrated.

To demonstrate a desired effect as a parenteral agent, although the dose varies depending on the age and the body weight of the patient and the severity of the disease thereof, usually, for an adult, it is suitable that the dose is, for example, 0.1 to 1000 mg, preferably 1 to 500 mg of the compound represented by the formula (I), which is administrated by intravenous injection, intravenous drip, subcutaneous injection, or intramuscular injection.

The compound represented by the formula (I), a salt thereof, a solvate thereof, or a prodrug thereof can be contained in a pharmaceutical composition as the only active ingredient, or can be contained together with other active ingredients.

Further, the compound represented by the formula (I), a salt thereof, a solvate thereof, or a prodrug thereof may be used in combination with a different drug effective against SARS-CoV-2 infection. These agents are separately administrated in the course of the treatment, or are combined with the compound represented by the formula (I) into a single dosage form such as a tablet, an intravenous solution, or a capsule. Examples of such a different drug include Remdesivir.

It is known that coronaviruses infect a variety of animals, and SARS-CoV also infects a variety of animals beyond species. Thus, the subject to be treated with the anti-coronavirus drug according to the present invention is not limited to humans, and includes various animals such as pet animals (such as dogs and cats), pigs, camels, bats, palm civets, tigers, ferrets, golden hamsters, minks, and sparrows.

This specification encompasses the contents disclosed in the description and/or the drawing of Japanese Patent Application No. 2021-184512, which is a priority document of the present application.

### Examples

Hereinafter, the present invention will be more specifically described by way of Examples, but the scope of the present invention is not limited thereto.

### [Example 1] Anti-SARS-CoV-2 effect

An anti-SARS-CoV-2 assay is schematically shown in Figure 1.

VeroE6/TMPRSS2 cells highly sensitive to SARS-CoV-2 were seeded on microplates (2 × 10⁴ cells/well). After 24 hours of culture, drugs in various concentrations and SARS-CoV-2 (WK-521) (obtained from the National Institute of Infectious Diseases, Japan) were added with a multiplicity of infection (MOI) = 0.01, followed by culture at 37°C for 3 days. After the culture, 110 µL of culture supernatant was discarded, and 10 µL of Cell Counting Kit-8 (DOJINDO LABORATORIES, Co. Ltd., a viable cell counting kit using a water-soluble tetrazolium salt WST-8 (2-(2-methoxy-4-nitrophenyl)-3-(4-nitrophenyl)-5-(2,4-disulfophenyl)-2H-tetrazolium monosodium salt) as a coloring reagent) was added. After 2 hours of culture, 100 µL of 2-propanol hydrochloride was added, and was sufficiently mixed; thereafter, the absorbance of each well was measured at 450/620 nm. The anti-SARS-CoV-2 effect and the cell toxicity of the drug were determined by comparing the numbers of viable cells in infected cells and non-infected cells with those in the absence of the drugs.

The anti-SARS-CoV-2 effects of various quinazoline derivatives are shown in Tables 1 to 3.

**[Table 1]**

| 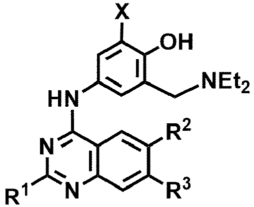 | | | | | | |
|---|---|---|---|---|---|---|
| | | | | | | |

| Compound | R¹ | R² | R³ | X | EC₅₀ (µM) | CC₅₀ (µM) |
|---|---|---|---|---|---|---|
| 1 (YMSA-0650) | H | H | H | H | 30.4 | >100 |
| 2 (YMSA-0656) | H | H | H | Cl | 30.1 | >100 |
| 3 (YMSA-0516) | H | H | H | Br | 23.9 | >100 |
| 4 (YMSA-0464) | H | H | H | I | 16.9 | >100 |
| 5 (YMSA-0517) | H | OMe | OMe | I | 31.6 | >100 |
| 6 (YMSA-0636) | NMe₂ | H | H | I | 12.9 | 49.9 |
| 7 (YMSA-0707) | NMe₂ | H | H | Cl | 7.2 | 49.2 |

**[Table 2]**

| 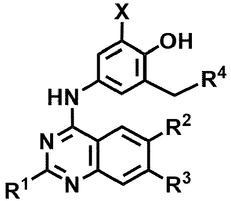 | | | | | | | |
|---|---|---|---|---|---|---|---|
| Compound | R¹ | R² | R³ | R⁴ | X | EC₅₀ (µM) | CC₅₀ (µM) |
| 8 (YMSA-0614) | H | H | H | NMeEt | I | 57.6 | >100 |
| 9 (YMSA-0628) | H | H | H | NMe(CH₂C₆H₅) | I | 8.0 | 38.1 |
| 10 (YMSA-0576) | H | H | H | 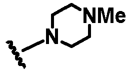 | I | 15.5 | >100 |
| 11 (YMSA-0808) | H | H | H | 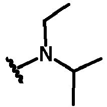 | Cl | 7.6 | >100 |
| 12 (YMSA-0828) | H | H | OMe | 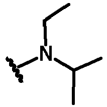 | Cl | 9.5 | >100 |
| 13 (YMSA-0613) | H | H | H | NHEt | I | 44.7 | >100 |
| 14 (YMSA-0644) | H | H | H | 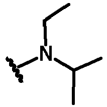 | I | 17.8 | >100 |
| 15 (YMSA-0785) | H | H | H | 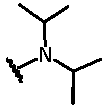 | Cl | 3.3 | >100 |
| 16 (YMSA-0781) | H | H | H | 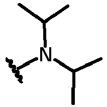 | I | 44.6 | >100 |
| 17 (YMSA-0842) | H | H | H | 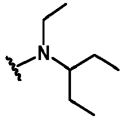 | Cl | 5.0 | >100 |
| 18 (YMSA-0832) | H | H | H | 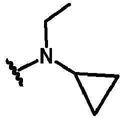 | Cl | 53.1 | >100 |

**[Table 3]**

| Compound | Formula | EC₅₀ (µM) | CC₅₀ (µM) |
|---|---|---|---|
| 19 (YMSA-0797) | 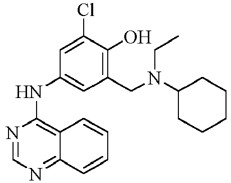 | 3.2 | >100 |
| 20 (YMSA-0823) | 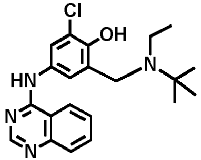 | 3.9 | >100 |
| 21 (YMSA-0864) | 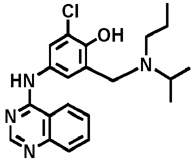 | 11.7 | >100 |
| 22 (YMSA-0897) | 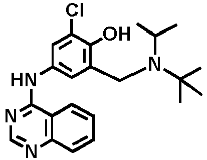 | 10.8 | >100 |
| 23 (YMSA-0908) | 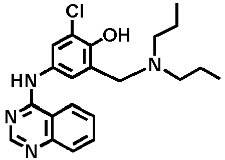 | 15.3 | >100 |
| 24 (YMSA-0863) | 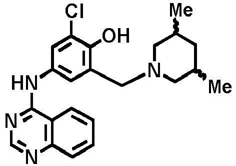 | 2.1 | >100 |
| 25 (YMSA-0907) | 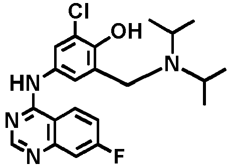 | 2.5 | >100 |
| 26 (YMSA-0868) | 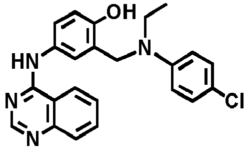 | 11.7 | >100 |
| 27 (YMSA-0884) | 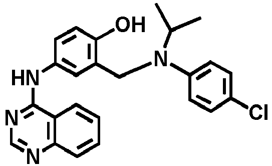 | 13.9 | >100 |
| 28 (YMSA-0909) | 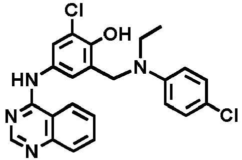 | 10.0 | >100 |
| 29 (YMSA-0833) | 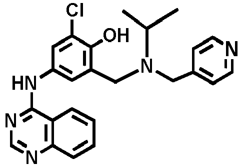 | 10.6 | >100 |
| 30 (YMSA-0944) | 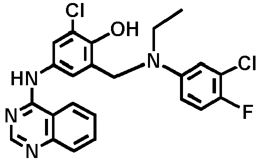 | 10.8 | >100 |
| 31 (YMSA-1012) | 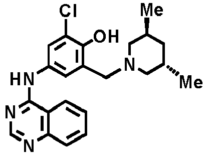 | 19.4 | 37.5 |
| 32 (YMSA-1033) | 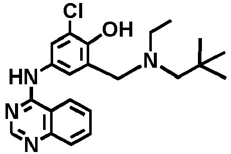 | 20.0 | 60.2 |
| 33 (YMSA-1066) | 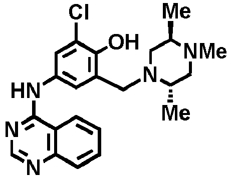 | 54.7 | >100 |
| 34 (YMSA-0974) | 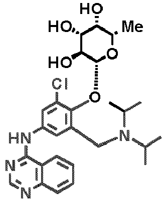 | 60.2 | >100 |
| Nirmatrelvir | | 8.3 | >100 |

| | | | |
|---|---|---|---|
| EC₅₀: 50% effective concentration (concentration of the drug which inhibits cell death induced by SARS-CoV-2 infection by 50%) CC₅₀: 50% toxicity concentration (concentration of the drug which reduces the number of viable cells in non-infected cells by 50%) | | | |

Table 4 shows the anti-SARS-CoV-2 effect of compounds in which hydroxyl group in the phenylamino group substituting the 4-position of the quinazoline ring is present at the 3-position in place of the 4-position of the phenylamino group as in the formula (I).

**[Table 4]**

| 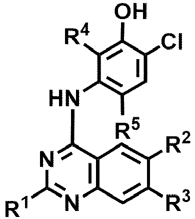 | | | | | | | |
|---|---|---|---|---|---|---|---|
| Compound | R¹ | R² | R³ | R⁴ | R⁵ | EC₅₀ (µM) | CC₅₀ (µM) |
| YMSA-0843 | H | H | H | 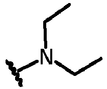 | H | >100 | >100 |
| YMSA-0850 | H | H | H | H | 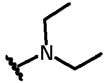 | >100 | >100 |

| | | | | | | | |
|---|---|---|---|---|---|---|---|
| EC₅₀: 50% effective concentration (concentration of the drug which inhibits cell death induced by SARS-CoV-2 infection by 50%) CC₅₀: 50% toxicity concentration (concentration of the drug which reduces the number of viable cells in non-infected cells by 50%) | | | | | | | |

Tables 1 to 3 reveal that the compounds represented by the formula (I) according to the present invention have the anti-SARS-CoV-2 effects. In contrast, Table 4 reveals that sufficient anti-SARS-CoV-2 effects were not observed in the compounds which have basic skeletons similar to that of the compound represented by the formula (I) according to the present invention but have the substituting hydroxyl group at a different position.

### [Example 2] Effect against SARS-CoV-2 mutant strain and the like

Among the compounds according to the present invention, four derivatives demonstrating strong anti-SARS-CoV-2 effects against VeroE6/TMPRSS2 cells were selected, and examined for their anti-SARS-CoV-2 effects using cells other than VeroE6/TMPRSS2. As a result, these four derivatives also demonstrated strong effects against HEK293T/ACE2 cells derived from humans. In particular, YMSA-0785 had 50% effective concentration (EC₅₀) of 0.12 µM, 50% toxicity concentration (CC₅₀) of >100 µM, and selectivity coefficient (CC₅₀/EC₅₀) of >833 (Table 5).

These drugs also had similar anti-SARS-CoV-2 effects against mutant strains as those against the standard strains (Table 5).

**[Table 5]**

| **Cells (Assay)** | **Drug** | | | |
|---|---|---|---|---|
| | **YMSA-0785** | **YMSA-0797** | **YMSA-0808** | **YMSA -0842** |
| VeroE6/TMPRSS2 (CPE) | **3.3** | **3.2** | **7.6** | **5.0** |
| | > 100 | > 100 | > 100 | > 100 |
| VeroE6/TMPRSS2 (RNA) | **1.9** | **2.9** | **1.9** | **2.0** |
| Vero (RNA) | **1.7** | **2.9** | **5.5** | **1.2** |
| HEK293T/ACE2 (RNA) | **0.12** | **0.14** | **0.12** | **0.31** |
| | > 100 | 36.1 | 85.4 | 13.8 |

| **Effects against mutant virus strain** (CPE assay in Vero/TMPRSS2) | | | | |
|---|---|---|---|---|
| Alpha strain (QK002) | **2.7** | **5.8** | **3.8** | **2.6** |
| Beta strain (TY8-612) | **6.8** | **8.6** | **9.3** | **8.1** |
| Gamma strain (TY7-501) | **3.5** | **4.2** | **5.4** | **3.4** |

The numerals in the upper rows indicate EC₅₀ (µM), and the numerals in the lower ro ws indicate CC₅₀ (µM).

The assay in HEK293T/ACE2 cells was performed as follows.
(Assay in HEK293T/ACE2 cells)

### (1) Determination of cell viability (absorbance)

HEK293T/ACE2 cells (2 × 10⁴ cells/well) were seeded on microplates with 100 µL of cell culture solution. After 24 hours of culture, 50 µL each of various drugs prepared by diluting drug stock solution to 4 times the final concentration was added to each well. Further, 50 µL of virus solution of SARS-CoV-2 (WK-521 strain) (obtained from the National Institute of Infectious Diseases, Japan) was added with MOI = 0.1 (plate for infection). To a plate for cells, 50 µL each of the various drugs prepared by diluting the drug stock solution to 4 times the final concentration was added to each well, and 50 µL of the cell culture solution was added thereto. After 3 days of culture, the culture supernatant on the plate for infection was transferred to a new plate, which was stored at -80°C. 110 µL of the culture solution was discarded from the plate for cells, and 10 µL of Cell Counting Kit-8 was added. After 2 hours of culture in a CO₂ incubator, absorbance at 450 nm (620 nm) was measured.

### (2) qPCR measurement of viral RNA in culture supernatant

To 50 µL of a culture supernatant, 50 µL of DNA/RNA Shield (Zymo Research) was added, and using Quick-RNA Viral 96 kit (Zymo Research), RNAs were extracted according to the instructions (15 µL). The extracted RNAs were diluted 10-fold with nuclease-free water, which was used as an RNA sample. Using High-Capacity RNA-to-cDNA^{™} Kit (Thermo Fisher Scientific), cDNAs were synthesized from the RNA sample, and the RNA amount in the sample was measured by real-time PCR.

### [Example 3] Synthesis of YMSA-0398 hydrochloride

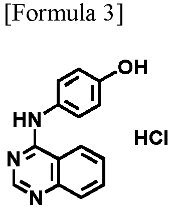

### YMSA-0398 hydrochloride

Using a microwave synthesizing apparatus (Discover, CEM, 50 W), an EtOH solution (5 mL) of 4-chloroquinazoline (144 mg, 0.88 mmol) and 4-aminophenol (99 mg, 1.2 mmol) was reacted at 80°C for 50 minutes. After the temperature of the reaction solution was returned to room temperature, Et₂O (5 mL) was added, and deposited solids were separated with a filter, and washed with diethyl ether. The obtained solids were recrystallized from Et₂O/MeOH, giving YMSA-0398 hydrochloride as a light yellow green powder (120 mg, 43%). mp 284-290°C.

### [Example 4] Synthesis of YMSA-0329 hydrochloride

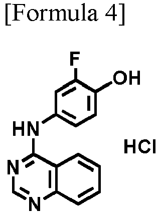

### YMSA-0329 hydrochloride

Using a microwave reactor (Discover, CEM, 50 W), an EtOH solution (5 mL) of 4-chloroquinazoline (119 mg, 0.73 mmol) and 4-amino-2-fluorophenol (93 mg, 0.73 mmol) was reacted at 80°C for 60 minutes. After the temperature of the reaction solution was returned to room temperature, Et₂O (5 mL) was added, and deposited solids were separated with a filter, and washed with diethyl ether. The obtained solids were recrystallized from Et₂O/MeOH, giving YMSA-0329 hydrochloride as a yellow green powder (117 mg, 63%). mp 249-265°C.

### [Example 5] Synthesis of YMSA-0721 hydrochloride

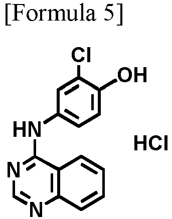

### YMSA-0721 hydrochloride

Using a microwave reactor (Discover, CEM, 50 W), an EtOH solution (5 mL) of 4-chloroquinazoline (189 mg, 1.2 mmol) and 4-amino-2-chlorophenol (164 mg, 1.1 mmol) was reacted at 80°C for 60 minutes. After the temperature of the reaction solution was returned to room temperature, Et₂O (5 mL) was added, and deposited solids were separated with a filter, and washed with diethyl ether. The obtained solids were recrystallized from MeOH, giving YMSA-0721 hydrochloride as a yellow powder (176 mg, 65%). mp 284-290°C.

### [Example 6] Synthesis of YMSA-0840 hydrochloride

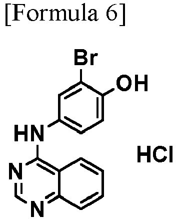

### YMSA-0840 hydrochloride

Using a microwave reactor (Discover, CEM, 50 W), a mixture of 4-chloroquinazoline (134 mg, 0.81 mmol) and 4-amino-2-bromophenol (153 mg, 0.82 mmol) was reacted in EtOH (5 mL) at 80°C for 60 minutes. After the temperature of the reaction solution was returned to room temperature, Et₂O (5 mL) was added, and deposited solids were separated with a filter, and washed with diethyl ether. The obtained solids were recrystallized from EtOH, giving YMSA-0840 1.25 hydrochloride as a yellow powder (96 mg, 37%). mp 277-288°C.

### [Example 7] Synthesis of YMSA-0414 hydrochloride

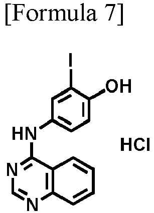

### YMSA-0414 hydrochloride

Using a microwave reactor (Discover, CEM, 50 W), a mixture of 4-chloroquinazoline (91 mg, 0.56 mmol) and 4-amino-2-iodophenol (133 mg, 0.56 mmol) was reacted in EtOH (5 mL) at 80°C for 60 minutes. After the temperature of the reaction solution was returned to room temperature, Et₂O (5 mL) was added, and deposited solids were separated with a filter, and washed with diethyl ether. The obtained solids were recrystallized from MeOH, giving YMSA-0414 hydrochloride as a yellow powder (104 mg, 51%). mp 250-255°C.

### [Example 8] Synthesis of YMSA-0891 hydrochloride

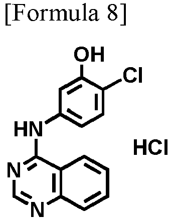

### YMSA-0891 hydrochloride

Using a microwave reactor (Discover, CEM, 50 W), a mixture of 4-chloroquinazoline (119 mg, 0.73 mmol) and 5-amino-2-chlorophenol (103 mg, 0.72 mmol) was reacted in EtOH (5 mL) at 80°C for 60 minutes. After the temperature of the reaction solution was returned to room temperature, Et₂O (5 mL) was added, and deposited solids were separated with a filter, and washed with diethyl ether. The obtained solids were recrystallized from MeOH, giving YMSA-0891 hydrochloride as a yellow powder (118 mg, 60%). mp 287-289°C.

### [Example 9] Synthesis of YMSA-0464

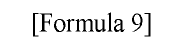

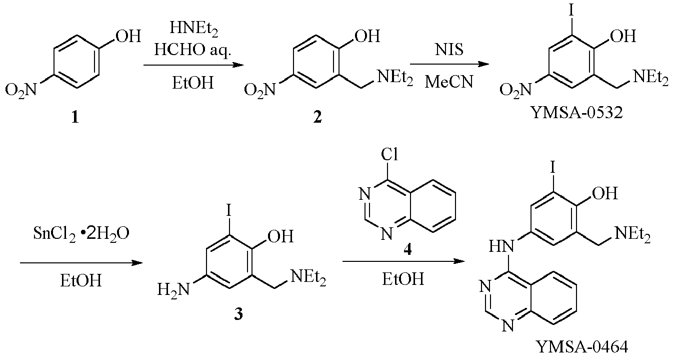

### 2-((Diethylamino)methyl)-4-nitrophenol (2)

Diethylamine (8.6 mL, 83.5 mmol) and a 37% HCHO aqueous solution (8.5 mL, 0.10 mol) were added to an EtOH (100 mL) solution of 4-nitrophenol 1 (2.9 g, 20.8 mmol), followed by heating under reflux for 44 hours. After the reaction, the reaction solution was concentrated under reduced pressure. H₂O (50 mL) was added, followed by extraction with AcOEt (50 mL × 3). The combined organic phases were washed with saturated saline water, dried over Na₂SO₄, filtered, and concentrated under reduced pressure. The resulting residue was purified by silica gel column chromatography (hexane/AcOEt = 1/1 to 1/3), giving Compound 2 as a yellow oily substance (3.5 g, 76%).

### 2-((Diethylamino)methyl)-6-iodo-4-nitrophenol (YMSA-0532)

N-iodosuccinimide (1.6 g, 7.3 mmol) was added to an MeCN solution (35 mL) of 2 (1.6 g, 7.3 mmol), and these were stirred under room temperature for 30 minutes. The reaction solution was concentrated under reduced pressure, and purified by silica gel column chromatography (hexane/AcOEt = 1/1 to 1/2). Hexane/CHCl₃ was added to the resulting compound, insolubles were filtered out, and the filtrate was concentrated under reduced pressure, giving Compound 3 as a yellow solid (1.2 g, 47%). mp 118°C (dec.).

### 4-Amino-2-((diethylamino)methyl)-6-iodophenol (3)

SnCl₂·2H₂O (0.31 g, 1.4 mmol) was added to an EtOH (8 mL) solution of YMSA-0532 (96 mg, 0.27 mmol), and the reaction solution was heated under reflux for 1.5 hours. The reaction solution was concentrated under reduced pressure, and a saturated aqueous NaHCO₃ solution (30 mL) was added, followed by extraction with AcOEt (30 mL × 3). The combined organic phases were washed with saturated saline water, dried over Na₂SO₄, filtered, and concentrated under reduced pressure, giving Compound 3 as a yellow solid (82.6 mg, 90%). mp 69°C (dec.).

### YMSA-0464

Compound 3 (36 mg, 0.11 mmol) was added to an EtOH solution (1 mL) of 4-chloroquinazoline (4) (18 mg, 0.11 mmol), and the reaction solution was heated under reflux for 3 hours. The reaction solution was concentrated under reduced pressure, and the resulting residue was purified by silica gel column chromatography (hexane/AcOEt = 1/1). The resulting oily substance was formed into solids with Et₂O/MeOH, giving YMSA-0464 as a beige powdery substance (5.5 mg, 11%). mp 185-189°C. IR (ATR): v = 2971, 1618, 1572, 1533, 1497, 1444, 1387, 1353, 1319, 1284, 1246, 1169, 1115, 1067, 1049, 997, 956, 908, 876, 816, 765, 680, 671, 637, 591, 581, 564, 512, 502, 473, 463, 434, 414 cm⁻¹. ¹H NMR (300 MHz, CDCl₃/TMS): δ = 8.73 (s, 1H), 7.90-7.81 (m, 4H), 7.56 (t, J = 6.9 Hz, 1H), 7.43 (d, J = 2.7 Hz, 1H), 3.81 (s, 2H), 2.68 (q, J = 7.2 Hz, 4H), 1.15 (t, J = 7.2 Hz, 6H) ppm. ¹³C NMR (100 MHz, CDCl₃/TMS): δ = 157.8, 155.6, 155.2, 150.0, 133.0, 132.1, 130.1, 129.1, 126.7, 123.7, 122.1, 120.3, 115.0, 84.6, 57.0, 46.3, 11.1 ppm. HRMS (FAB⁺): calcd for [M+H]⁺, C₁₉H₂₂IN₄O: 449.0838; found 449.0839. Anal. Calcd (%) for C₁₉H₂₁IN₄O: C, 50.90; H, 4.72; N, 12.50. found: C, 50.51; H, 4.63; N, 12.33.

### [Example 10] Synthesis of YMSA-0514

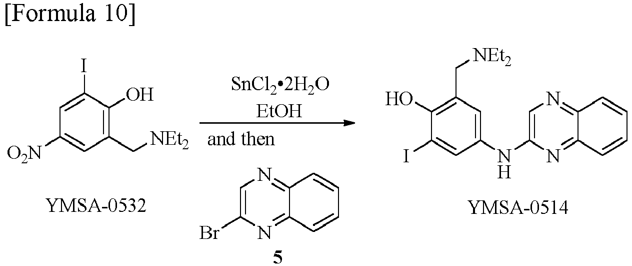

SnCl₂·2H₂O (0.25 g, 1.1 mmol) was added to an EtOH (4 mL) solution of YMSA-0532 (80 mg, 0.22 mmol), and the reaction solution was heated under reflux for 30 minutes. 2-Bromoquinoxaline (5) (36 mg, 0.22 mmol) was added thereto, followed by further heating under reflux for 4 hours. The reaction solution was concentrated under reduced pressure, and the resulting residue was purified by silica gel column chromatography (CHCl₃/MeOH = 50/1 to 10/1). The resulting oily substance was formed into solids with Et₂O/MeOH, giving YMSA-0514 as a yellow powder substance (98 mg, 97%). mp 37-40°C. IR (ATR): v = 3303, 2969, 2930, 1668, 1611, 1582, 1543, 1459, 1412, 1386, 1349, 1287, 1228, 1191, 1163, 1112, 1044, 1019, 991, 953, 928, 907, 864, 807, 756, 715, 660, 636, 597, 547, 504, 414 cm⁻¹. ¹H NMR (399 MHz, CDCl₃/TMS): δ = 8.34 (s, 1H), 7.92-7.89 (m, 2H), 7.75 (dd, J = 8.6, 1.2 Hz, 1H), 7.62 (td, J = 6.8, 1.2 Hz, 1H), 7.45 (td, J = 7.8, 1.2 Hz, 1H), 7.33 (d, J= 1.8 Hz, 1H), 6.66 (brs, 1H), 3.78 (s, 2H), 2.67 (q, J = 7.2 Hz, 4H), 1.14 (t, J = 7.2 Hz, 6H) ppm. ¹³C NMR (100 MHz, CDCl₃/TMS): δ = 155.0, 149.8, 137.9, 131.2, 130.7, 130.4, 128.9, 126.8, 125.5, 122.4, 122.2, 116.5, 84.9, 57.0, 46.4, 46.3, 11.1 ppm. HRMS (FAB⁺): calcd for [M+H]⁺, C₁₉H₂₂IN₄O: 449.0833; found 449.0834. Anal. Calcd (%) for C₂₀H₁₃IN₄O·0.3H₂O, 0.1AcOEt: C, 51.42; H, 5.16; N, 11.76. found: C, 51.45; H, 5.04; N, 11.47.

### [Example 11] Synthesis of YMSA-0516

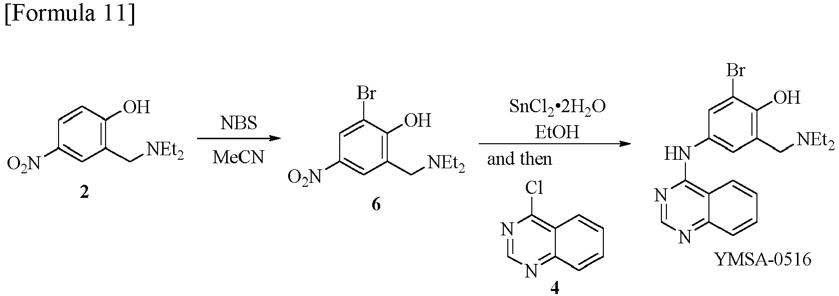

### 2-Bromo-6-((diethylamino)methyl)-4-nitrophenol (6)

Compound 6 was obtained as a yellow solid from Compound 2 and N-bromosuccinimide (NBS) by the same method as that in the synthesis of YMSA-0532 (0.16 g, 50%). mp 148°C (dec.).

### YMSA-0516

YMSA-0516 was obtained as a yellow solid by the same method as that in YMSA-0514 (0.11 g, 88%). mp 181-185°C. IR(ATR): v = 2975, 1619, 1573, 1534, 1497, 1465, 1403, 1354, 1319, 1287, 1247, 1224, 1184, 1119, 1069, 1051, 998, 957, 917, 875, 854, 817, 765, 723, 676, 639, 592, 584, 566, 514, 464, 437, 423, 408 cm⁻¹. ¹H NMR (399 MHz, DMSO-d₆/TMS): δ = 8.73 (s, 1H), 7.91 (d, J = 8.0 Hz, 1H), 7.85-7.78 (m, 2H), 7.71 (d, J= 2.0 Hz, 1H), 7.55 (t, J = 7.2 Hz, 1H), 7.35 (d, J = 2.0 Hz, 1H), 7.30 (s, 1H), 3.82 (s, 2H), 2.67 (q, J = 6.8 Hz, 4H), 1.14 (t, J= 6.8 Hz, 6H) ppm. ¹³C NMR (100 MHz, CDCl₃/TMS): δ = 157.9, 155.1, 153.1, 150.0, 133.0, 129.6, 129.0, 126.7, 126.4, 123.1, 122.5, 120.4, 115.0, 110.0, 57.0, 46.4, 11.1 ppm. HRMS (FAB⁺): calcd for [M+H]⁺, C₁₉H₂₂BrN₄O: 401.0977; found 401.0976. Anal. Calcd (%) for C₁₉H₂₁BrN₄O·0.2H₂O: C, 56.36; H, 5.33; N, 13.84. found: C, 56.34; H, 5.09; N 13.50.

### [Example 12] Synthesis of YMSA-0517

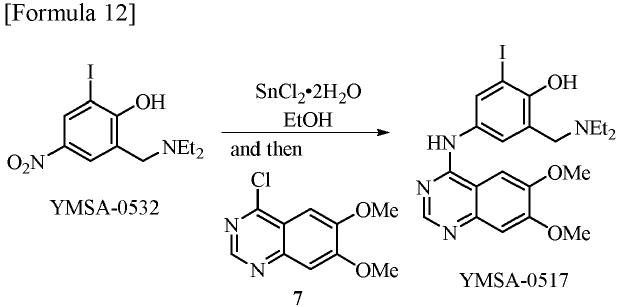

YMSA-0517 was obtained as a yellow powder substance by the same method as that in the synthesis of YMSA-0514 (53 mg, 69%). mp 125°C (dec.). IR (ATR): v = 2971, 1622, 1579, 1504, 1456, 1430, 1335, 1240, 1218, 1145, 1112, 1072, 1030, 1004, 914, 846, 804, 748, 687, 661, 568, 557, 508, 465, 420 cm⁻¹. ¹H NMR (300 MHz, DMSO-d₆/TMS): δ = 8.77 (s, 1H), 8.23 (s, 1H), 8.13 (d, J = 2.4 Hz, 1H), 7.85 (d, J = 2.4 Hz, 1H), 7.30 (s, 1H), 4.34 (s, 2H), 4.01 (s, 3H), 3.99 (s, 3H), 3.13 (q, J = 6.9 Hz, 4H), 1.28 (t, J = 6.9 Hz, 6H) ppm. ¹³C NMR (100 MHz, DMSO-d₆/TMS): δ = 157.5, 155.2, 155.1, 154.1, 149.9, 147.9, 132.8, 132.3, 124.6, 123.1, 109.8, 108.3, 102.9, 85.0, 57.3, 57.2, 56.9, 46.7, 12.0 ppm. HRMS (FAB⁺): calcd for [M+H]⁺, C₂₁H₂₆IN₄O₃:509.1050; found 509.1050. Anal. Calcd (%) for C₂₁H₂₅IN₄O₃·0.5H₂O: C, 48.05; H, 5.07; N, 10.83. found: C, 48.37; H, 4.74; N, 10.62.

### [Example 13] Synthesis of YMSA-0576

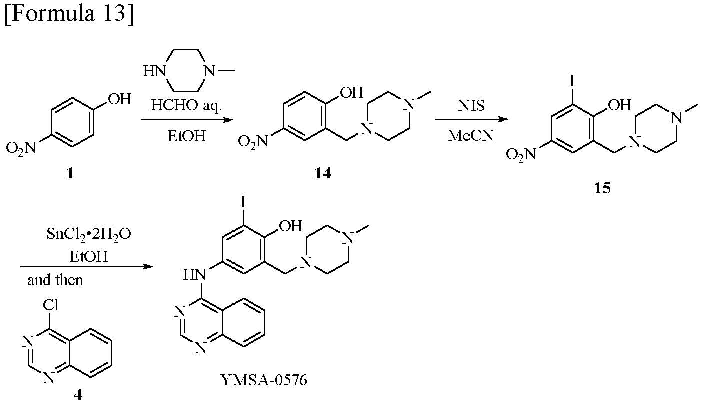

### 2-((4-Methylpoperazin-1-yl)methyl)-4-nitrophenol (14)

Compound 14 was obtained as a yellow oily substance by the same method as that in the synthesis of Compound 2 (0.50 g, 67%).

### 2-Iodo-6-((4-methylpoperazin-1-yl)methyl)-4-nitrophenol (15)

Compound 15 was obtained as a yellow solid by the same method as that in the synthesis of YMSA-0532 (0.17 g, 38%). mp 148°C(dec.).

### YMSA-0576

YMSA-0576 was obtained as a yellow solid by the same method as that in YMSA-0514 (24 mg, 34%). mp 190°C (dec.). IR (ATR): v = 2938, 2795, 1619, 1589, 1567, 1529, 1499, 1442, 1433, 1410, 1390, 1356, 1342, 1321, 1302, 1281, 1240, 1157, 1134, 1117, 1087, 1075, 1050, 1005, 987, 924, 913, 885, 867, 815, 801, 775, 724, 681, 653, 631, 596, 580, 567, 528, 508, 488, 469, 432, 428 cm⁻¹. ¹H NMR (399 MHz, CDCl₃/TMS): δ = 8.73 (s, 1H), 7.93-7.90 (m, 2H), 7.85-7.79 (m, 2H), 7.76 (t, J = 6.8 Hz, 1H), 7.44 (d, J= 2.4 Hz), 7.28 (brs, 1H), 3.74 (s, 2H), 2.60 (brs, 8H), 2.32 (s, 3H) ppm. ¹³C NMR (100 MHz, CDCl₃/TMS): δ = 157.7, 155.0, 154.7, 149.9, 133.0, 132.2, 130.4, 129.0, 126.6, 123.8, 121.2, 120.1, 114.8, 84.4, 61.4, 54.6, 52.4, 45.8 ppm. HRMS (FAB⁺): calcd for [M+H]⁺, C₂₀H₂₃IN₅O: 476.0947; found 479.0946. Anal. Calcd (%) for C₂₀H₂₂IN₅O·0.5H₂O: C, 49.60; H, 4.79; N, 14.46. found: C, 49.54; H, 4.76; N, 14.45.

### [Example 14] Synthesis of YMSA-0613

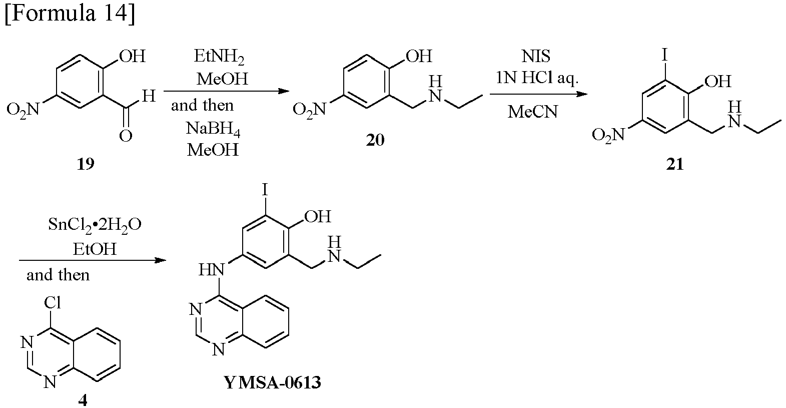

### 2-((Ethylamino)methyl)-4-nitrophenol (20)

A 70% ethylamine aqueous solution (85 µL, 1.0 mmol) was added to an MeOH (5 mL) solution of 5-nitrosalicylaldehyde (0.17 g, 1.0 mmol). After the reaction solution was stirred at room temperature for 1 hour, an MeOH (3 mL) solution of NaBH₄ (44 mg, 1.2 mmol) was added, and the reaction solution was further stirred at room temperature for 1 hour. The formed solid was separated with a filter, and washed with MeOH, giving Compound 20 as a yellow powdery substance (0.16 g, 79%). mp 209-210°C.

### 2-((Ethylamino)methyl)-6-iodo-4-nitrophenol (21)

A 1N HCl aqueous solution (10 drops) was added to an MeCN solution of Compound 20 (0.10 g, 0.50 mmol) until the reaction solution became colorless. Further, NIS (0.13 g, 0.57 mmol) was added, and the reaction solution was stirred at room temperature for 30 minutes. The reaction was stopped with saturated NaHCO₃ aqueous solution, and the pH was adjusted to 7. The formed solids were filtered out, and the filtrate was concentrated under reduced pressure. The resulting residue was purified by silica gel column chromatography (CHCl₃/MeOH = 10/1), giving Compound 21 as a yellow solid (0.17 g, 100%). mp 145°C (dec.).

### YMSA-0613

YMSA-0613 was obtained as a yellow powder substance by the same method as that in the synthesis of YMSA-0514 (18 mg, 28%). mp 115°C (dec.). IR (ATR): v = 2970, 1618, 1576, 1516, 1463, 1392, 1357, 1318, 1125, 1073, 918, 868, 765, 681, 570, 470, 418, 407 cm⁻¹. ¹H NMR (399 MHz, CDCl₃/TMS): δ = 8.72 (s, 1H), 7.91 (d, J = 8.4 Hz, 1H), 7.85-7.78 (m, 3H), 7.55 (t, J= 8.4 Hz, 1H), 7.43 (s, 1H), 4.01 (s, 2H), 2.77 (q, J = 8.4 Hz, 2H), 1.20 (t, J= 7.6 Hz, 3H) ppm. ¹³C NMR (100 MHz, CDCl₃/TMS): δ = 155.5, 155.0, 149.9, 132.8, 132.2, 130.0, 129.0, 126.6, 123.8, 122.5, 120.2, 114.9, 84.8, 52.5, 43.1, 14.7 ppm. HRMS (FAB⁺): calcd for [M+H]⁺, C₁₇H₁₈IN₄O: 421.0525; found 421.0526. Anal. Calcd (%) for C₁₇H₁₇IN4O·0.1AcOEt: C, 48.71; H, 4.18; N, 13.06. found: C, 48.58; H, .4.31; N, 12.81.

### [Example 15] Synthesis of YMSA-0614

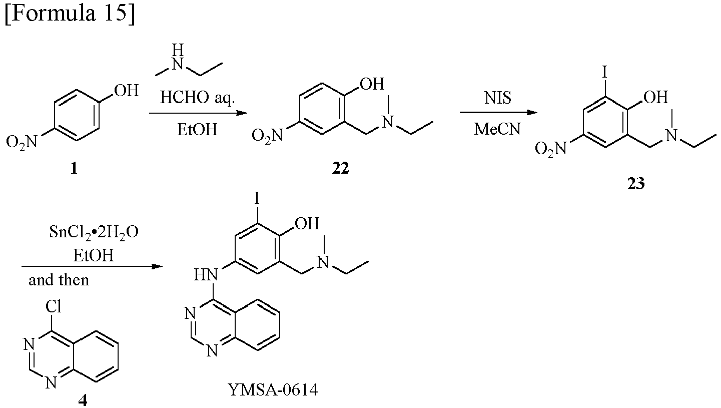

### 2-((Ethyl(methyl)amino)methyl)-4-nitrophenol (22)

Compound 22 was obtained as a yellow solid by the same method as that in the synthesis of Compound 2 (0.75 g, 71%). mp 48-51°C.

### 2-((Ethyl(methyl)amino)methyl)-6-iodo-4-nitrophenol (23)

Compound 23 was obtained as a yellow solid by the same method as that in the synthesis of YMSA-0532 (0.41 g, 81%). mp 121-131°C.

### YMSA-0614

YMSA-0614 was obtained as a yellow solid by the same method as that in the synthesis of YMSA-0464 (55 mg, 85%). mp 163°C (dec.). IR(ATR): v = 2967, 1618, 1572, 1531, 1497, 1452, 1389, 1355, 1318, 1223, 1166, 1074, 1029, 913, 867, 818, 762, 725, 677, 573, 560, 466, 440, 419 cm⁻¹. ¹H NMR (399 MHz, CDCl₃/TMS): δ = 8.72 (s, 1H), 7.91 (d, J = 8.4 Hz, 1H), 7.87-7.85 (m, 2H), 7.80 (t, J= 8.0 Hz, 1H), 7.55 (t, J = 6.3 Hz, 1H), 7.41 (d, J = 2.4 Hz, 1H), 7.38 (brs, 1H), 2.61 (q, J= 7.2 Hz, 2H), 2.32 (s, 2H), 1.17 (t, J= 7.2 Hz, 3H) ppm. ¹³C NMR (100 MHz, CDCl₃/TMS): δ = 156.7, 154.3, 154.0, 148.9, 131.9, 131.1, 129.1, 127.9, 125.6, 122.6, 120.8, 119.3, 113.6, 83.5, 59.7, 49.7, 39.5, 10.9 ppm. HRMS (FAB⁺): calcd for [M+H]⁺, C₁₈H₂₀IN₄O: 435.0682; found 435.0682. Anal. Calcd (%) for C₁₈H₁₉IN₄O·0.3AcOEt: C, 50.06; H, 4.68; N, 12.16. found: C, 49.90; H, 4.42; N, 11.91.

### [Example 16] Synthesis of YMSA-0636

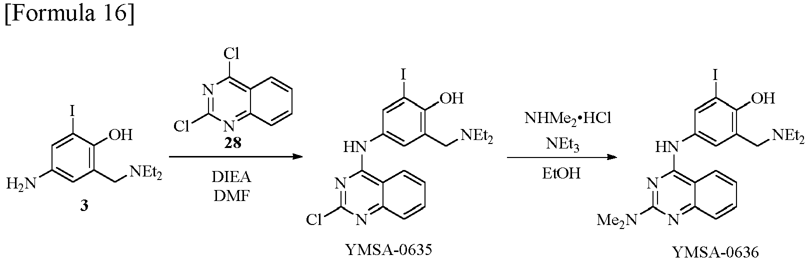

### YMSA-0635

Compound 3 (76 mg, 0.24 mmol) and N,N-diisopropylethylamine (DIEA) (45 µL, 0.25 mmol) were added to a DMF (2 mL) solution of 2,4-dichloroquinazoline 28 (34 mg, 0.17 mmol). After the reaction solution was stirred for 2.5 hours, H₂O (20 mL) was added, followed by extraction with EtOAc (20 mL × 3). The combined organic phases were washed with saturated saline water, dried over Na₂SO₄, filtered, and concentrated under reduced pressure. The resulting residue was purified by silica gel column chromatography (CHCl₃/MeOH = 100/1), and formed into a solid with hexane, giving YMSA-0635 as a beige solid (45 mg, 54%). mp 183-185°C. IR (ATR): v = 3263, 2969, 2848, 2930, 1616, 1567, 1528, 1494, 1446, 1388, 1365, 1354, 1340, 1278, 1249, 1195, 1167, 1116, 1982, 1067, 1048, 997, 948, 906, 875, 854, 815, 787, 761, 723, 686, 676, 639, 598, 571, 517, 503, 473, 407 cm⁻¹. ¹H NMR (399 MHz, CDCl₃/TMS): δ = 7.82-7.79 (m, 4H), 7.55-7.48 (m, 3H), 3.78 (s, 2H), 2.67 (q, J = 7.2 Hz, 4H), 1.14 (t, J= 7.2 Hz, 6H) ppm. ¹³C NMR (100 MHz, CDCl₃/TMS): δ = 158.7, 157.4, 155.9, 151.4, 133.9, 131.5, 129.5, 128.3, 126.8, 123.3, 122.1, 120.6, 113.3, 84.4, 56.9, 46.3, 11.0 ppm. HRMS (FAB⁺): calcd for [M+H]⁺, C₁₉H₂₁IClN₄O: 483.0449; found 483.0449. Anal. Calcd (%) for C₁₉H₂₀ClIN₄O: C, 47.27; H, 4.18; N, 11.61. found: C, 47.40; H, 4.12; N, 11.46.

### YMSA-0636

Dimethylamine hydrochloride (80 mg, 0.98 mmol) and NEt₃ (0.17 mL, 1.2 mmol) were added to an EtOH (5 mL) solution of YMSA-0635 (0.12 g, 0.25 mmol). After the reaction solution was heated under reflux for 4 hours, water (20 mL) was added, followed by extraction with EtOAc (20 mL × 3). The combined organic phases were washed with saturated saline water, dried over Na₂SO₄, filtered, and concentrated under reduced pressure. The resulting residue was purified by silica gel column chromatography (AcOEt), and formed into a solid with hexane/CHCl₃, giving YMSA-0636 as a light yellow solid (91 mg, 75%). mp 168-176°C. IR (ATR): v = 2969, 1620, 1580, 1559, 1523, 1459, 1379, 1305, 1259, 1191, 1160, 1122, 1087, 1042, 1019, 877, 861, 805, 790, 759, 719, 674, 651, 635, 562, 509, 433, 408 cm⁻¹. ¹H NMR (399 MHz, CDCl₃/TMS): δ = 8.14 (d, J = 2.4 Hz, 1H), 7.60 (d, J = 8.0 Hz, 1H), 7.52-7.51 (m, 2H), 7.28 (d, J = 2.0 Hz, 1H), 7.18 (brs, 1H), 7.07 (td, J= 7.2, 1.6 Hz, 1H), 3.71 (s, 2H), 3.23 (s, 6H), 2.63 (q, J = 7.2 Hz, 4H), 1.10 (t, J = 7.2 Hz, 6H) ppm. HRMS (FAB⁺): calcd for [M+H]⁺, C₂₁H₂₇IN₅O: 492.1260; found 492.1258. Anal. Calcd (%) for C₂₁H₂₆IN₅O·0.1CHCl₃: C, 50.35; H, 5.23; N, 13.91. found: C, 50.59; H, 4.98; N, 13.66.

### [Example 17] Synthesis of YMSA-0656

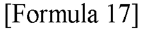

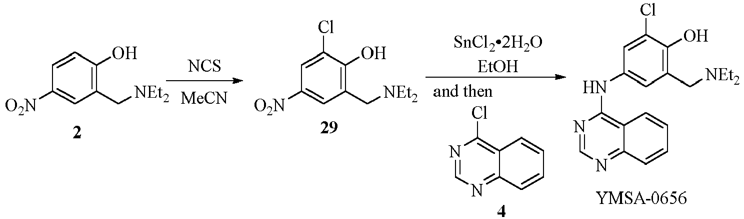

### 2-Chloro-6-((diethylamino)methyl)-4-nitrophenol (29)

Compound 29 was obtained as a yellow powder solid (1.1 g, 69%) as in the synthesis of YMSA-0532 by reacting Compound 2 and N-chlorosuccinimide. mp 142-150°C.

### YMSA-0656

YMSA-0656 was obtained as a yellow solid by the same method as that for YMSA-0464 (90 mg, 42%). mp 170-174°C. IR(ATR): v = 2969, 1617, 1572, 1532, 1469, 1405, 1354, 1318, 1246, 1227, 1190, 1165, 1125, 1072, 959, 919, 867, 818, 761, 725, 677, 587, 570, 545, 517, 465, 438, 424 cm⁻¹. ¹H NMR (399 MHz, CDCl₃/TMS): δ = 8.71 (s, 1H), 7.97 (d, J = 8.4 Hz, 1H), 7.90-7.86 (m, 2H), 7.77 (t, J= 7.2 Hz, 1H), 7.53 (d, J = 2.4 Hz, 1H), 7.50 (t, J = 7.2 Hz, 1H), 7.22 (d, J = 2.0 Hz, 1H), 3.75 (s, 2H), 2.62 (q, J = 7.6 Hz, 4H), 1.09 (t, J = 7.6 Hz, 6H) ppm. ¹³C NMR(100 MHz, CDCl₃/TMS): δ = 158.4, 155.4, 150.2, 133.4, 129.6, 129.0, 127.0, 124.1, 123.5, 122.2, 121.2, 121.0, 115.4, 57.2, 46.7, 11.4 ppm. HRMS (ESI⁺): calcd for [M+H]⁺, C₁₉H₂₂ClN₄O: 357.1477; found 357.1472. Anal. Calcd (%) for C₁₉H₂₁ClN₄O·0.03CHCl₃: C, 63.41; H, 5.88; N, 15.54. found: C, 63.78; H, 5.53; N, 15.19.

### [Example 18] Synthesis of YMSA-0707

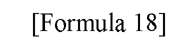

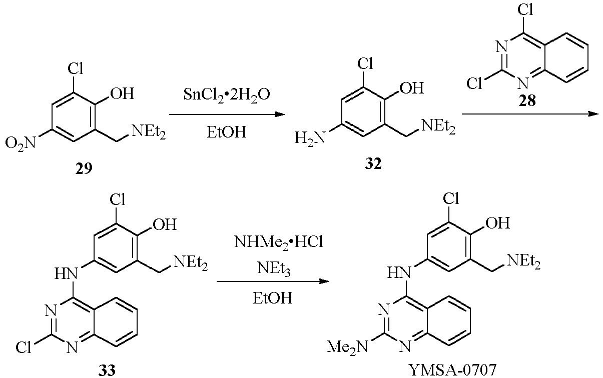

### 4-Amino-2-chloro-6-((diethylamino)methyl)phenol (32)

Compound 32 was obtained as a yellow oily substance by the same method as that in the synthesis of Compound 3 (0.33 g, 59%).

### 2-Chloro-4-((2-chloroquinazolin-4-yl)amino)-6-((diethylamino)methyl)phenol (33)

Compound 33 was obtained as a light yellow powder solid by the same method as that in the synthesis of YMSA-0635 (0.11 g, 55%). mp 90-99°C.

### YMSA-0707

YMSA-0707 was obtained as a light yellow solid by the same method as that in the synthesis of YMSA-0636 (38 mg, 46%). mp 176-180°C. IR (ATR): v = 3304, 3133, 3054, 2972, 2937, 1622, 1581, 1558, 1528, 1464, 1383, 1350, 1303, 1264, 1250, 1228, 1191, 1165, 1125, 1106, 1089, 1063, 1045, 1019, 996, 926, 875, 853, 807, 793, 780, 771, 742, 719, 663, 635, 584, 566, 549, 538, 508, 483, 426, 407 cm⁻¹. ¹H NMR (399 MHz, CDCl₃/TMS): δ = 7.76 (d, J = 2.8 Hz, 1H), 7.59 (d, J = 8.0 Hz, 1H), 7.52 (td, J = 7.4, 2.0 Hz, 2H), 7.23 (d, J = 2.4 Hz, 1H), 7.11-7.07 (m, 2H), 3.78 (s, 2H), 3.24 (s, 6H), 2.65 (q, J = 7.2 Hz, 4H), 1.13 (t, J= 7.2 Hz, 6H) ppm. ¹³C NMR (100 MHz, CDCl₃/TMS): δ = 159.4, 157.3, 153.1, 150.7, 132.8, 130.4, 126.2, 123.0, 122.4, 121.0, 120.4, 110.0, 56.9, 46.4, 37.2, 11.1 ppm. HRMS (FAB⁺): calcd for [M]⁺, C₂₁H₂₆ClN₅O:399.1826; found 399.1826. Anal. Calcd (%) for C₂₁H₂₆ClN₅O·0.2AcOEt: C, 62.71; H, 6.66; N, 16.77. found: C, 62.83; H, 6.41; N, 16.56.

### [Example 19] Synthesis of YMSA-0781

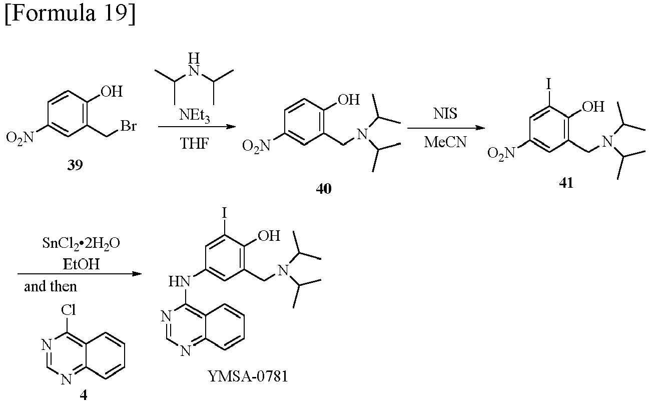

### 2-((Diisopropylamino)methyl)-4-nitrophenol (40)

Diisopropylamine (0.26 mL, 1.8 mmol) and Et₃N (0.42 mL, 3.0 mmol) were added to a THF (7 mL) solution of 2-hydroxy-5-nitrobenzyl bromide 39 (0.35 g, 1.5 mmol), and the reaction solution was heated under reflux for 30 minutes. The reaction solution was concentrated under reduced pressure, and the resulting residue was dissolved in AcOEt (20 mL), and washed with H₂O (20 mL × 3) and saturated saline water (20 mL). The organic phase was dried over Na₂SO₄, filtered, and concentrated under reduced pressure, giving Compound 40 as a yellow solid (0.33 g, 88%). mp 112-115°C.

### 2-((Diisopropylamino)methyl)-6-iodo-4-nitrophenol (41)

Compound 41 was obtained as a yellow solid by the same method as that in the synthesis of YMSA-0532 (0.24 g, 75%). mp 151-153°C.

### YMSA-0781

YMSA-0781 was obtained as a light yellow solid by the same method as that in the synthesis of YMSA-0464 (15 mg, 47%). mp 111-120°C. IR (ATR): v = 2968, 1619, 1571, 1530, 1498, 1465, 1390, 1369, 1315, 1239, 1192, 1169, 1118, 1073, 948, 914, 869, 819, 764, 735, 707. 680, 569, 551, 498, 467, 432, 419 cm⁻¹. ¹H NMR (399 MHz, CDCl₃/TMS): δ = 8.73

(s, 1H), 7.91 (d, J = 8.4 Hz, 1H), 7.86-7.78 (m, 3H), 7.55 (td, J = 7.4, 1.2 Hz, 1H), 7.41 (d, J = 2.8 Hz, 1H), 7.35 (brs, 1H), 3.85 (s, 2H), 3.21-3.15 (m, 2H), 1.15 (d, J= 6.8 Hz, 12H) ppm. ¹³C NMR (100 MHz, CDCl₃/TMS): δ = 157.9, 156.0, 155.2, 150.0, 133.0, 131.8, 129.9, 129.1, 126.7, 123.5, 122.7, 120.4, 115.0, 84.7, 49.1, 48.4, 19.8 ppm. HRMS (FAB⁺): calcd for [M+H]⁺, C₂₁H₂₆IN₄O:477.1151; found 477.1153. Anal. Calcd (%) for C₂₁H₂₅IN₄O·0.1H₂O: C, 52.75; H, 5.31; N, 11.72. found: C, 52.68; H, 5.47; N, 11.33.

### [Example 20] Synthesis of YMSA-0797

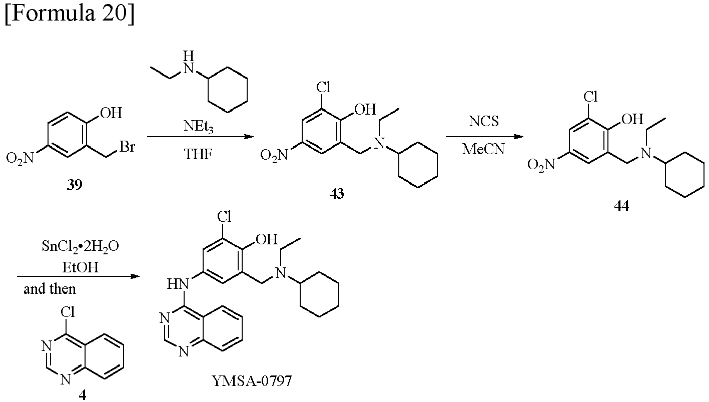

### 2-((Cyclohexyl(ethyl)amino)methyl)-4-nitrophenol (43)

Compound 43 was obtained as a yellow oily substance by the same method as the method of synthesizing Compound 40 (0.52 g, 100%).

### 2-Chloro-6-((cyclohexyl(ethyl)amino)methyl)-4-nitrophenol (44)

Compound 44 was obtained as a yellow oily substance by the same method as that in the synthesis of Compound 29 (92 mg, 37%).

### YMSA-0797

YMSA-0797 was obtained as a yellow solid by the same method as that in the synthesis of YMSA-0464 (35 mg, 35%). mp 80-85°C. IR (ATR): v = 2928, 2854, 1669, 1618, 1573, 1532, 1470, 1391, 1356, 1319, 1234, 1171, 1123, 1073, 967, 915, 868, 765, 680, 581, 533, 465, 429, 416 cm⁻¹. ¹H NMR (399 MHz, CDCl₃/TMS): δ = 8.72 (s, 1H), 7.91-7.88 (m, 2H), 7.81-7.79 (m, 2H), 7.55-7.53 (m, 3H), 3.87 (s, 2H), 2.66-2.64 (m, 3H), 1.89 (d, J= 12 Hz, 2H), 1.82 (d, J = 12 Hz, 2H), 1.65 (d, J = 13 Hz, 1H), 1.34-1.22 (m, 4H), 1.14 (t, J = 6.8 Hz, 3H), 0.89 (t, J = 6.8 Hz, 1H) ppm. ¹³C NMR(100 MHz, CDCl₃/TMS): δ = 157.9, 155.1, 152.5, 149.9, 143.6, 134.9, 133.0, 128.9, 126.7, 123.5, 121.7, 120.8, 120.5, 115.0, 58.9, 53.6, 43.9, 28.1, 26.1, 25.9, 13.0 ppm. HRMS (FAB⁺): calcd for [M+H]⁺, C₂₃H₂₈ClN₄O:411.1952; found 411.1950. Anal. Calcd (%) for C₂₃H₂₇ClN₄O·0.4H₂O: C, 66.07; H, 6.70; N, 13.40. found: C, 66.04; H, 6.64; N, 13.10.

### [Example 21] Synthesis of a variety of quinazoline derivatives

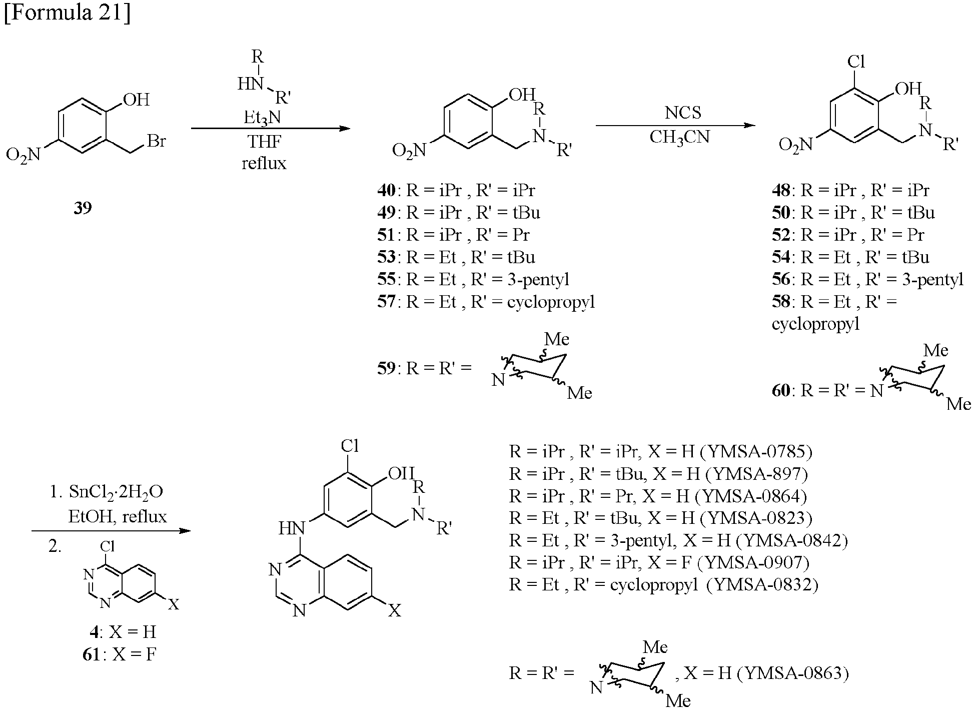

### 2-((Diisopropylamino)methyl)-6-chloro-4-nitrophenol (48)

N-chlorosuccinimide (203 mg, 1.52 mmol) was added to a solution of Compound 40 (319 mg, 1.26 mmol) dissolved in 10 mL of acetonitrile, followed by stirring at room temperature for 23 hours. After confirming the completion of the reaction, the solvent was distilled off under reduced pressure, and a 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 48 (241 mg, 67%) as a yellow solid. mp 154-157°C.

### YMSA-0785

Lead chloride dihydrate (0.18 g, 0.80 mmol) was added to a solution of Compound 48 (57 mg, 0.20 mmol) dissolved in 5 mL of ethanol, followed by stirring under reflux conditions for 1 hour. After the reaction solution was cooled to room temperature, 4-chloroquinazoline 4 (66 mg, 0.40 mmol) was added to the reaction solution, which was again stirred under reflux conditions for 3 hours. After confirming the completion of the reaction, the solvent was distilled off under reduced pressure. Then, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0785 (76 mg, quant.) as a yellow solid. mp 258°C (dec.). IR (ATR): v = 2971, 1619, 1590, 1569, 1528, 1472, 1446, 1421, 1390, 1355, 1315, 1292, 1250, 1195, 1147, 1119, 1074, 1016, 970, 918, 899, 872, 799, 787, 776, 678, 647, 579, 554, 526, 507, 465, 450, 428, 405 cm⁻¹. ¹H NMR (300 MHz, CDCl₃/TMS): δ = 8.74 (s, 1H), 7.93-7.90 (m, 1H), 7.84-7.78 (m, 2H), 7.59-7.52 (m, 2H), 7.31 (d, J = 2.1 Hz, 1H), 3.92 (s, 2H), 3.25-3.19 (m, 2H), 1.16 (d, J = 6.7 Hz, 12H) ppm. ¹³C NMR(100 MHz, CDCl₃/TMS): δ = 158.9, 155.7, 152.1, 150.7, 134.1, 131.2, 128.9, 127.3, 125.2, 124.0, 123.8, 123.0, 119.5, 116.2, 49.2(2C), 49.0, 20.5(4C) ppm. HRMS (ESI⁺): calcd for [M+H]⁺, C₂₁H₂₆ClN₄O:385.1790; found 385.1791. Anal. Calcd (%) for C₂₁H₂₆ClN₄O+ 0.25 H₂O: C 64.77, H 6.60, N 14.39; found: C 64.93, H 6.55, N 14.07.

### 2-((tert-Butyl(isopropyl)amino)methyl)-4-nitrophenol (49)

N-tert-butylisopropylamine (0.19 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, and were stirred under reflux conditions for 3 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 49 (0.18 g, 69%) as a yellow solid. mp 142-147°C.

### 2-((tert-Butyl(isopropyl)amino)methyl)-6-chloro-4-nitrophenol (50)

N-chlorosuccinimide (101 mg, 0.76 mmol) was added to a solution of Compound 49 (183 mg, 0.69 mmol) dissolved in 5 mL of acetonitrile, followed by stirring at room temperature for 23 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 50 (130 mg, 63%) as a yellow solid. mp 167-172°C.

### YMSA-0897

Lead chloride dihydrate (0.18 g, 0.80 mmol) was added to a solution of Compound 50 (60 mg, 0.20 mmol) dissolved in 2 mL of ethanol, followed by stirring under reflux conditions for 1 hour. After the reaction solution was cooled to room temperature, 4-chloroquinazoline 4 (66 mg, 0.40 mmol) was added to the reaction solution, which was again stirred under reflux conditions for 2 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0897 (71 mg, 89%) as a yellow solid. mp 170°C (Dec.). IR (ATR): v = 2970, 1732, 1620, 1601, 1572, 1537, 1500, 1476, 1431, 1414, 1392, 1372, 1356, 1323, 1309, 1288, 1239, 1195, 1172, 1146, 1125, 1077, 1027, 969, 916, 886, 869, 813, 797, 786, 734, 699, 679, 668, 584, 573, 520, 497, 468, 426, 417 cm⁻¹. ¹H NMR (300 MHz, CDCl₃/TMS): δ = 8.74 (s, 1H), 7.93-7.90 (m, 1H), 7.83-7.78 (m, 2H), 7.58-7.52 (m, 2H), 7.46 (d, J = 2.7 Hz, 1H), 4.02 (s, 2H), 3.53 (sep, J = 6.6 Hz, 1H), 1.22 (s, 9H), 1.17 (d, J = 6.6 Hz, 6H) ppm. ¹³C NMR (100 MHz, CDCl₃/TMS): δ = 158.9, 155.8, 152.1, 150.7, 134.1, 131.1, 128.9, 127.9, 127.3, 124.0, 123.1, 121.4, 119.7, 116.2, 58.3, 48.7, 46.8, 28.3(3C), 22.2(2C) ppm. HRMS (ESI⁺): calcd for [M+H]⁺, C₂₂H₂₈ClN₄O: 399.1946; found 399.1945. Anal. Calcd (%) for C₂₂H₂₇ClN₄O + 0.25 CHCl₃: C 62.33, H 6.41, N 13.07; found: C 62.59, H 6.51, N 12.68.

### 2-((Isopropyl(propyl)amino)methyl)-4-nitrophenol (51)

N-isopropylpropylamine (0.12 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 1 hour. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 51 (0.22 g, 88%) as a yellow oil.

### 2-((Isopropyl(propyl)amino)methyl)-6-chloro-4-nitrophenol (52)

N-chlorosuccinimide (175 mg, 1.3 mmol) was added to a solution of Compound 51 (221 mg, 0.88 mmol) dissolved in 5 mL of acetonitrile, followed by stirring at room temperature for 23 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 52 (132 mg, 52%) as a yellow solid. mp 67-68°C.

### YMSA-0864

Lead chloride dihydrate (0.24 g, 1.04 mmol) was added to a solution of Compound 52 (75 mg, 0.26 mmol) dissolved in 3 mL of ethanol, followed by stirring under reflux conditions for 1 hour. After the reaction solution was cooled to room temperature, 4-chloroquinazoline 4 (86 mg, 0.52 mmol) was added to the reaction solution, which was again stirred under reflux conditions for 2 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0864 (80 mg, 79%) as a yellow solid. mp 72-78°C. IR(ATR): v = 2967, 1620, 1574, 1534, 1499, 1393, 1358, 1319, 1237, 1168, 1125, 1074, 1027, 959, 918, 871, 767, 681, 583, 504, 466, 430, 417, 407 cm⁻¹. ¹H NMR (300 MHz, CDCl₃/TMS): δ = 8.74 (s, 1H), 7.93-7.90 (m, 1H), 7.83-7.78 (m, 2H), 7.58-7.53 (m, 2H), 7.30 (d, J = 2.7 Hz, 1H), 7.21 (br, 1H), 3.85 (s, 2H), 3.13 (sep, J= 6.6 Hz, 1H), 2.48 (t, J = 7.5 Hz, 2H), 1.61 (sext, J = 7.5 Hz, 2H),1.12 (d, 6H), 0.91 (t, J = 7.5 Hz, 3H) ppm. ¹³C NMR (100 MHz, CDCl₃/TMS): δ = 158.9, 155.7, 151.8, 150.7, 134.1, 131.4, 128.9, 127.3, 124.8, 124.0, 123.9, 123.1, 119.6, 116.2, 54.1, 51.6, 50.6, 21.6 (2C), 18.1, 12.8 ppm. HRMS (ESI⁺): calcd for [M+2H]⁺, C₂₁H₂₇ClN₄O: 193.0931; found 193.0931. Anal. Calcd (%) for C₂₁H₂₆ClN₄O + 0.25H₂O: C 64.77, H 6.60, N 14.39; found: C 64.42, H 6.53, N 14.14.

### 2-((tert-Butyl(ethyl)amino)methyl)-4-nitrophenol (53)

N-tert-butylethylamine (0.17 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 3 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 53 (0.23 g, 91%) as a yellow solid. mp 104-109°C.

### 2-((tert-Butyl(ethyl)amino)methyl)-6-chloro-4-nitrophenol (54)

N-chlorosuccinimide (145 mg, 1.1 mmol) was added to a solution of Compound 53 (229 mg, 0.91 mmol) dissolved in 5 mL of acetonitrile, followed by stirring at room temperature for 13 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 54 (124 mg, 48%) as a yellow solid. mp 145-155°C.

### YMSA-0823

Lead chloride dihydrate (0.17 g, 0.76 mmol) was added to a solution of Compound 54 (54 mg, 0.19 mmol) dissolved in 3 mL of ethanol, followed by stirring under reflux conditions for 1 hour. After the reaction solution was cooled to room temperature, 4-chloroquinazoline 4 (62 mg, 0.38 mmol) was added to the reaction solution, which was again stirred under reflux conditions for 1.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating YMSA-0823 (66 mg, 91%) as a yellow solid. mp 155°C (dec.). IR (ATR): v = 2963, 1619, 1598, 1573, 1533, 1500, 1472, 1411, 1391, 1356, 1321, 1288, 1239, 1197, 1123, 1076, 1028, 996, 952, 916, 884, 869, 820, 788, 736, 704, 679, 666, 583, 546, 524, 510. 496, 460, 427, 418 cm⁻¹. ¹H NMR (300 MHz, CDCl₃/TMS): δ = 8.74 (s, 1H), 7.93-7.90 (m, 1H), 7.84-7.78 (m, 2H), 7.58-7.50 (m, 2H), 7.30 (d, J = 1.5 Hz, 1H), 3.99 (s, 2H), 2.75 (br, 2H), 1.24 (s, 9H), 1.14 (t, J = 7.5 Hz, 3H) ppm. ¹³C NMR(100MHz, CDCl₃/TMS): δ =158.9, 155.7, 152.2, 150.7, 134.0, 131.1, 128.9, 127.3, 126.1, 123.9, 123.6, 121.9, 119.8, 116.2, 57.3, 53.4, 45.1, 27.2 (3C), 15.4 ppm. HRMS (ESI⁺): calcd for [M+H]⁺, C₂₁H₂₆ClN₄O: 385.1790; found 385.1789. Anal. Calcd (%) for C₂₁H₂₆ClN₄O + 0.02CHCl₃: C 65.19, H 6.51, N 14.47; found: C 65.18, H 6.53, N 14.51.

### 2-((Ethyl(pentan-3-yl)amino)methyl)-4-nitrophenol (55)

N-ethyl(-3-pentyl)amine (0.17 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 13 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 55 (0.28 g, quant.) as a yellow solid. mp 44-45°C.

### 2-((Ethyl(pentan-3-yl)amino)methyl)-6-chloro-4-nitrophenol (56)

N-chlorosuccinimide (160 mg, 1.2 mmol) was added to a solution of Compound 55 (266 mg, 1.0 mmol) dissolved in 4 mL of acetonitrile, followed by stirring at room temperature for 15 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 56 (180 mg, 59%) as a yellow solid. mp 58-68°C.

### YMSA-0842

Lead chloride dihydrate (0.53 g, 2.4 mmol) was added to a solution of Compound 56 (180 mg, 0.59 mmol) dissolved in 6 mL of ethanol, followed by stirring under reflux conditions for 1 hour. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was dissolved in 2 mL of ethanol, 4-chloroquinazoline 4 (62 mg, 0.38 mmol) was added, and these were stirred under reflux conditions for 6.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating YMSA-0842 (82 mg, 82%) as a yellow solid. mp 75-80°C. IR (ATR): v = 2964, 2933, 2876, 1621, 1601, 1573, 1532, 1500, 1404, 1357, 1320, 1287, 1239, 1184, 1161, 1122, 1074, 1029, 972, 944, 919, 868, 818, 766, 737, 720, 679, 661, 581, 567, 524, 497, 466, 425 cm⁻¹. ¹H NMR (300 MHz, CDCl₃/TMS): δ = 8.74 (s, 1H), 7.93-7.90 (m, 1H), 7.84-7.78 (m, 2H), 7.58-7.53 (m, 2H), 7.30 (d, J= 1.5 Hz, 1H), 3.87 (s, 2H), 2.64-2.51 (m, 3H), 1.68-1.56 (m, 2H), 1.49-1.39 (m, 2H), 1.17 (t, J = 6.9 Hz, 3H), 1.00 (t, J = 7.5 Hz, 6H) ppm. ¹³C NMR (100 MHz, CDCl₃/TMS): δ = 158.9, 155.7, 151.5, 150.7, 134.1, 131.6, 128.9, 127.3, 125.2, 124.0, 123.2, 119.6, 116.2, 64.0, 54.0, 44.0, 22.6(2C), 14.1, 13.0(2C) ppm. HRMS (ESI⁺): calcd for [M+H]⁺, C₂₂H₂₈ClN₄O: 399.1946; found 399.1945. Anal. Calcd (%) for C₂₂H₂₇ClN₄O + 0.5H₂O: C 64.77, H 6,92, N 13.73; found: C 64.90, H 6.96, N 13.70.

### 2-((Ethyl(cyclopropyl)amino)methyl)-4-nitrophenol (57)

N-isopropylpropylamine (0.17 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 1 hour. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 57 (0.22 g, 88%) as a yellow oil.

### 2-((Ethyl(cyclopropyl)amino)methyl)-6-chloro-4-nitrophenol (58)

N-chlorosuccinimide (175 mg, 1.3 mmol) was added to a solution of Compound 57 (266 mg, 1.0 mmol) dissolved in 5 mL of acetonitrile, followed by stirring at room temperature for 23 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 58 (132 mg, 52%) as a yellow amorphous substance.

### YMSA-0832

Lead chloride dihydrate (0.24 g, 1.04 mmol) was added to a solution of Compound 58 (75 mg, 0.26 mmol) dissolved in 3 mL of ethanol, followed by stirring under reflux conditions for 1 hour. After the reaction solution was cooled to room temperature, 4-chloroquinazoline 4 (86 mg, 0.52 mmol) was added to the reaction solution, which was again stirred under reflux conditions for 2 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0832 (80 mg, 79%) as a yellow solid. mp 140-145°C. IR (ATR): v = 3061, 2975, 2480, 1620, 1573, 1535, 1499, 1482, 1464, 1447, 1357, 1320, 1297, 1262, 1249, 1224, 1185, 1125, 1075, 1031, 934, 919, 870, 852, 817, 796, 784, 769, 722, 677, 641, 596, 574, 516, 465, 432, 420, 411 cm⁻¹. ¹H NMR (300 MHz, CDCl₃/TMS): δ = 8.75 (s, 1H), 7.93-7.91 (d, J= 7.8 Hz, 1H), 7.84-7.78 (m, 2H), 7.60-7.54 (m, 2H), 7.30 (d, J = 2.7 Hz, 1H), 7.25 (br, 1H), 4.02 (s, 2H), 2.76 (q, J = 6.9 Hz, 2H), 1.99-1.92 (m, 1H), 1.19 (t, J = 6.9 Hz, 2H), 0.66-0.63 (m, 4H) ppm. ¹³C NMR (75 MHz, CDCl₃/TMS): δ = 157.7, 155.0, 151.4, 150.0, 132.9, 129.5, 129.0, 126.6, 123.4(2C), 121.3, 120.6, 120.2, 114.9, 58.7, 48.6, 36.3, 10.7, 6.5(2C) ppm. HRMS (ESI⁺): calcd for [M+2H]⁺, C₂₀H₂₂ClN₄O: 369.1477; found 369.1478. Anal. Calcd (%) for C₂₀H₂₁ClN₄O + 0.2 MeOH: C 64.65, H 5.86, N 14.93; found: C 64.96, H 5.83, N 14.56.

### 2-((3,5-Dimethylpiperidin-1-yl)methyl)-4-nitrophenol (59)

3,5-Dimethylpiperidine (0.16 mL, 1.2 mmol) and triethylamine (0.31 mL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 2.5 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 59 (0.28 g, quant.) as a yellow oil.

### 2-((3,5-Dimethylpiperidin-1-yl)methyl)-6-chloro-4-nitrophenol (60)

N-chlorosuccinimide (160 mg, 1.2 mmol) was added to a solution of Compound 59 (274 mg, 1.0 mmol) dissolved in 5 mL of acetonitrile, followed by stirring at room temperature for 15 hours. After confirming the completion of the reaction, the solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 60 (68 mg, 23%) as a yellow oil.

### YMSA-0863

Lead chloride dihydrate (0.21 g, 0.91 mmol) was added to a solution of Compound 60 (68 mg, 0.23 mmol) dissolved in 3 mL of ethanol, followed by stirring under reflux conditions for 1.5 hours. After the reaction solution was cooled to room temperature, 4-chloroquinazoline 4 (75 mg, 0.38 mmol) was added to the reaction solution, which was again stirred under reflux conditions for 2 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating YMSA-0863 (78 mg, 86%) as a yellow solid. mp 80-85°C. IR (ATR): v = 2953, 1620, 1573, 1534, 1499, 1405, 1357, 1317, 1286, 1236, 1124, 1067, 1027, 996, 961, 918, 869, 820, 765, 728, 680, 591, 536, 499, 467, 431, 418 cm⁻¹. ¹H NMR (300 MHz, CDCl₃/TMS): δ = 8.74 (s, 1H), 7.93-7.90 (m, 1H), 7.84-7.81 (m, 2H), 7.61-7.53 (m, 2H), 7.29 (d, J = 2.4 Hz, 1H), 3.74 (s, 2H), 2.97-2.94 (m, 2H), 1.80-1.76 (m, 2H), 1.70-1.62 (m, 2H), 1.27-1.26 (m, 2H), 0.87 (d, J= 6.3 Hz, 6H) ppm. ¹³C NMR (100 MHz, CDCl₃/TMS): δ = 157.9, 155.1, 151.8, 150.0, 133.0, 129.3, 129.0, 126.7, 123.7, 122.9, 121.8, 120.8, 120.5, 115.0, 62.2, 61.7, 60.6, 41.6, 38.3, 31.2, 22.7, 19.4, 14.2 ppm. HRMS (ESI⁺): calcd for [M+H]⁺, C₂₂H₂₆ClN₄O: 397.1795; found 397.1784. Anal. Calcd (%) for C₂₂H₂₅ClN₄O + 0.5 H₂O: C 65.10, H 6.46, N 13.80; found: C 65.02, H 6.48, N 13.50.

### YMSA-0907

Lead chloride dihydrate (0.18 g, 0.80 mmol) was added to a solution of Compound 48 (57 mg, 0.2 mmol) dissolved in 3 mL of ethanol, followed by stirring under reflux conditions for 1 hour. After the reaction solution was cooled to room temperature, 4-chloro-7-fluoroquinazoline 61 (73 mg, 0.38 mmol) was added to the reaction solution, which was again stirred under reflux conditions for 5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating YMSA-0907 (74 mg, 91%) as a yellow solid. mp 170-175°C. IR (ATR): v = 2974, 1625, 1600, 1576, 1529, 1457, 1444, 1425, 1394, 1344, 1322, 1287, 1202, 1149, 1113, 1072, 1010, 962, 910, 839, 808, 788, 722, 672, 651, 602, 581, 556, 533, 493, 478, 439, 422 cm⁻¹. ¹H NMR (300 MHz, CDCl₃/TMS): δ = 8.70 (s, 1H), 7.87-7.82 (m, 1H), 7.56-7.49 (m, 2H), 7.34-7.28 (m, 2H), 7.20 (s, 1H), 3.92 (s, 2H), 3.21 (sep, J = 6.6 Hz, 2H), 1.16 (d, J = 6.6 Hz, 12H) ppm. ¹³C NMR (75 MHz, CDCl₃/TMS): δ = 157.7, 156.1, 152.7, 128.6, 123.7, 123.5, 123.1, 123.0, 121.6, 120.6, 116.4, 116.1, 113.2, 112.9, 48.8, 48.3 (2C), 19.6 (4C) ppm. HRMS (ESI⁺): calcd for [M+H]⁺, C₂₁H₂₅ClFN₄O: 403.1695; found 403.1695. Anal. Calcd (%) for C₂₁H₂₄ClN₄OF: C 62.66, H 6.01, N 13.93; found: C 62.35, H 5.86, N 13.65.

### [Example 22] Synthesis of YMSA-0808 and YMSA-0644

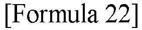

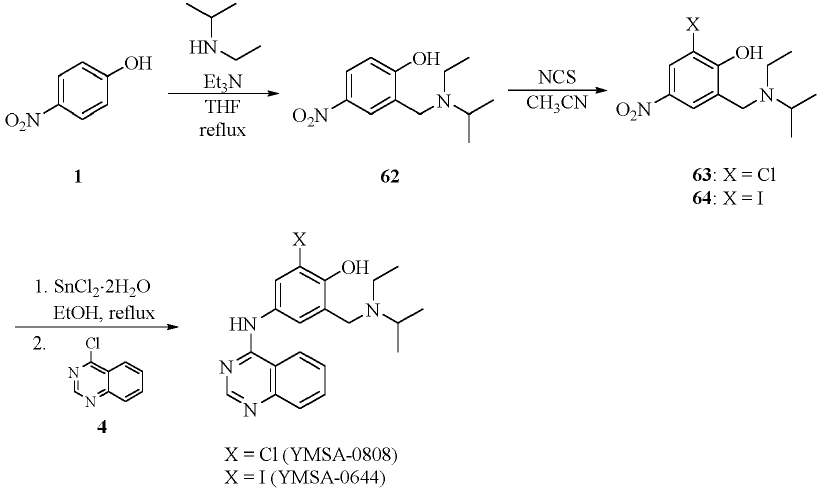

### 2-((Ethyl(isopropyl)amino)methyl)-4-nitrophenol (62)

Ethylisopropylamine (1.80 mL, 15.0 mmol) and 37% formaldehyde aqueous solution (1.1 mL, 15.0 mmol) were added to a solution of 4-nitrophenol 1 (1.39 g, 10.00 mmol) dissolved in 8 mL of ethanol, followed by stirring under reflux conditions for 16 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 62 (414 mg, 15%) as a yellow solid. mp 65-67°C.

### 2-((Ethyl(isopropyl)amino)methyl)-6-chloro-4-nitrophenol (63)

N-chlorosuccinimide (0.46 g, 2.5 mmol) was added to a solution of Compound 62 (0.41 g, 1.74 mmol) dissolved in 5 mL of acetonitrile, followed by stirring at room temperature for 16 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate:methanol = 9:1), thus isolating Compound 63 (172 mg, 36%) as a yellow solid. mp 158-164°C.

### 2-((Ethyl(isopropyl)amino)methyl)-6-iodo-4-nitrophenol (64)

N-iodosuccinimide (0.21 g, 0.94 mmol) was added to a solution of Compound 62 (0.18 g, 0.63 mmol) dissolved in 5 mL of acetonitrile, followed by stirring at room temperature for 22 hours. After confirming the completion of the reaction, the solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate:methanol = 9:1), thus isolating Compound 64 (213 mg, 83%) as a yellow solid. mp 145-155°C.

### YMSA-0808

Lead chloride dihydrate (0.57 g, 2.52 mmol) was added to a solution of Compound 63 (172 mg, 0.63 mmol) dissolved in 6 mL of ethanol, followed by stirring under reflux conditions for 1 hour. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was dissolved in 1 mL of ethanol, 4-chloroquinazoline 4 (16.5 mg, 0.10 mmol) was added, and these were stirred under reflux conditions for 3 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate:methanol = 9:1), thus isolating YMSA-0808 (21.5 mg, 58%) as a yellow solid. mp 70-75°C. IR (ATR): v = 2969, 1620, 1573, 1532, 1499, 1393, 1356, 1319, 1236, 1192, 1170, 1123, 1073, 1023, 966, 918, 869, 766, 720, 681, 581, 565, 535, 492, 465, 427, 409 cm⁻¹. ¹H NMR (300 MHz, CDCl₃/TMS): δ = 8.73 (s, 1H), 7.92-7.77 (m, 3H), 7.56-7.51 (m, 2H), 7.43 (br, 1H), 3.82 (s, 2H), 3.15 (sep, J = 6.6 Hz, 1H), 2.59 (q, J = 7.2 Hz, 2H), 1.18-1.11 (m, 9H) ppm. ¹³C NMR (100 MHz, CDCl₃/TMS): δ = 158.9, 155.7, 152.0, 150.7, 134.1, 131.4, 128.9, 127.3, 124.7, 124.0, 123.1, 119.6, 116.2, 53.4, 50.4, 43.9, 18.2, 14.1 ppm. HRMS (ESI⁺): calcd for [M+2H]⁺, C₂₀H₂₅ClN₄O: 186.0853; found 186.0853. Anal. Calcd (%) for C₂₀H₂₃ClN₄O + 0.75 CH₃OH: C 63.11, H 6.64, N 14.19; found: C 63.19, H 6.28, N 14.02.

### YMSA-0644

Lead chloride dihydrate (0.46 g, 2.05 mmol) was added to a solution of Compound 64 (210 mg, 0.51 mmol) dissolved in 10 mL of ethanol, followed by stirring under reflux conditions for 2 hours. After the reaction solution was cooled to room temperature, 4-chloroquinazoline 4 (170 mg, 1.03 mmol) was added to the reaction solution, which was again stirred under reflux conditions for 5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0644 (173.2 mg, 73%) as a yellow solid. mp 155°C (dec.). IR (ATR): v = 2972, 1620, 1574, 1536, 1499, 1457, 1409, 1384, 1354, 1321, 1287, 1249, 1227, 1171, 1114, 1064, 995, 960, 918, 880, 858, 809, 786, 767, 750, 719, 640, 592, 583, 570, 556, 513, 503, 464, 436, 421 cm⁻¹. ¹H NMR (300 MHz, CDCl₃/TMS): δ = 8.73 (s, 1H), 7.93-7.77 (m, 4H), 7.58-7.52 (m, 1H), 7.41 (d, J = 2.7 Hz, 1H), 3.81 (s, 2H), 3.15 (sep, J= 6.6 Hz, 1H), 2.60 (q, J = 7.2 Hz, 2H), 1.18-1.11 (m, 9H) ppm. ¹³C NMR (75 MHz, CDCl₃/TMS): δ = 157.9, 155.7, 155.0, 149.8, 132.9, 132.0, 129.9, 128.7, 126.5, 123.7, 122.1, 120.5, 114.9, 84.6, 52.9, 49.4, 43.1, 17.3(2C), 12.8 ppm. HRMS (ESI⁺): calcd for [M+2H]⁺, C₂₀H₂₅IN₄O: 232.0531; found 232.0531. Anal. Calcd (%) for C₂₀H₂₃IN₄O + 0.25 H₂O: C 51.46, H 5.07, N 12.00; found: C 51.56, H 4.96, N 11.72.

### [Example 23] Synthesis of compounds (YMSA-0843 and YMSA-0850) in which a hydroxy group is present at the 3-position in place of the 4-position of the phenylamino group substituting the 4-position of the quinazoline ring in formula (I)

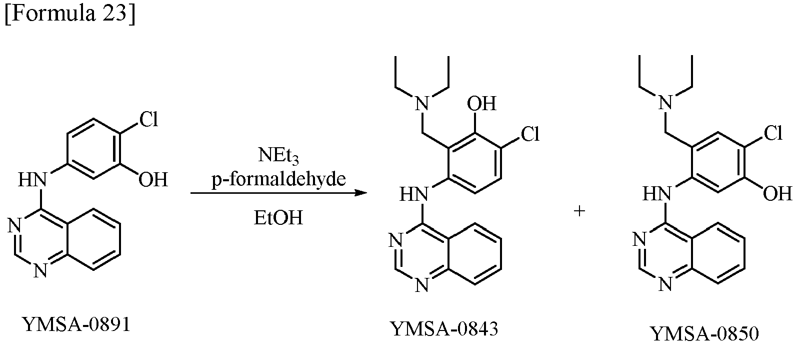

Diethylamine (82 µL, 0.8 mmol) and 37% formaldehyde aqueous solution (60 µL, 0.8 mmol) were added to a solution of YMSA-0891 (54 mg, 0.2 mmol) dissolved in 8 mL of ethanol, followed by stirring under reflux conditions for 5 hours. After confirming the completion of the reaction, the solvent was distilled off under reduced pressure. The resulting mixture was purified by silica gel column chromatography (ethyl acetate), thus isolating YMSA-0843 (18.6 mg, 26%) and YMSA-0850 (35.6 mg, 50%) as a yellow solid. mp 195°C (dec.). IR (ATR): v = 2963, 2923, 2838, 2580, 1613, 1579, 1558, 1538, 1500, 1415, 1375, 1359, 1330, 1314, 1282, 1241, 1210, 1165, 1151, 1125, 1080, 1059, 1038, 977, 932, 886, 846, 820, 799, 779, 707, 685, 674, 646, 596, 567, 539, 514, 502, 468, 453, 426 cm⁻¹. ¹H NMR (300 MHz, CDCl₃/TMS): δ = 8.69 (s, 1H), 7.94-7.91 (m, 2H), 7.84-7.79 (m, 1H), 7.59-7.54 (m, 1H), 7.29 (d, J = 8.4 Hz, 1H), 3.84 (s, 2H), 2.62 (q, J = 7.2 Hz, 4H), 1.12 (t, J = 7.2 Hz, 6H) ppm. ¹³C NMR (75 MHz, CDCl₃/TMS): δ = 158.6, 155.1, 153.7, 150.0, 136.6, 133.0, 128.9, 128.4, 126.6, 121.2, 118.3, 118.1, 116.1, 115.4, 51.4, 46.5(2C), 11.0(2C) ppm. HRMS (ESI⁺): calcd for [M+H]⁺, C₁₉H₂₂N₄O: 357.1477; found 357.1477. Anal. Calcd (%) for C₁₉H₂₁N₄O + 0.25 EtOAc: C 63.40, H 6.12, N 14.79; found: C 63.72, H 6.22, N 14.58. YMSA-0850:mp 165-170°C. IR (ATR): v = 2969, 1605, 1577, 1530, 1499, 1459, 1413, 1386, 1366, 1307, 1212, 1191, 1164, 1129, 1085, 1020, 1036, 963, 920, 867, 813, 761, 722, 653, 617, 569, 526, 506, 458, 424, 413 cm⁻¹. ¹H NMR (300 MHz, CDCl₃/TMS): δ = 9.18 (s, 1H), 8.32 (s,1H), 7.97 (d, J = 8.4 Hz, 1H), 7.89 (d, J = 7.5 Hz, 1H), 7.81-7.76 (m, 1H), 7.55-7.50 (m, 1H), 7.15 (s, 1H), 3.70 (s, 2H), 2.68 (q, J = 7.2 Hz, 4H), 1.13 (t, J= 7.2 Hz, 6H) ppm. ¹³C NMR (75 MHz, CDCl₃/TMS): δ = 157.6, 155.8, 152.6, 149.1, 138.3, 133.1, 130.5, 127.9, 126.6, 121.4, 119.5, 116.1, 114.6, 109.6, 57.0, 46.1, 11.0ppm. HRMS (ESI⁺): calcd for [M+H]⁺, C19H22N4O: 357.1477; found 357.1477. Anal. Calcd (%) for C₁₉H₂₁N₄O + 0.25 H₂O: C 63.15, H 6.00, N 15.50; found: C 63.05, H 5.83, N 15.64.

### [Example 24] Synthesis of YMSA-0650

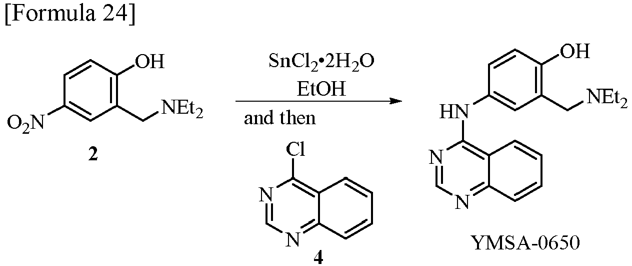

Lead chloride dihydrate (0.46 g, 2.05 mmol) was added to a solution of Compound 2 (57 mg, 0.20 mmol) dissolved in 3 mL of ethanol, followed by stirring under reflux conditions for 1 hour. After the reaction solution was cooled to room temperature, 4-chloroquinazoline 4 (72.8 mg, 0.4 mmol) was added to the reaction solution, which was again stirred under reflux conditions for 4.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating YMSA-0650 (74 mg, 91%) as a yellow solid. mp 70-75°C. IR (ATR): v = 3263, 3056, 2975, 2826, 1616, 1604, 1567, 1535, 1457, 1441, 1409, 1386, 1349, 1322, 1300, 1255, 1223, 1195, 1169, 1155, 1120, 1112, 1087, 1066, 1054, 1026, 998, 978, 945, 911, 894, 875, 832, 818, 798, 786, 765, 677, 632, 579, 561, 524, 499, 474, 466, 445, 433, 423 cm⁻¹. ¹H NMR (300 MHz, CDCl₃/TMS): δ = 8.71 (s, 1H), 7.91-7.76 (m, 3H), 7.57-7.51 (m, 1H), 7.38-7.30 (m, 3H), 6.85 (d, J= 8.4 Hz, 1H), 3.82 (s, 2H), 2.63 (q, J = 6.9 Hz, 4H), 1.13 (t, J = 7.2 Hz, 6H) ppm. ¹³C NMR (75 MHz, CDCl₃/TMS): δ = 158.0, 156.0, 155.2, 149.9, 132.7, 129.1, 128.8, 126.4, 123.5(2C), 122.5, 120.5, 116.5, 115.0, 56.9, 46.3(2C), 11.2(2C) ppm. HRMS (ESI⁺): calcd for [M+2H]⁺, C₁₉H₂₄N₄O: 162.0970; found 162.0970. Anal. Calcd (%) for C₁₉H₂₂N₄O + 0.1 H₂O: C 70.39, H 6.90, N 17.28; found: C 70.35, H 6.78, N 17.25.

### [Example 25] Synthesis of YMSA-0828

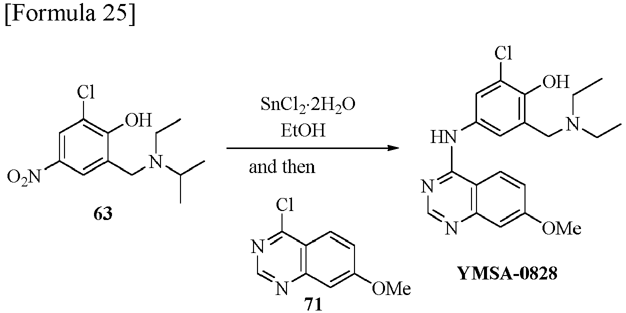

Lead chloride dihydrate (0.25 g, 1.1 mmol) was added to a solution of Compound 63 (0.10 g, 0.37 mmol) dissolved in 4 mL of ethanol, followed by stirring under reflux conditions for 3 hours. After the reaction solution was cooled to room temperature, 4-chloro-7-methoxyquinazoline 71 (72 mg, 0.37 mmol) was added to the reaction solution, which was again stirred under reflux conditions for 12 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate:methanol =30:1), thus isolating YMSA-0828 (32 mg, 21%) as a yellow solid. mp 163-165°C. IR(ATR): v = 3371, 2974, 2921, 1621, 1604, 1579, 1533, 1497, 1469, 1452, 1416, 1386, 1372, 1340, 1305, 1243, 1227, 1193, 1172, 1126, 1068, 1024, 994, 952, 929, 909, 893, 875, 846, 831, 796, 779, 753, 722, 712, 678, 660, 590, 542, 517, 422, 413 cm⁻¹. ¹H NMR (300 MHz, CDCl₃/TMS): δ = 8.66 (s, 1H), 7.72 (d, J = 9.3 Hz, 1H), 7.52 (d J = 2.4, 1H), 7.26-7.23 (m, 2H), 7.16-7.12 (m, 2H), 3.95 (s, 3H), 3.84 (s, 2H), 3.20-3.11 (m, 1H), 2.60 (q, J = 6.9, 2H), 1.19-1.11 (m, 9H) ppm. ¹³C NMR (100 MHz, CDCl₃/TMS): δ = 163.3, 157.7, 155.7, 152.3, 152.2, 129.3, 123.7, 123.3, 122.2, 121.9, 120.7, 118.7, 109.3, 107.4, 55.8, 53.0, 49.6, 43.3, 29.8, 17.4, 13.1 ppm. HRMS (FAB⁺): calcd for [M+H]⁺, C₂₁H₂₆ClN₄O₂: 401.1744; found 401.1741. Anal. Calcd (%) for C₂₁H₂₅ClN₄O₂·0.55H₂O: C, 61.40; H, 6.40; N, 13.64. found: C, 61.50; H, 6.31; N, 13.35.

### [Example 26] Synthesis of YMSA-0628

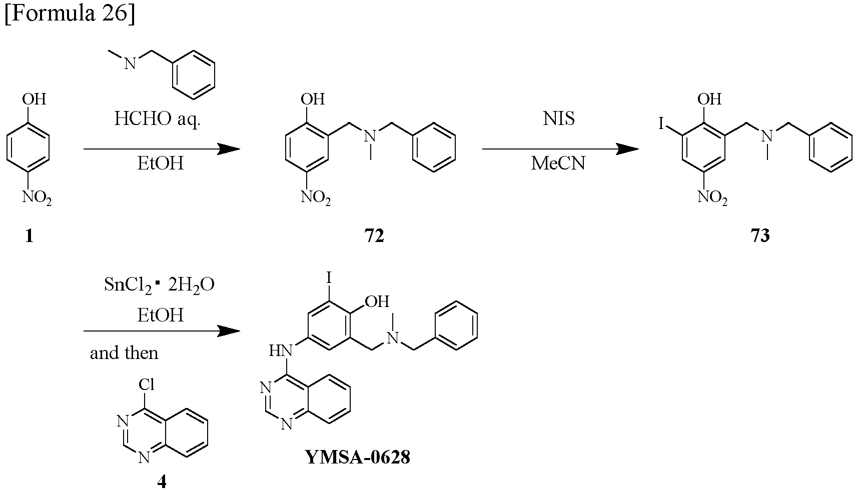

### 2-((Benzyl(methyl)amino)methyl)-4-nitrophenol (72)

N-methylbenzylamine (1300 µE, 10.0 mmol) and 37% formaldehyde aqueous solution (1020 µL, 12.5 mmol) were added to a solution of 1 (346.9 mg, 2.49 mmol) dissolved in 25 mL of ethanol, followed by stirring under reflux conditions for 46 hours. After confirming the completion of the reaction, the solvent was distilled off under reduced pressure. Thereafter, saturated ammonium chloride aqueous solution was added, followed by extraction with ethyl acetate. The organic phase was washed with saturated saline water, and dried over sodium sulfate. Solids were removed by filtration, and the solvent was distilled off under reduced pressure. Chloroform was added to the resulting residue, followed by back-extraction with 1N hydrochloric acid aqueous solution. Saturated sodium hydrogen carbonate aqueous solution was added to the aqueous phase, and the pH was adjusted to 8 by neutralization, followed by extraction with chloroform. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate (+1% AcOH) = 4:1 to 1:3), thus isolating Compound 72 (205.6 mg, 30%) as a light yellow solid. mp 80-81°C.

### 2-((Benzyl(methyl)amino)methyl)-6-iodo-4-nitrophenol (73)

N-iodosuccinimide (98.3 mg, 0.43 mmol) was added to a solution of Compound 72 (105.9 mg, 0.38 mmol) dissolved in 1.9 mL of acetonitrile, followed by stirring at 0 °C for 1.5 hours. After the reaction solution was diluted with methylene chloride, the organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate =2:1), thus isolating Compound 73 (117.4 mg, 76%) as a yellow solid. mp 39-40°C.

### YMSA-0628

YMSA-0628 was obtained as a light yellow powder solid by the same method as that in the synthesis of YMSA-0828 (84.6 mg, 76%). mp 76-77°C. IR (ATR): v = 2847, 1618, 1571, 1527, 1497, 1453, 1417, 1391, 1356, 1315, 1239, 1118, 1073, 1016, 984, 914, 850, 818, 743, 698, 679, 613, 566, 494, 451, 465, 433, 411 m⁻¹. ¹H NMR (300 MHz, CDCl₃/TMS): δ= 8.73 (s, 1H), 7.93-7.88 (m, 2H), 7.80 (t, J =7.5 Hz, 2H), 7.56 (t, J = 6.9 Hz, 1H), 7.48 (s, 1H), 7.37-7.22 (m, 5H), 3.78 (s, 2H), 3.67 (s, 2H), 2.28 (s, 3H) ppm. ¹³C NMR (100 MHz, DMSO-d₆/TMS): δ= 158.8, 155.7, 154.5, 150.6, 137.8, 134.0, 132.8, 132.7, 130.4, 129.7, 128.9, 128.8, 127.3, 124.9, 123. 9, 123.0, 116.1, 85.0, 61.3, 60.6, 41.5 ppm. HRMS (FAB⁺): calcd for [M+H]⁺, C₂₃H₂₂N₄OI:497.0838; found 497.0838. Anal. Calcd (%) for C₂₃H₂₁IN₄O·0.1CHCl₃:C, 54.59; H, 4.18; N,11.02. found: C, 54. 47; H, 4.33; N 11.05.

### [Example 27] Synthesis of YMSA-0908

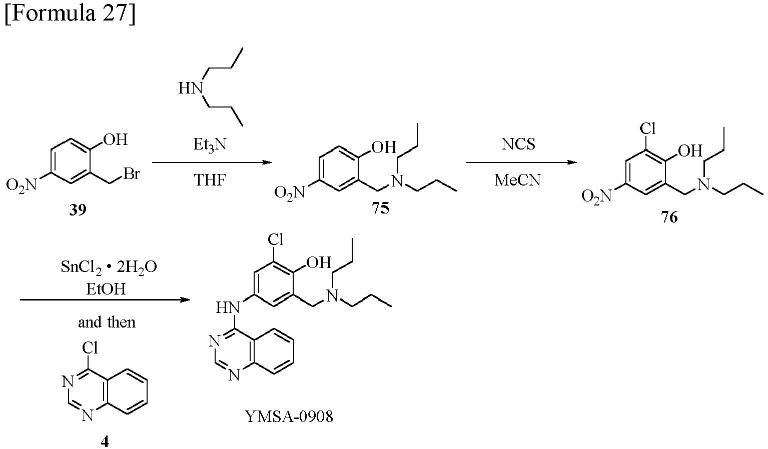

### 2-((Dipropylamino)methyl)-4-nitrophenol (75)

Dipropylamine (168 µL, 1.2 mmol) and Et₃N (167 µL, 1.2 mmol) were added to THF (4 mL) solution of 2-hydroxy-5-nitrobenzyl bromide (39) (232 mg, 1.0 mmol), and the reaction solution was heated under reflux for 1 hour. The reaction solution was concentrated under reduced pressure, and saturated NaHCO₃ aqueous solution was added, followed by extraction with AcOEt. The combined organic phases were washed with saturated saline water, dried over Na₂SO₄, filtered, and concentrated under reduced pressure. The resulting residue was purified by silica gel column chromatography (hexane/AcOEt = 1/1), thus giving Compound 75 as a yellow oily substance (249.9 mg, 99 %).

### 2-Chloro-6-((dipropylamino)methyl)-4-nitrophenol (76)

N-chlorosuccinimide (152.2 mg, 1.14 mmol) was added to MeCN (5 mL) solution of Compound 75 (238.5 mg, 0.95 mmol), and the reaction solution was stirred at room temperature for 27.5 hours. The reaction solution was concentrated under reduced pressure, and 10% Na₂S₂O₃ aqueous solution was added, followed by extraction with AcOEt (30 mL × 4). The combined organic phases were washed with saturated saline water, dried over Na₂SO₄, filtered, and concentrated under reduced pressure. The resulting residue was purified by silica gel column chromatography (hexane/AcOEt/ = 1/3), giving Compound 76 as a yellow solid (101.1 mg, 37 %). mp 124°C.

### YMSA-0908

YMSA-0908 was obtained as a yellow solid by the same method as that in the synthesis of YMSA-0464 (120.4 mg, 89 %). mp 144-145°C. IR (ATR): v= 2963, 1706, 1667, 1618, 1574, 1536, 1499, 1468, 1406, 1357, 1321, 1229, 1126, 1075, 1026, 968, 922, 868, 842, 820, 796, 764, 684, 592, 572, 547, 468, 423, 411 cm⁻¹. ¹H NMR (300MHz, CDCl₃/TMS): δ= 8.73 (s, 1H), 7.90 (d, J = 8.1 Hz, 2H), 7.79 (t, J =7.5 Hz, 1H), 7.58-7.51 (m, 3H), 3.77 (s, 2H), 2.54-2.48 (m, 4H), 1.64-1.52 (m, 4H), 0.90 (t, J = 7.4 Hz, 6H) ppm. ¹³C NMR (100 MHz, CDCl₃/TMS): δ = 162.3, 158.0, 154.7, 151.5, 149.4, 134.4, 132.8, 129.2, 128.0, 127.3, 127.0, 126.3, 123.6, 123.0, 122.6, 121.7, 120.3, 115.0, 57.8, 55.0, 19.0 ppm. HRMS (ESI⁺): calcd for [M+H]⁺, C₂₁H₂₆ClN₄O: 385.1790; found 385.1780. Anal. Calcd (%) for C₂₁H₂₆ClN₄O: C 65.53, H 6.55, N 14.56; found C 65.49, H 6.14, N 14.63.

### [Example 28] Synthesis of YMSA-0868

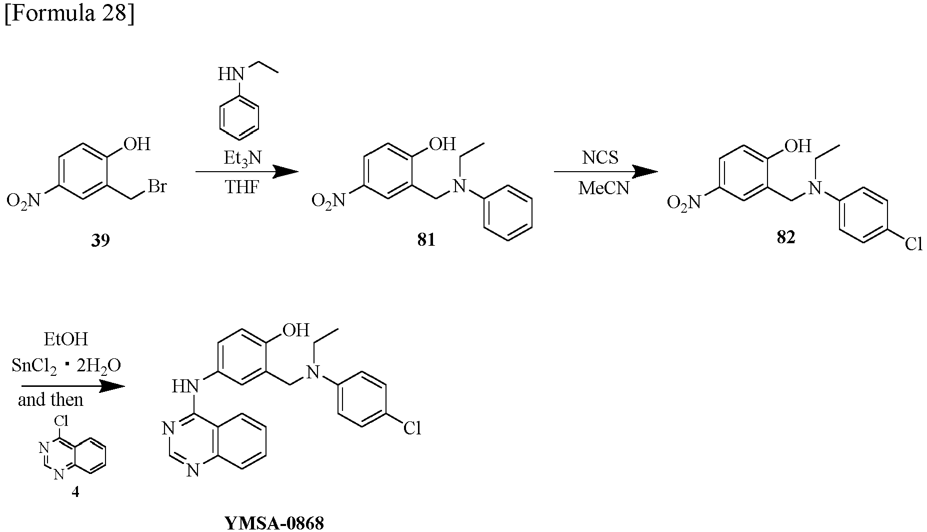

### 2-((Ethyl(phenyl)amino)methyl)-4-nitrophenol (81)

N-ethylaniline (151.5 µL, 1.2 mmol) and triethylamine (306.1 µL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 20.5 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane: ethyl acetate = 4:1), thus isolating Compound 81 (257 mg, 97%) as a yellow solid. mp 113-117°C.

### 2-(((4-Chlorophenyl)(ethyl)amino)methyl)-4-nitrophenol (82)

N-chlorosuccinimide (155.9 mg, 1.17 mmol) was added to a solution of Compound 81 (265 mg, 0.97 mmol) dissolved in 5 mL of acetonitrile, followed by stirring at room temperature for 72 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 82 (127.7 mg, 43%) as a yellow solid. mp 105-114°C.

### YMSA-0868

Lead chloride dihydrate (179.9 mg, 0.8 mmol) was added to a solution of Compound 82 (61.1 mg, 0.2 mmol) dissolved in 2 mL of ethanol, followed by stirring under reflux conditions for 2.5 hours. After the reaction solution was cooled to room temperature, 4-chloroquinazoline 4 (65.7 mg, 0.4 mmol) was added to the reaction solution, which was again stirred under reflux conditions for 2 hours. After confirming the completion of the reaction, the solvent was distilled off under reduced pressure, 2N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0868 (27.7 mg, 34%) as a white solid. mp 215°C (dec.). IR (ATR): v = 2967, 1616, 1596, 1578, 1499, 1443, 1402, 1384 1351, 1304, 1290, 1272, 1242, 1208, 1187, 1147, 1131, 1099,1071, 985, 959, 911, 863, 817, 786, 761, 745, 709, 683, 652, 592, 562, 545, 511, 462, 423, 416 cm⁻¹. ¹HNMR (300 MHz, CDCl₃/TMS): δ= 8.72 (s, 1H), 7.91 (d, J= 8.1 Hz, 1H), 7.85-7.78 (m, 2H), 7.55 (t, J = 7.65 Hz, 1H), 7.47 (s, 1H), 7.40 (d, J = 8.4 Hz, 1H), 7.27-7.24 (m, 2H), 7.03 (d, J = 9.0 Hz, 2H), 6.89 (d, J =8.4 Hz, 1H), 4.38 (s, 2H), 3.34-3.27 (m, 2H), 1.07 (t, J = 6.9 Hz, 3H) ppm. HRMS (FAB⁺): calcd for [M+H]⁺, C₂₃H₂₁ClN₄O: 404.1405; found 404.1404.

### [Example 29] Synthesis of YMSA-0884

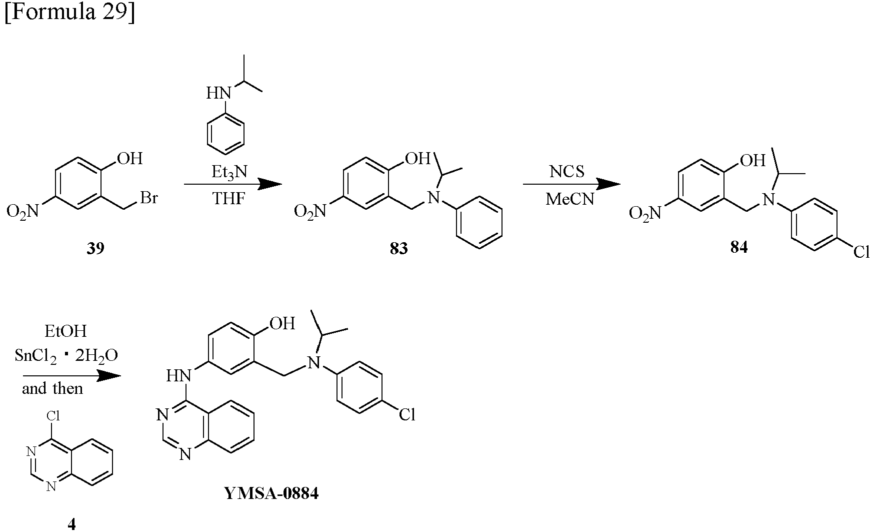

### 2-((Isopropyl(phenyl)amino)methyl)-4-nitrophenol (83)

N-isopropylaniline (172.6 µL, 1.2 mmol) and triethylamine (306.1 µL, 2.2 mmol) were added to a solution of 2-hydroxy-5-nitrobenzyl bromide 39 (0.23 g, 1.0 mmol) dissolved in 4 mL of tetrahydrofuran, followed by stirring under reflux conditions for 20.5 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 4:1), thus isolating Compound 83 (233.2 mg, 81%) as a yellow solid. mp 129-133°C.

### 2-(((4-Chlorophenyl)(isopropyl)amino)methyl)-4-nitrophenol (84)

N-chlorosuccinimide (130.4 mg, 0.98 mmol) was added to a solution of Compound 83 (233.2 mg, 0.81 mmol) dissolved in 5 mL of acetonitrile, followed by stirring at room temperature for 72 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 84 (224.3 mg, 85%) as a yellow solid. mp 83-85°C.

### YMSA-0884

Lead chloride dihydrate (287.5 mg, 1.27 mmol) was added to a solution of Compound 84 (102 mg, 0.32 mmol) dissolved in 2 mL of ethanol, followed by stirring under reflux conditions for 2.5 hours. After the reaction solution was cooled to room temperature, 4-chloroquinazoline 4 (104.9 mg, 0.64 mmol) was added to the reaction solution, which was again stirred under reflux conditions for 2 hours. After confirming the completion of the reaction, the solvent was distilled off under reduced pressure. 2N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating YMSA-0884 (72.6 mg, 55%) as a yellow solid. mp 94-105°C. IR(ATR): v = 2970, 1619, 1576, 1532, 1493, 1443, 1391, 1358, 1319, 1295, 1252, 1187, 1126, 1071, 1040, 987, 959, 919, 808, 746, 695, 681, 666, 595, 546, 513, 466 cm⁻¹. ¹HNMR (300 MHz, CDCl₃/TMS): δ = 8.72 (s, 1H), 7.90 (d, J = 7.8 Hz, 1H), 7.83-7.77 (m, 2H), 7.54 (t, J = 7.5 Hz, 1H), 7.47 (d, J = 3.0 Hz, 1H), 7.31-7.25 (m, 2H), 7.15 (d, J = 7.8 Hz, 2H), 7.09-7.05 (m,1H), 6.80 (d, J =9.0 Hz, 1H), 4.42 (s, 2H), 3.70 (hep, J =6.6, 1H), 1.19 (d, J= 6.6 Hz, 6H) ppm. ¹³CNMR (100MHz, CDCl₃/TMS): δ= 158.0, 155.1, 149.7, 147.1, 133.0, 129.5, 129.0, 128.8, 126.6, 124.1, 123.7, 123.5, 123.4, 123.0, 120.5, 116.7, 115.0, 54.4, 51.0, 19.1 ppm. HRMS (FAB⁺): calcd for [M+H]⁺, C₂₄H₂₃ClN₄O: 419.1636; found 419.1639. Anal. Calcd (%) for C₂₄H₂₃ClN₄O: C, 68.81; H, 5.53; N, 13.37. found: C, 67.07; H, 5.66; N, 13.14.

### [Example 30] Synthesis of YMSA-0909

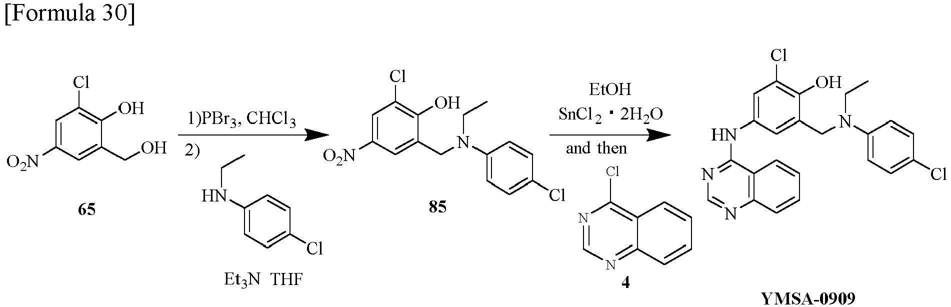

### 2-Chloro-6-(((4-chlorophenyl)(ethyl)amino)methyl)-4-nitrophenol (85)

Phosphorus tribromide (100 µL, 1.05 mmol) was added to a solution of Compound 65 (205.0 mg, 1.00 mmol) dissolved in 4 mL of chloroform, followed by stirring under room temperature for 23 hours. Water was added to the reaction solution to quench the reaction, followed by extraction with chloroform three times. The organic phase was washed with saturated saline water, and dried over sodium sulfate. Solids were removed by filtration, and the solvent was distilled off under reduced pressure. The resulting mixture (214.9 mg) was dissolved in tetrahydrofuran (3.2 mL), N-ethyl-4-chloroaniline (136 µL, 0.978 mmol) and triethylamine (248 µL, 1.78 mmol) were added, and the mixture was stirred under reflux conditions for 2.5 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane: ethyl acetate = 10:1 to 1:1), thus isolating Compound 85 (153.8 mg, 45%) as a yellow solid. mp 118-119°C.

### YMSA-0909

Lead chloride dihydrate (0.18 g, 0.80 mmol) was added to a solution of Compound 85 (68 mg, 0.20 mmol) dissolved in 2 mL of ethanol, followed by stirring under reflux conditions for 2.5 hours. After the reaction solution was cooled to room temperature, 4-chloroquinazoline 4 (72.8 mg, 0.4 mmol) was added to the reaction solution, which was again stirred under reflux conditions for 2.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate =5:1), thus isolating YMSA-0909 (12 mg, 14%) as a light brown solid. mp 185-192°C. IR (ATR): v= 2969, 1741, 1621, 1595, 1574, 1534, 1500, 1474, 1428, 1406, 1364, 1307, 1289, 1266, 1221, 1168, 1129, 1074, 982, 922, 865, 823, 812, 786, 761, 718, 701, 679, 647, 623, 599, 577, 557, 531, 506, 467, 438, 427, 414 cm⁻¹. ¹H NMR (300 MHz, CDCl₃/TMS): δ = 8.72 (s, 1H), 7.93-7.90 (m, 1H), 7.84-7.78 (m, 3H), 7.61-7.53 (m, 1H), 7.24-7.20 (m, 3H), 6.83 (d, J = 8.7 Hz, 2H), 4.45 (s, 2H), 3.34 (q, J = 6.9 Hz, 2H), 1.14 (t, J = 6.9 Hz, 3H) ppm. HRMS (FAB): Calcd for [M+H]⁺, C₂₃H₂₁Cl₂N₄O: 439.1092; found 439.1091.

### [Example 31] Synthesis of YMSA-0651

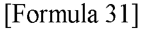

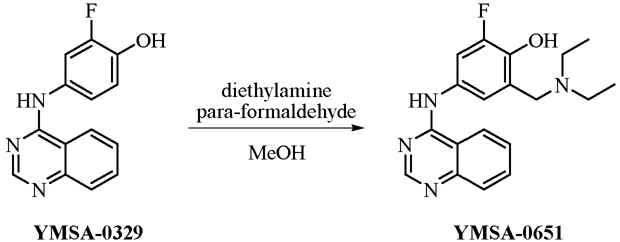

Diethylamine (82.7 µL, 0.80 mmol) and para-formaldehyde (30 mg, 5.2 mmol) were added to a solution of YMSA-0329 (51 mg, 0.20 mmol) dissolved in 2 mL of methanol, followed by stirring under reflux conditions for 17 hours. After confirming the completion of the reaction, the solvent was distilled off under reduced pressure. Then, the resulting mixture was purified by silica gel column chromatography (ethyl acetate:methanol = 1:0 to 9:1), thus isolating YMSA-0651 (18.5 mg, 27%) as a yellow solid. mp 163-167°C. IR (ATR): v = 3264, 3058, 2974, 2829, 1619, 1568, 1538, 1492, 1475, 1446, 1406, 1394, 1354, 1331, 1318, 1288, 1271, 1242, 1212, 1194, 1167, 1150, 1120, 1088, 1055, 1028, 1003, 965, 916, 867, 819, 798, 766, 725, 678, 614, 591, 557, 530, 506, 466, 434, 424, 410 cm⁻¹. ¹H NMR (300 MHz, CDCl₃/TMS): δ = 8.74 (s, 1H), 7.93 (d, J = 8.1 Hz, 1H), 7.84-7.78 (m, 2H), 7.56 (t, J = 7.2 Hz, 1H), 7.46-7.41 (m, 1H), 7.11 (s, 1H), 3.86 (s, 2H), 2.67 (q, J = 7.2 Hz, 2H), 1.15 (t, J = 7.2 Hz, 9H) ppm. ¹³C NMR (75 MHz, CDCl₃/TMS): δ = 157.8, 154.9, 152.3, 150.0, 149.1, 144.0, 143.8, 132.9, 128.8, 128.6, 126.5, 124.1, 124.0, 120.6, 117.8, 117.7, 115.0, 110.7, 110.5, 56.8, 46.4, 11.1 ppm. HRMS (ESI⁺): calcd for [M+H]⁺, C₁₉H₂₂FN₄O: 341.1772; found 341.1771. Anal. Calcd (%) for C₁₉H₂₁FN₄O + 0.5MeOH: C 65.71, H 6.50, N 15.72; found: C65.69, H 6.12, N 15.79.

### [Example 32] Synthesis of YMSA-0833

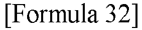

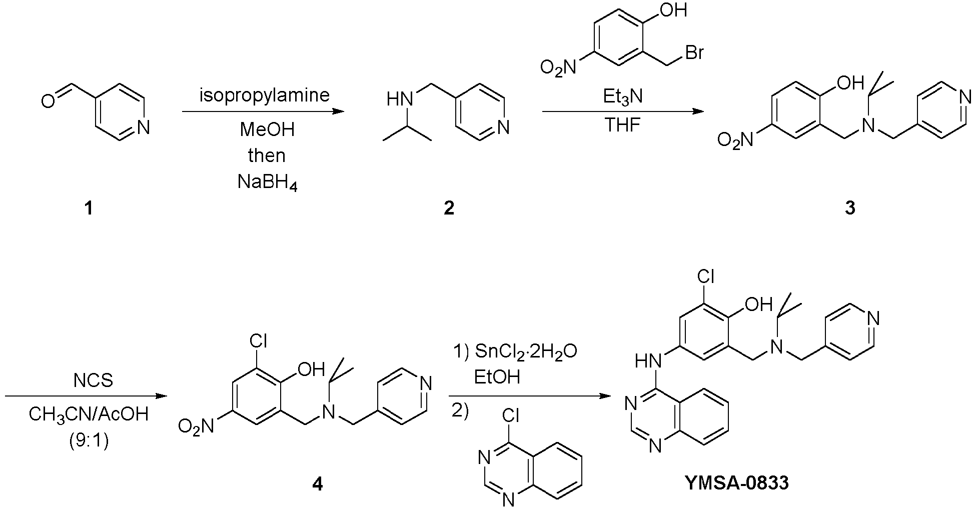

### N-(pyridin-4-ylmethyl)propan-2-amine (2)

Compound 1 (469 µL, 5.0 mmol) was dissolved in 5 mL of methanol, isopropylamine (428 µL, 5.0 mmol) was added thereto, and the mixture was stirred at room temperature for 2.5 hours. Sodium borohydride (208 mg, 5.5 mmol) was added to the reaction solution, which was further stirred for 13 hours. After confirming the completion of the reaction, H₂O and 3N sodium hydroxide aqueous solution were added, followed by extraction with chloroform. The organic phase was washed with saturated saline water, and dried over sodium sulfate. Solids were removed by filtration, and the solvent was distilled off under reduced pressure. The resulting mixture was purified by silica gel column chromatography (chloroform:methanol:triethylamine = 10:1:0.1), thus isolating Compound 2 (329 mg, 44%) as a colorless liquid. IR (ATR): v = 3284, 2965, 2115, 1603, 1561, 1468, 1415, 1381, 1337, 1309, 1220, 1174, 1127, 1092, 1065, 994, 800, 748, 663, 594, 528, 477, 443, 415, 407 cm⁻¹. ¹H NMR (300 MHz, CDCl₃/TMS): δ= 8.54-8.52 (m, 2H), 7.27-7.26 (m, 2H), 3.81 (s, 2H), 2.84 (sept, J = 6.3 Hz, 1H), 1.10 (d, J = 6.3 Hz, 9H) ppm. ¹³CNMR (100MHz, CDCl₃/TMS): δ= 150.0, 149.7, 149.6, 123.1, 121.2, 50.2, 48.4, 22.9(2C) ppm. HRMS (ESI⁺): calcd for [M+H]⁺, C₉H₁₅N₂: 151.1230; found 151.1225.

### 2-((Isopropyl(pyridin-4-ylmethyl)amino)methyl)-4-nitrophenol (3)

Compound 2 (329 mg, 2.2 mmol) was dissolved in 10 mL of tetrahydrofuran, triethylamine (306 µL, 2.2 mmol) and 2-hydroxy-5-nitrobenzyl bromide (425 g, 1.83 mmol) were added thereto, and the mixture was stirred under reflux conditions for 1.5 hours. After confirming the completion of the reaction, the solvent was distilled off, and the residue was diluted with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1→0:1), thus isolating Compound 3 (227 mg, 43%) as a yellow solid. IR (ATR): v = 1621, 1589, 1524, 1477, 1416, 1397, 1374, 1343, 1287, 1273, 1224, 1180, 1158, 1132, 1090, 1073, 1045, 977, 923, 911, 883, 856, 835, 813, 776, 762, 752, 736, 713, 670, 635, 614, 547, 494, 447, 422 cm⁻¹. ¹H NMR (300 MHz, CDCl₃/TMS): δ= 8.52-8.50 (m, 2H), 8.23 (brs,1H), 8.09-8.06 (m, 1H), 7.69 (d, J = 8.1 Hz, 1H), 7.46 (t, J = 8.1 Hz, 1H), 7.29 (d, J = 6.0 Hz, 1H), 3.66 (s, 2H), 3.59 (s, 2H), 2.90 (sept, J = 6.6 Hz, 1H), 1.11 (d, J = 6.6 Hz, 9H) ppm. ¹³CNMR (100MHz, CDCl₃/TMS): δ= 162.9, 149.5, 148.7, 139.4, 126.0, 124.8, 124.2, 123.5, 115.3, 52.2, 49.8, 48.3, 17.4 ppm. HRMS (ESI⁺): calcd for [M+H]⁺, C₁₆H₂₀N₃O₃: 302.1505; found 302.1495.

### 2-Chloro-6-((isopropyl(pyridin-4-ylmethyl)amino)methyl)-4-nitrophenol (4)

N-chlorosuccinimide (80 mg, 0.6 mmol) was added to a solution of Compound 3 (143 mg, 0.5 mmol) dissolved in 4.5 mL of acetonitrile and 0.2 mL of acetic acid, followed by stirring at room temperature for 44 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate), thus isolating Compound 4 (63.1 mg, 38%) as a yellow solid. IR (ATR): v = 3089, 2980, 1599, 1563, 1468, 1420, 1374, 1329, 1230, 1215, 1173, 1091, 1022, 991, 910, 894, 876, 852, 822, 811, 747, 715, 666, 655, 594, 546, 529, 480, 412 cm⁻¹. ¹H NMR (300 MHz, CDCl₃/TMS): δ= 8.62 (d, J = 5.7 Hz, 2H), 8.20 (d, J = 3.0 Hz, 1H), 7.87 (d, J = 2.7 Hz, 1H), 7.26-7.25 (m, 1H), 3.91 (s, 2H), 3.67 (s, 2H), 3.08 (sept, J = 6.6 Hz, 1H), 1.21 (d, J = 6.6 Hz, 9H) ppm. ¹³CNMR (100MHz, CDCl₃/TMS): δ= 171.8, 166.5, 149.9, 143.0, 124.8, 124.5, 120.9, 120.7, 52.1, 51.9, 51.8, 16.4 ppm. HRMS (ESI⁺): calcd for [M+H]⁺, C₁₆H₁₉ClN₃O₃: 336.1115; found 336.1109.

### YMSA-0833

Tin chloride dihydrate (133 mg, 0.59 mmol) was added to a solution of Compound 4 (49.5 mg, 0.15 mmol) dissolved in 4 mL of ethanol, followed by stirring under reflux conditions for 1 hour. After the reaction solution was cooled to room temperature, 4-chloroquinazoline (48.5 mg, 0.29 mmol) was added to the reaction solution, which was again stirred under reflux conditions for 1.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (ethyl acetate), thus isolating the compound YMSA-0833 (30 mg, 47%) as a light yellow solid. M.p. 98-101°C.IR(ATR): v = 2967, 1620, 1601, 1574, 1533, 1477, 1393, 1358, 1318, 1236, 1157, 1127, 1073, 987, 919, 869, 765, 721, 680, 623, 580, 556, 534, 506, 467, 421, 413 ppm. ¹H NMR (300 MHz, CDCl₃/TMS): δ= 8.73 (s, 1H), 8.59 (d, J = 6.0 Hz, 2H), 7.93 (d, J = 8.4 Hz, 1H), 7.85-7.82 (m, 2H), 7.61-7.53 (m, 2H), 7.53 (d, J = 2.7 Hz, 1H), 7.41 (d, J = 2.4 Hz, 1H), 3.86 (s, 2H), 3.66(s, 2H), 3.09 (sept, J = 6.6 Hz, 1H), 1.18 (d, J = 6.6 Hz, 9H) ppm. ¹³CNMR (100MHz, CDCl₃/TMS): δ= 157.7, 154.5, 149.8, 149.5, 149.2, 147.4, 132.9, 131.0, 127.8, 126.2, 124.3, 124.0, 122.8, 122.7, 122.3, 118.8, 115.0, 51.8, 51.4, 49.3, 17.0 ppm. HRMS (ESI⁺): calcd for [M+H]⁺, C₂₄H₂₅ClN₅O: 434.1748; found 434.1742.

### [Example 33] Synthesis of YMSA-0866

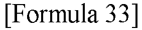

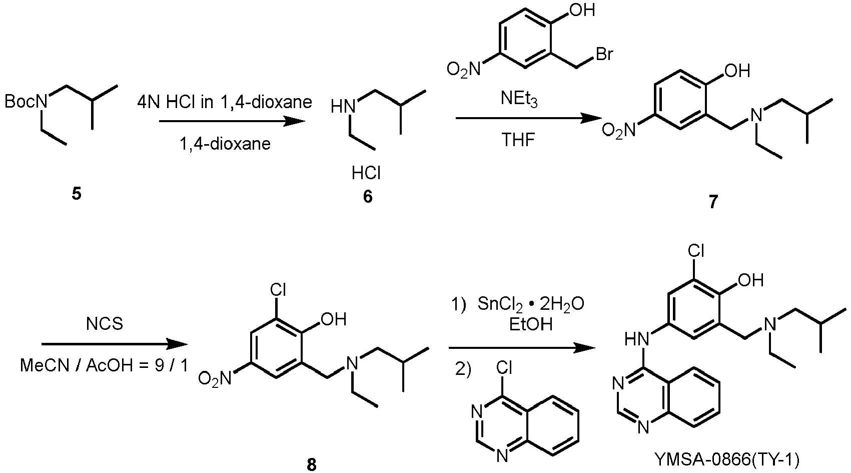

### N-ethyl-2-methylpropan-1-amine (6)

Compound 5 (0.20 g, 0.99 mmol) was dissolved in 1 mL of dioxane, 4N dioxane hydrochloride solution (3.0 mL, 12 mmol) was added, and the mixture was stirred at room temperature for 1 hour. After confirming the completion of the reaction, the solvent was distilled off under reduced pressure. The resulting solid was washed with diethyl ether, thus isolating Compound 6 (84 mg, 62%) as a colorless solid.

M.p. 192-194°C. IR (ATR): v = 2963, 2808, 2748, 2690, 2528, 2446, 2393, 2323, 1601, 1512, 1480, 1468, 1446, 1348, 1327, 1251, 1181, 1157, 1100, 1039, 1019, 973, 960, 943, 923, 865, 825, 802, 498, 442, 419cm⁻¹. ¹H NMR (300 MHz, CDCl₃/TMS): δ = 9.40 (s, 1H), 3.13-3.02 (m, 2H), 2.79-2.73 (m, 2H), 2.33-2.19 (m, 1H), 1.50 (t, J = 7.2 Hz, 3H), 1.12 (d, J = 6.6, 6H) ppm. ¹³C NMR (100 MHz, CDCl₃/TMS): δ = 54.4, 43.4, 25.9, 20.8, 11.0 ppm. HRMS (ESI (Positive mode)): calcd for [M+H]⁺, C₆H₁₆N: 102.1277; found 102.1278.

### 2-((Ethyl(isobutyl)amino)methyl)-4-nitrophenol (7)

Compound 6 (41 mg, 0.30 mmol) and triethylamine (80 µL, 0.60 mmol) were added to a solution of 2-hydroxy-5-nitrobenzyl bromide (71 mg, 0.31 mmol) dissolved in 1 mL of tetrahydrofuran, followed by stirring under reflux conditions for 2 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 4:1), thus isolating Compound 7 (68.6 mg, 91%) as a yellow solid. M.p. 78.5-79.8°C. IR (ATR): v = 2956, 2873, 1591, 1553, 1509, 1478, 1435, 1402, 1380, 1340, 1305, 1264, 1204, 1142, 1114, 1088, 1053, 982, 966, 897, 881, 870, 822, 795, 761, 746, 714, 535, 523, 481, 465 cm⁻¹. ¹H NMR (300 MHz, CDCl₃/TMS): δ = 8.09 (dd, J = 3.0, 2.4 Hz, 1H), 7.93 (d, J = 3.0, 1H), 6.82 (d, J = 9.0, 1H), 3.82 (s, 2H), 2.62 (q, J = 7.2, 2H), 2.34 (d, J = 7.5, 2H), 1.99-1.86 (m, J = 6.6, 1H), 1.11 (t, J = 6.9, 3H), 0.96 (d, J = 6.6, 6H) ppm. ¹³C NMR (100 MHz, CDCl₃/TMS): δ = 165.2, 139.7, 125.3, 124.5, 121.9, 116.3, 61.7, 57.6, 46.7, 25.7, 20.8, 10.0 ppm. HRMS (ESI (Positive mode)): calcd for [M+H]⁺, C₁₃H₂₁N₂O₃: 253.1547; found 253.1547.

### 2-Chloro-6-((ethyl(isobutyl)amino)methyl)-4-nitrophenol (8)

N-chlorosuccinimide (25 mg, 0.19 mmol) was added to a solution of Compound 7 (40 mg, 0.16 mmol) dissolved in 0.9 mL of acetonitrile and 0.1 mL of acetic acid, followed by stirring at room temperature for 21.5 hours. The solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 2:1 to 1:5), thus isolating Compound 8 (32.8 mg, 71%) as a yellow solid. M.p. 99.8-102.1°C. IR (ATR): v = 3508, 3395, 3093, 2924, 1603, 1583, 1519, 1462, 1422, 1338, 1255, 1208, 1169, 1085, 1051, 979, 940, 899, 865, 799, 752, 742, 710, 639, 557, 542, 521, 472, 417 cm⁻¹. ¹H NMR (300 MHz, CDCl₃/TMS): δ = 8.23 (d, J = 2.4 Hz 1H), 7.84 (d, J = 2.4, 1H), 3.92 (s, 2H), 2.73 (q, J = 7.2, 2H), 2.47 (d, J = 7.5, 2H), 2.04-1.94 (m, 1H), 1.18 (t, J = 7.2, 3H), 1.02 (d, J = 6.6, 6H) ppm. ¹³C NMR (100 MHz, CDCl₃/TMS): δ = 163.6, 137.7, 125.7, 122.9, 121.8, 120.5, 61.4, 57.7, 46.7, 25.5, 20.8, 9.5 ppm. HRMS (ESI (Positive mode)): calcd for [M+H]⁺, C₁₃H₂₀³⁵ClN₂O₃: 287.1157; found 287.1157.

### YMSA-0866 (TY-1)

Tin chloride dihydrate (45 mg, 0.21 mmol) was added to a solution of Compound 8 (19 mg, 0.06 mmol) dissolved in 1 mL of ethanol, followed by stirring under reflux conditions for 1.5 hours. After the reaction solution was cooled to room temperature, 4-chloroquinazoline (12 mg, 0.07 mmol) was added to the reaction solution, which was again stirred under reflux conditions for 2 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating the compound YMSA-0866 (21 mg, 84%) as a yellow solid. M.p. 137.3-140.2°C. IR (ATR): v = 2959, 1620, 1572, 1532, 1499, 1467, 1389, 1356, 1317, 1287, 1237, 1191, 1163, 1125, 1074, 1043, 990, 958, 917, 868, 820, 763, 726, 679, 581, 544, 466, 433, 408 cm⁻¹. ¹H NMR (300 MHz, CDCl₃/TMS): δ = 8.74 (s, 1H), 7.93-7.90 (m, 1H), 7.83-7.78 (m, 2H), 7.60-7.53 (m, 2H), 7.30 (d, J = 2.7 Hz, 1H), 7.22 (s, 1H), 3.81 (s, 2H), 2.62 (q, J = 7.2, 2H), 2.34 (d, J = 7.5, 2H), 1.93 (m, 1H), 1.11 (t, J = 7.2, 3H), 0.98 (d, J = 6.6, 6H) ppm. ¹³C NMR (100 MHz, CDCl₃/TMS): δ = 157.7, 155.0, 151.6, 150.0, 132.9, 129.3, 129.0, 126.6, 123.4, 121.6, 120.7, 120.1, 114.9, 62.0, 58.1, 46.8, 25.8, 21.0, 10.2 ppm. HRMS (ESI (Positive mode)): calcd for [M+H]⁺, C₂₁H₂₆³⁵ClN₄O: 385.1790; found 385.1788.

### [Example 34] Synthesis of YMSA-0911

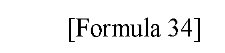

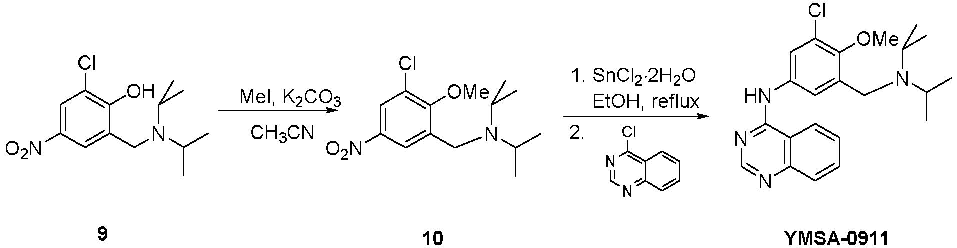

### N-(3-chloro-2-methoxy-5-nitrobenzyl)-N-isopropylpropan-2-amine (10)

Potassium carbonate (138 mg, 1 mmol) and methyl iodide (62 µL, 1 mmol) were added to a solution of Compound 9 (57.3 mg, 0.2 mmol) dissolved in 4 mL of acetonitrile, followed by stirring under reflux conditions for 3 hours. After the solvent was distilled off under reduced pressure, H₂O was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 9:1), thus isolating Compound 10 (49.2 mg, 82%) as a colorless oily substance. ¹H NMR (300 MHz, CDCl₃/TMS): δ = 8.47 (d, J = 2.4 Hz, 1H), 8.12 (d, J = 2.1 Hz, 1H), 3.93 (s, 3H), 3.72 (s, 2H), 3.05 (sept, J = 6.6 Hz, 1H), 1.04 (d, J = 6.6 Hz, 12H) ppm. HRMS (ESI⁺): calcd for [M+H]⁺, C₁₄H₂₂ClN₂O₃: 301.1319; found:301.1313.

### YMSA-0911 (TTn-146)

Tin chloride dihydrate (81 mg, 0.36 mmol) was added to a solution of Compound 10 (28 mg, 0.09 mmol) dissolved in 1.5 mL of ethanol, followed by stirring under reflux conditions for 1 hour. After the reaction solution was cooled to room temperature, 4-chloroquinazoline (30 mg, 0.18 mmol) was added to the reaction solution, which was again stirred under reflux conditions for 3 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating the compound YMSA-0911 (95 mg, quant.) as a yellow solid. ¹H NMR (300 MHz, CDCl₃/TMS): δ = 8.80 (s, 1H), 7.95-7.89 (m, 3H), 7.85-7.79 (m, 1H), 7.67 (d, J = 3.0 Hz, 1H), 7.61-7.56 (m, 1H), 7.41 (s, 1H), 3.86 (s, 3H), 3.76-3.73 (m, 2H), 3.12-3.01 (m, 1H), 1.06 (d, J = 6.6 Hz, 12H) ppm. HRMS (ESI⁺): calcd for [M+H]⁺, C₂₂H₂₈ClN₄O: 399.1952; found 399.1947.

### [Example 35] Synthesis of YMSA-0944

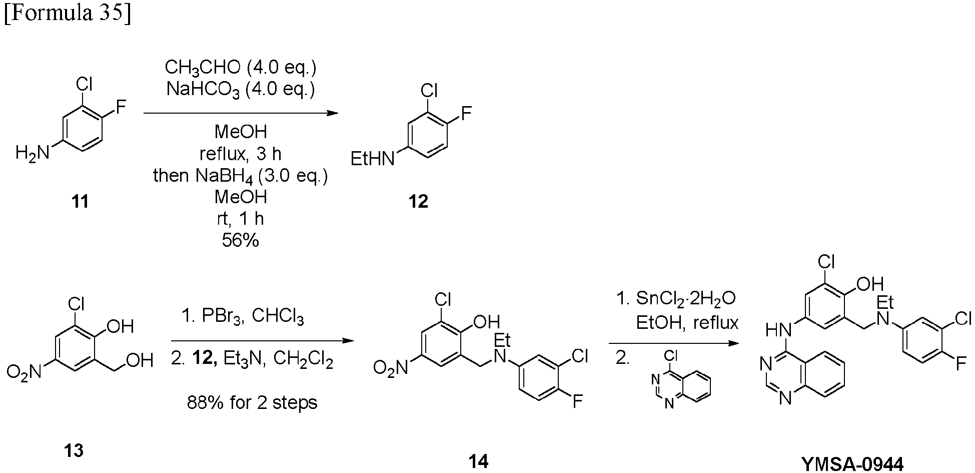

### 3-Chloro-N-ethyl-4-fluoroaniline (12)

Sodium hydrogen carbonate (1154.1 mg, 13.7 mmol) and acetaldehyde (776 uL, 13.7 mmol) were added to a solution of Compound 11 (500.0 mg, 3.4 mmol) dissolved in 6 mL of methanol, followed by stirring under reflux conditions for 3 hours. After the reaction solution was cooled to room temperature, the solvent and acetaldehyde were distilled off under reduced pressure. 15 mL of methanol and sodium borohydride (390.4 mg, 10.3 mmol) were added to the resulting residue, and the mixture was stirred at room temperature for 1 hour. After confirming the completion of the reaction, the reaction solution was slowly added to a mixture of ethyl acetate and 2M hydrochloric acid, and the organic phase was extracted with 2N hydrochloric acid. Thereafter, the resulting aqueous phase was neutralized with 5N sodium hydroxide aqueous solution until the pH reached 11. The neutralized aqueous phase was extracted with chloroform three times, and then dried over sodium sulfate. Solids were removed by filtration, and the solvent was distilled off under reduced pressure. The resulting mixture was purified by silica gel column chromatography (hexane), thus isolating Compound 12 (336.7 mg, 56%) as a colorless oil. IR (ATR): v = 3420, 2972, 2876, 1612, 1503, 1452, 1396, 1323, 1260, 1219, 1148, 1099, 1063, 1046, 946, 923, 840, 799, 781, 742, 702, 689, 584, 518, 443, 418 cm⁻¹. ¹H NMR (300 MHz, CDCl₃/TMS): δ = 6.93 (t, J = 9.0Hz, 1H), 6.58 (dd, J = 6.2, 2.9 Hz, 1H), 6.41 (td, J = 9.0, 3.3 Hz, 1H), 3.48 (brs, 1H), 3.09 (q, J = 6.9 Hz, 2H), 1.25 (t, J = 6.9 Hz, 3H) ppm; ¹³C NMR (100 MHz, CDCl₃/TMS): δ = 150.8 (d, J_{C,F} = 235.7 Hz), 145.5, 121.0 (d, J_{C,F} = 18.2 Hz), 116.8 (d, J_{C,F} = 22.0 Hz), 113.5, 112.1 (d, J_{C,F} = 5.8 Hz), 38.9, 14.7 ppm; HRMS (ESI positive): calcd for [M+H]⁺, C₈H₁₀³⁵ClFN: 174.0480; found 178.0480.

### 2-Chloro-6-(((3 -chloro-4-fluorophenyl)(ethyl)amino)methyl)-4-nitrophenol (14)

PBr₃ (85.5 µL, 0.90 mmol) was added to a solution of Compound 13 (61.1 mg, 0.3 mmol) dissolved in 1 mL of chloroform, followed by stirring at room temperature for 16 hours. After confirming the completion of the reaction, the reaction solution was diluted with chloroform. The organic phase was washed with water and saturated saline water, then dried over sodium sulfate. Solids were removed by filtration, and the solvent was distilled off under reduced pressure. The resulting mixture (264.6 mg) was dissolved in 1 mL of dichloroethane, and dichloromethane solution (2 mL) of triethylamine (125 µL, 0.90 mmol) and 12 (78.1 mg, 0.45 mmol) was added, followed by stirring at room temperature for 2 hours. After confirming the completion of the reaction, the reaction solution was diluted with chloroform. The organic phase was washed with 1N hydrochloric acid, saturated sodium hydrogen carbonate aqueous solution, and saturated saline water, and then dried over sodium sulfate. Solids were removed by filtration, and the solvent was distilled off under reduced pressure. The resulting mixture was purified by silica gel column chromatography (hexane:ethyl acetate =5:1), thus isolating Compound 14 (94.3 mg, 0.26 mmol, 88% for steps) as a yellow solid. M.p. 101-102°C. IR (ATR): v = 3403, 3102, 2979, 2936, 1610, 1584, 1504, 1466, 1440, 1398, 1376, 1325, 1284, 1271, 1223, 1193, 1178, 1130, 1068, 1049, 981, 941, 922, 896, 868, 842, 794, 742, 722, 681, 560, 524, 490, 443, 413 cm⁻¹. ¹H NMR (300 MHz, CDCl₃/TMS): δ = 8.23 (d, J = 3.0 Hz, 1H), 7.91(d, J= 2.7 Hz, 1H), 7.07-7.15 (m, 2H), 6.92-6.97 (m, 1H), 4.40 (s, 2H), 3.23 (q, J = 4.2 Hz, 2H), 1.11 (t, J = 4.2 Hz, 2H) ppm; ¹³C NMR (100 MHz, CDCl₃/TMS): δ = 158.6, 154.8 (d, J_{C,F} = 244.9 Hz), 153.6, 144.1 (d, J_{C,F} = 2.9 Hz), 140.4, 125.2, 123.3, 122.6 (d, J = 9.6 Hz), 122.0, 121.8, 121.5, 120.4 (d, J_{C,F} = 6.7 Hz), 117.3 (d, J_{C,F} = 21.1 Hz), 55.8, 49.1, 11.3 ppm; HRMS (ESI positive): calcd for [M+H]⁺, C₁₅H₁₄³⁵Cl₂FN₂O₃: 359.0360; found 359.0359.

### YMSA-0944

Tin chloride dihydrate (147.0 mg, 0.65 mmol) was added to a solution of Compound 14 (78.0 mg, 0.22 mmol) dissolved in 2 mL of ethanol, followed by stirring under reflux conditions for 30 minutes. After the reaction solution was cooled to room temperature, 4-chloroquinazoline (46.4 mg, 0.28 mmol) was added to the reaction solution, which was again stirred under reflux conditions for 4 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 10:1 to 2:1, 1% triethylamine) and preparative TLC (hexane:ethyl acetate = 3:1, 1% triethylamine), thus isolating the compound YMSA-0911 (7.1 mg, 7%) as a light yellow solid. ¹H NMR (300 MHz, CDCl₃/TMS): δ = 8.72 (s, 1H), 7.78-7.93 (m, 4H), 7.55 (t, J = 6.9 Hz, 1H), 7.23 (t, J = 2.4 Hz, 1H), 7.02 (t, J = 9.0 Hz, 1H), 6.95 (dd, J = 6.2, 2.9 Hz, 1H), 4.41 (s, 2H), 3.33 (q, J = 7.2 Hz, 2H), 1.15 (t, J = 7.2 Hz,) ppm; HRMS (ESI positive): calcd for [M+H]⁺, C₂₃H₂₀³⁵Cl₂FN₄O: 457.0993; found 457.0992.

### [Example 36] Synthesis of YMSA-0958

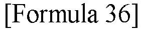

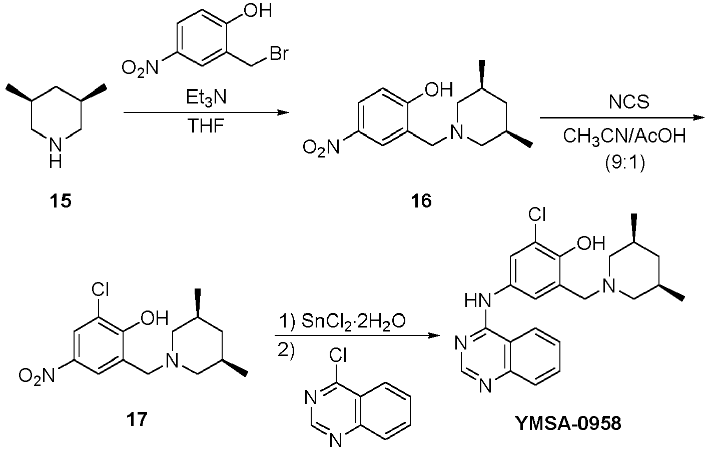

### 2-(((3R,S S)-3,5-dimethylpiperidin-1-yl)methyl)-4-nitrophenol (16)

Compound 15 (89.5 mg, 0.79 mmol) and triethylamine (202 µL, 1.5 mmol) were added to a solution of 2-hydroxy-5-nitrobenzyl bromide (153 mg, 0.66 mmol) dissolved in 2 mL of tetrahydrofuran, followed by stirring under reflux conditions for 2 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 16 (173 mg, 99%) as a yellow oily substance. ¹H NMR (300 MHz, CDCl₃/TMS): δ = 8.09 (dd, J = 2.7, 9.0 Hz, 1H), 7.92 (d, J = 2.7 Hz, 1H), 6,83 (d, J = 8.7 Hz, 1H), 3.77 (s, 2H), 2.90 (d, J = 9.0 Hz, 2H), 1.81-1.66 (m, 5H), 0.89 (d, J = 6.0 Hz, 6H), 0.69-0.58 (m, 1H) ppm. HRMS (ESI⁺): calcd for [M+H]⁺, C₁₄H₂₁N₂O₃: 265.1552; found 265.1544.

### 2-Chloro-6-(((3R,5S)-3,5-dimethylpiperidin-1-yl)methyl)-4-nitrophenol (17)

N-chlorosuccinimide (93 mg, 0.70 mmol) was added to a solution of Compound 16 (154 mg, 0.58 mmol) dissolved in 3 mL of acetonitrile, followed by stirring at room temperature for 13 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 17 (34 mg, 19%) as a yellow solid. ¹H NMR (300 MHz, CDCl₃/TMS): δ = 8.23 (d, J = 3.0 Hz, 1H), 7.83 (d, J = 3.0 Hz, 1H), 3.85 (s, 2H), 2.98 (d, J = 6.6 Hz, 2H), 1.85-1.82 (m, 5H), 0.91 (d, J = 5.7 Hz, 6H), 0.67-0.66 (m, 1H) ppm.

### YMSA-0958

Tin chloride dihydrate (90.4 mg, 0.40 mmol) was added to a solution of Compound 17 (29.8 mg, 0.10 mmol) dissolved in 2 mL of ethanol, followed by stirring under reflux conditions for 1.5 hours. After the reaction solution was cooled to room temperature, 4-chloroquinazoline (33 mg, 0.20 mmol) was added to the reaction solution, which was again stirred under reflux conditions for 2.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (hexane:ethyl acetate = 1:1), thus isolating the compound YMSA-0958 (34.6 mg, 87%) as a light yellow solid. M.p. 48-49°C. IR(ATR): v = 2955, 2911, 1619, 1574, 1537, 1498, 1466, 1405, 1357, 1319, 1228, 1125, 1967, 999, 962, 921, 899, 868, 819, 796, 777, 761, 732, 684, 654, 594, 547, 519, 503, 467, 446, 417 ppm. ¹H NMR (300 MHz, CDCl₃/TMS): δ = 8.74 (s, 1H), 7.92 (d, J= 8.1 Hz, 1H), 7.84-7.78 (m, 2H), 7.59-7.53 (m, 2H), 7.28-7.23 (m, 1H), 3.74 (s, 2H), 2.95 (d, J = 9.3 Hz, 2H), 1.79-1.62 (m, 5H), 0.88 (d, J= 6.3 Hz, 6H), 0.67-0.59 (m, 1H) ppm. ¹³CNMR (100MHz, CDCl₃/TMS): δ= 165.2, 125.2, 124.6, 121.3, 116.4, 61.0, 60.3, 41.4, 31.1, 19.2 ppm. HRMS (ESI⁺): calcd for [M+H]⁺, C₂₂H₂₆ClN₄O: 397.1795; found 397.1789.

### [Example 37] Synthesis of YMSA-0968

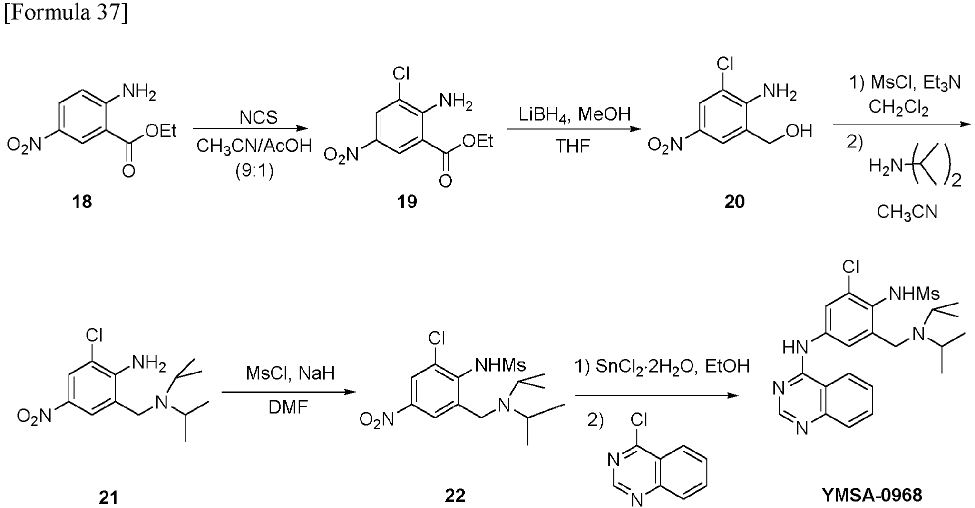

### Ethyl 2-amino-3-chloro-5-nitrobenzoate (19)

N-chlorosuccinimide (340 mg, 2.54 mmol) was added to a solution of Compound 18 (538 mg, 2.54 mmol) dissolved in 9 mL of acetonitrile and 1 mL of acetic acid, followed by stirring at room temperature for 8 hours. N-chlorosuccinimide (340 mg, 2.54 mmol) was further added to the reaction solution, followed by stirring at room temperature for 13 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 5:1), thus isolating Compound 19 (604 mg, 97%) as a yellow solid. M.p. 145-146°C. IR (ATR): v = 3470, 3339, 3103, 2988, 1851, 1804, 1695, 1603, 1585, 1557, 1505, 1467, 1429, 1374, 1314, 1251, 1220, 1141, 1115, 1081, 1025, 938, 929, 906, 874, 823, 793, 742, 710, 662, 600, 507, 461 cm⁻¹. ¹H NMR (300 MHz, CDCl₃/TMS): δ= 8.79 (d, J = 2.4 Hz, 1H), 8.33 (d, J = 2.7 Hz, 1H), 4.41 (q, J = 7.2 Hz, 2H), 1.44 (t, J = 7.2 Hz, 3H) ppm. ¹³C NMR (100MHz, CDCl₃/TMS): δ= 166.4, 151.0, 136.2, 128.5, 126.9, 120.0, 110.2, 61.8, 14.2 ppm. HRMS (ESI⁺): calcd for [M+H]⁺, C₉H₁₀ClN₂O₄: 245.0329; found 245.0328.

### 2-Amino-3 -chloro-5 -nitrophenyl)methanol (20)

LiBH₄ in THF (2 M solution, 1.5 mL, 3 mmol) and methanol (125 µL, 3 mmol) were added to a solution of Compound 19 (490 mg, 2 mmol) dissolved in 2 mL of tetrahydrofuran, followed by stirring at room temperature for 1 hour. After confirming the completion of the reaction, methanol was added until bubbling no longer occurred. After the solvent was distilled off, ethyl acetate was added to the resulting residue. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 20 (305 mg, 76%) as a colorless solid. M.p. 158-159°C. IR (ATR): v = 3521, 3417, 3336, 3233, 3099, 2867, 1651, 1597, 1577, 1484, 1457, 1439, 1395, 1297, 1220, 1188, 1105, 1054, 979, 947, 896, 855, 821, 743, 734, 628, 543, 480 cm⁻¹. ¹H NMR (300 MHz, CDCl₃/TMS): δ= 8.06 (s, 1H), 6.56 (s, 1H), 5.51 (s, 1H), 4.46 (d, J= 4.5 Hz, 2H) ppm. ¹³C NMR (100MHz, CDCl₃/TMS): δ= 147.9, 135.7, 126.3, 123.8, 121.5, 115.9, 59.7 ppm. HRMS (ESI⁺): calcd for [M+H]⁺, C₇H₈ClN₂O₃: 203.0223; found 203.0224.

### 2-Chloro-6-((ethyl(isopropyl)amino)methyl)-4-nitroaniline (21)

Triethylamine (416 µL, 3 mmol) and methane sulfonyl chloride (155 µL, 2 mmol) were added to a solution of Compound 20 (101 mg, 0.5 mmol) dissolved in 5 mL of dichloromethane, followed by stirring at room temperature for 1.5 hours. After confirming the completion of the reaction, 1N hydrochloric acid and dichloromethane were added. The organic phase was washed with saturated saline water, and then dried over sodium sulfate. Then, solids were removed by filtration. The solvent was distilled off under reduced pressure, giving a mesylated product (118 mg, 85%). The resulting mesylated product was dissolved in acetonitrile (4 mL), and diisopropylamine (295 µL, 2.1 mmol) was added, followed by stirring at room temperature for 1 hour. After the solvent was distilled off, purification was performed by silica gel column chromatography (hexane:ethyl acetate = 4:1), thus isolating Compound 21 (68 mg, 56%) as a colorless solid.

¹H NMR (300 MHz, CDCl₃/TMS): δ= 8.15 (d, J = 2.7 Hz, 1H), 7.90 (d, J = 2.1 Hz, 1H), 6.47 (br, 1H), 3.81 (s, 2H), 3.08-2.99 (m, 2H), 1.07 (m, 12H) ppm. ¹³C NMR (100MHz, CDCl₃/TMS): δ= 150.1, 137.3, 124.5, 123.0, 49.2, 47.3, 29.7, 20.0 ppm. HRMS (ESI⁺): calcd for [M+H]⁺, C₁₃H₂₁ClN₃O₂: 286.1322; found 286.1319.

### N-(2-Chloro-6-((ethyl(isopropyl)amino)methyl)-4-nitrophenyl)methanesulfonamide (22)

Sodium hydride (4.4 mg, 0.1 mmol) was added to a solution of Compound 21 (28.6 mg, 0.1 mmol) dissolved in 0.5 mL of dimethylformamide, followed by stirring for 30 minutes. Methanesulfonyl chloride (7.7 µL, 0.1 mmol) was added to the reaction solution, which was further stirred at room temperature for 34 hours. After confirming the completion of the reaction, saturated sodium hydrogen carbonate aqueous solution and ethyl acetate were added. The organic phase was washed with saturated saline water, and then dried over sodium sulfate. Then, solids were removed by filtration. After the solvent was distilled off, purification was performed by silica gel column chromatography (hexane:ethyl acetate = 10:1), thus isolating Compound 22 (14 mg, 39%) as a colorless solid. ¹H NMR (300 MHz, CDCl₃/TMS): δ = 8.27 (d, J = 2.4 Hz, 1H), 8.02 (d, J = 2.4 Hz, 1H), 4.06 (s, 2H), 3.36 (s, 3H), 3.27-3.14 (m, 3H), 1.15 (d, J = 6.6 Hz, 12H).

### YMSA-0968

Tin chloride dihydrate (33.6 mg, 0.15 mmol) was added to a solution of Compound 22 (13.5 mg, 0.037 mmol) dissolved in 1 mL of ethanol, followed by stirring under reflux conditions for 1 hour. After the reaction solution was cooled to room temperature, 4-chloroquinazoline (14.4 mg, 0.087 mmol) was added to the reaction solution, which was again stirred under reflux conditions for 1 hour. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (ethyl acetate), thus isolating the compound YMSA-0968 (6.1 mg, 36%) as a light yellow solid. ¹H NMR (300 MHz, CDCl₃/TMS): δ = 8.80 (s, 1H), 8.01-7.93 (m, 3H), 7.86-7.81 (m, 1H), 7.63-7.58 (m, 3H), 3.84 (s, 2H), 3.36 (m, 3H), 3.12-3.03 (m, 2H), 1.05 (d, J= 6.6 Hz, 12H) ppm. ¹³CNMR (100MHz, CDCl₃/TMS): δ= 157.8, 155.0, 151.7, 149.9, 132.9, 129.3, 128.9, 126.6, 123.6, 122.7, 121.6, 120.7, 120.3, 61.6, 60.5, 41.5, 31.1, 19.3 ppm.

### [Example 38] Synthesis of YMSA-0973

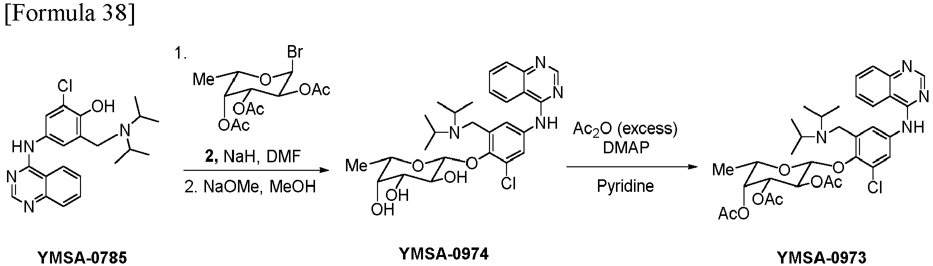

### YMSA-0974

Sodium hydride (55% in paraffin oil, 4.4 mg, 0.10 mmol) was added to a solution of the compound YMSA-0785 (35.0 mg, 0.091 mmol) dissolved in 0.5 mL of dimethylformamide, followed by stirring at room temperature for 15 minutes. Subsequently, DMF solution (0.5 mL) of 2 was added, followed by stirring at room temperature for 6 hours. Subsequently, the reaction solution was stirred at 80°C for 12 hours. After the reaction solution was cooled to room temperature, the solvent was distilled off under reduced pressure. The resulting residue was diluted with chloroform, and the organic phase was washed with water and saturated saline water, then dried over sodium sulfate. Solids were removed by filtration, and the solvent was distilled off under reduced pressure. The resulting residue was purified by silica gel column chromatography (hexane/ethyl acetate = 2: 1 to 1:1, 1% Et₃N), giving a mixture (18 mg) containing a deprotected product. The resulting mixture (18 mg) was dissolved in mL of methanol, and sodium methoxide was added, followed by a reaction at room temperature for 1 hour. After the solvent was distilled off, the resulting residue was purified by silica gel column chromatography (chloroform/methanol = 50:1 to 10:1, 1% Et₃N), giving the compound YMSA-0974 (8.4 mg, 17% for 2 steps) as a colorless solid. ¹H NMR (400 MHz, CD₃OD/TMS): δ = 8.40 (d, J = 8.8 Hz, 1H), 8.01 (d, J = 2.4 Hz, 1H), 7.85-7.87 (m, 1H), 7.80 (d, J = 8.4 Hz, 1H), 7.74 (d, J = 2.4 Hz, 1H), 7.64-7.66 (m, 1H), 4.92 (d, J = 8.0 Hz, 1H), 4.05 (d, J = 17.2 Hz, 1H), 3.85 (dd, J = 9.8, 7.4 Hz, 1H), 3.56-3.65 (m, 3H), 1.23 (d, J = 6.4 Hz, 3H), 1.09 (d, J = 6.0 Hz, 12H) ppm; HRMS (ESI positive): calcd for [M+H]⁺, C₂₇H₃₆ClN₄O₅: 531.2369; found 531.2369.

### YMSA-0973

Acetic anhydride (150 µL, 1.59 mmol) and dimethylaminopyridine (1.3 mg, 1.1 µmol) were added to YMSA-0974 (5.8 mg, 0.011 mmol) dissolved in 0.15 mL of pyridine, followed by stirring at room temperature for 24 hours. After confirming the completion of the reaction, the solvent was distilled off under reduced pressure. The resulting residue was purified by silica gel column chromatography (hexane:ethyl acetate = 2:1, 1% Et₃N), giving the compound YMSA-0973 (7.0 mg, 0.011 mmol, 98%) as a colorless solid. ¹H NMR (300 MHz, CDCl₃/TMS): δ = 8.78 (s, 1H), 8.02 (d, J = 2.7 Hz, 1H), 7.92-7.95 (m, 2H), 7.80-7.85 (m, 1H), 7.73 (m, 1H), 7.56-7.62 (m, 1H), 7.48 (brs, 1H), 5.51 (dd, J= 10.5, 7.8 Hz, 1H), 5.25 (d, J = 2.7 Hz, 1H), 5.11-5.16 (m, 2H), 4.02 (d, J = 18.3 Hz, 1H), 3.68-3.76 (m, 2H), 2.22 (s, 3H), 2.16 (s, 3H), 2.03 (s, 3H), 1.16 (d, J = 6.3 Hz, 3H), 1.05 (d, J = 6.3 Hz, 6H), 1.03 (d, J = 6.0 Hz, 6H) ppm; HRMS (ESI positive): calcd for [M+H]⁺, C₃₃H₄₂³⁵ClN₄O₈: 657.2686; found 657.2685.

### [Example 39] Synthesis of YMSA-1012

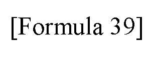

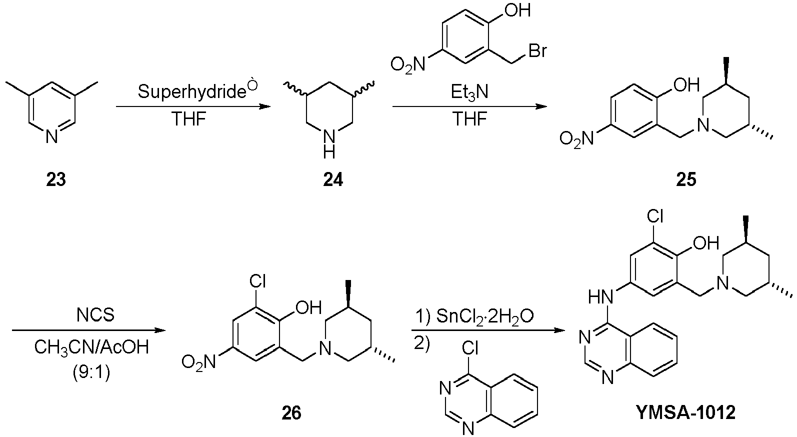

### 2-((trans-3,5-Dimethylpiperidin-1-yl)methyl)-4-nitrophenol (25)

Superhydride^{™} in THF (1M solution, 60 mL, 60 mmol) was added to a solution of 3,5-dimethylpyridine 23 (2.3 mL, 20 mmol) dissolved in 20 mL of tetrahydrofuran, followed by stirring at room temperature for 14 hours. After confirming the completion of the reaction, methanol was added until bubbling no longer occurred. The resulting mixture was diluted with diethyl ether, and 1N hydrochloric acid was added, followed by extraction. An aqueous layer was neutralized with 3N sodium hydroxide aqueous solution, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure, giving 2.72 g of a mixture of 24. 1.35 g of the resulting mixture was dissolved in 10 mL of tetrahydrofuran, and triethylamine (2.8 mL, 20 mmol) and 2-hydroxy-5-nitrobenzyl bromide (1.15 g, 5 mmol) were added, followed by stirring under reflux conditions for 11 hours. After confirming the completion of the reaction, the solvent was distilled off, and the residue was diluted with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was diluted with diethyl ether, dioxane hydrochloride solution was added until precipitates were generated, and then the solution was cooled with ice. The resulting precipitates were washed with diethyl ether, and the solvent was distilled off under reduced pressure. The resulting white solid was dissolved in water, and purified by HPLC (mobile phase: 0.1% TFA aqueous solution/0.1% TFA acetonitrile solution = 85/15). After lyophilization, 25 (34.2 mg, 1%) and its cis form (29.5 mg, 1%) were obtained as white solids. ¹H NMR (300 MHz, CDCl₃/TMS): 8.09 (dd, J = 3.0, 9.0Hz, 1H), 7.92 (d, J = 3.0 Hz, 1H), 6.83 (d, J = 9.0 Hz, 1H), 3.77 (s, 2H), 2.90 (d, J = 8.4 Hz, 2H), 1.81-1.66 (m, 5H), 0.99 (brs, 1H), 0.89 (d, J = 5.7 Hz, 6H) ppm.

### 2-Chloro-6-((trans-3,5 -dimethylpiperidin-1-yl)methyl)-4-nitrophenol (26)

N-chlorosuccinimide (20.8 mg, 0.16 mmol) was added to a solution of Compound 25 (34.2 mg, 0.13 mmol) dissolved in 1.8 mL of acetonitrile and 0.2 mL of acetic acid, followed by stirring at room temperature for 5.5 hours. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 26 (21.3 mg, 55%) as a yellow solid. ¹H NMR (300 MHz, CDCl₃/TMS): δ= 8.23 (d, J = 2.7 Hz, 1H), 7.83 (d, J = 2.7 Hz, 1H), 3.88 (d, J = 14.4 Hz, 1H), 3.69 (d, J = 14.4 Hz, 1H), 2.10-2.04 (m, 5H), 1.03 (br, 9H) ppm.

### YMSA-1012

Tin chloride dihydrate (64 mg, 0.29 mmol) was added to a solution of Compound 26 (21.3 mg, 0.07 mmol) dissolved in 2 mL of ethanol, followed by stirring under reflux conditions for 1.5 hours. After the reaction solution was cooled to room temperature, 4-chloroquinazoline (23 mg, 0.14 mmol) was added to the reaction solution, which was again stirred under reflux conditions for 1.5 hours. After confirming the completion of the reaction, the solvent was distilled off under reduced pressure. Thereafter, 2N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating the compound YMSA-1012 (26.1 mg, 93%) as a light yellow solid. M.p. 89-90°C. IR(ATR): v = 2924, 2162, 1620, 1572, 1533, 1499, 1468, 1394, 1357, 1235, 1122, 1067, 1027, 995, 960, 917, 868, 820, 765, 727, 679, 590, 557, 537, 498, 466, 433, 416 cm⁻¹. ¹H NMR (300 MHz, CDCl₃/TMS): δ= 8.74 (s, 1H), 7.93-7.90 (m, 1H), 7.84-7.81 (m, 2H), 7.60-7.56 (m, 2H), 7.30-7.23 (m, 2H), 3.80 (d, J = 13.8 Hz, 1H), 3.57 (d, J = 3.57, 1H), 2.05 (br, 4H), 1.37-1.26 (m, 4H), 1.00 (brs, 6H) ppm.¹³CNMR (100MHz, CDCl₃/TMS): δ= 166.8, 157.7, 154.6, 150.1, 149.5, 133.0, 130.5, 127.8, 126.2, 123.0, 122.9, 122.8, 122.0, 118.5, 60.8, 59.3, 48.6, 37.8, 27.2, 18.7 ppm. HRMS (ESI⁺): calcd for [M+H]⁺, C₂₂H₂₆ClN₄O: 397.1795; found 397.1790.

### [Example 40] Synthesis of YMSA-1015

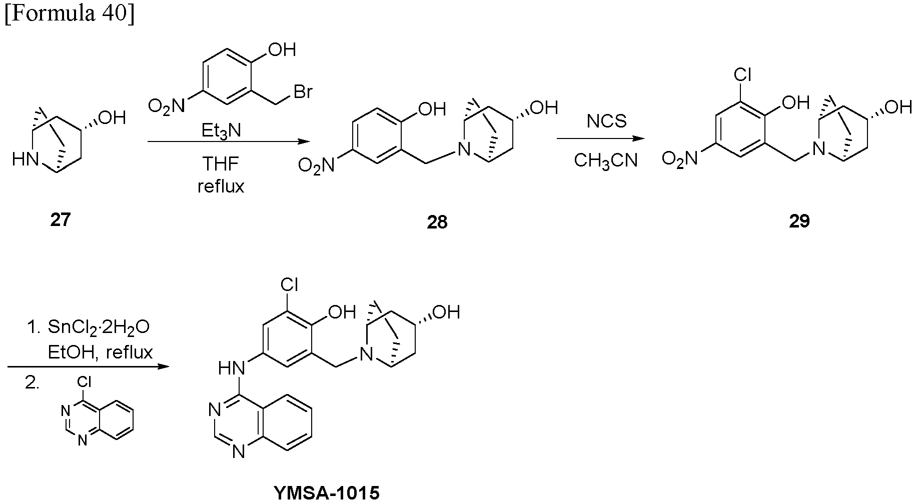

### 3-(2-Hydroxy-5-nitrobenzyl)-3-azabicyclo[3.2.1]octan-8-ol (28)

27 (152 mg, 1.2 mmol) and triethylamine (0.31 mL, 2.2 mmol) were added to a solution of 2-hydroxy-5-nitrobenzyl bromide (0.23 g, 1.0 mmol) dissolved in 4 mL of tetrahydrofuran, followed by stirring under reflux conditions for 3 hours. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate:methanol = 4:1), thus isolating Compound 28 (0.26 g, 94%) as a yellow solid. ¹H NMR (300 MHz, CDCl₃/TMS): δ = 8.10 (dd, J = 8.7, 2.7, 1H), 7.90 (s, 1H), 6.82 (d, J = 9.3 Hz, 1H), 4.16-4.09 (m, 1H), 3.77 (s, 2H), 3.31 (s, 2H), 2.30-2.26 (m, 2H), 2.18-2.06 (m, 4H), 1.88-1.83 (m, 2H).

### 3-(3-Chloro-2-hydroxy-5-nitrobenzyl)-3-azabicyclo[3.2.1]octan-8-ol (29)

N-chlorosuccinimide (151 mg, 1.1 mmol) was added to a solution of Compound 28 (260 mg, 0.94 mmol) dissolved in 9 mL of acetonitrile and 1 mL of acetic acid, followed by stirring at room temperature for 22 hours. The solvent was distilled off under reduced pressure, and a 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (ethyl acetate:methanol = 4:1), thus isolating Compound 29 (102 mg, 35%) as a yellow solid. ¹H NMR (300 MHz, DMSO-d₆/TMS): δ = 8.05-8.02 (m, 2H), 4.87 (s, 1H), 4.07 (s, 2H), 3.88 (s, 1H), 3.75 (s, 2H), 2.34-2.07 (m, 6H), 1.88-1.84 (m, 2H).

### YMSA-1015 (TTn-152)

Tin chloride dihydrate (0.18 g, 0.8 mmol) was added to a solution of Compound 29 (63 mg, 0.2 mmol) dissolved in 4 mL of ethanol, followed by stirring under reflux conditions for 1.5 hours. After the reaction solution was cooled to room temperature, 4-chloroquinazoline (66 mg, 0.4 mmol) was added to the reaction solution, which was again stirred under reflux conditions for 6 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate:methanol = 4:1), thus isolating the compound YMSA-1015 (95 mg, quant.) as a yellow solid. M.p. 60-62°C. IR(ATR): v = 2926, 1620, 1575, 1531, 1473, 1395, 1357, 1317, 1224, 1125, 1072, 1044, 967, 919, 867, 832, 749, 681, 584, 466, 431, 413 cm⁻¹. ¹H NMR (300 MHz, CDCl₃/TMS): δ = 8.74 (s, 1H), 7.93 (d, J = 7.8 Hz, 1H), 7.84-7.78 (m, 3H), 7.58-7.53 (m, 3H), 4.10 (s, 1H), 3.77 (s, 2H), 3.33 (s, 2H), 2.26-2.16 (m, 6H), 1.83-1.78 (m, 2H). ¹³CNMR (100MHz, CDCl₃/TMS): δ= 157.7, 154.6, 151.2, 149.5, 132.9,129.9, 127.7, 126.2, 123.2, 123.1, 122.8, 122.3, 118.7, 115.0, 79.2, 62.1(2C), 57.8(2C), 55.3(2C), 25.3(2C) ppm. HRMS (ESI⁺): calcd for [M+H]⁺, C₂₂H₂₄ClN₄O₂: 411.1588; found 411.1584.

### [Example 41] Synthesis of YMSA-1026

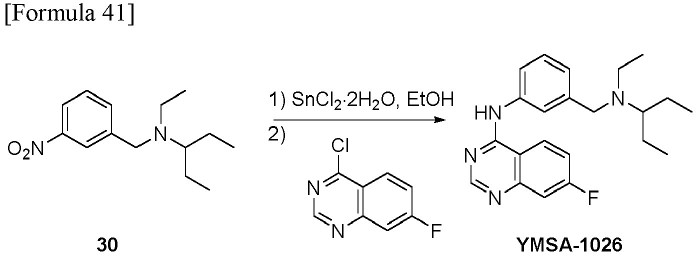

### YMSA-1026 (TTn-152)

Tin chloride dihydrate (0.12 g, 0.53 mmol) was added to a solution of Compound 30 (39.7 mg, 0.13 mmol) dissolved in 2 mL of ethanol, followed by stirring under reflux conditions for 1 hour. After the reaction solution was cooled to room temperature, 4-chloroquinazoline (47 mg, 0.26 mmol) was added to the reaction solution, which was again stirred under reflux conditions for 3 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating the compound YMSA-1026 (46.1 mg, 89%) as a yellow solid. ¹H NMR (300 MHz, CDCl₃/TMS): δ = 8.70 (s, 1H), 7.84 (dd, J = 5.7, 9.0 Hz, 1H), 7.56-7.52 (m, 2H), 7.34-7.21 (m, 2H), 3.87 (s, 2H), 2.62-2.51 (m, 3H), 1.68-1.59 (m, 3H), 1.49-1.39 (m, 3H), 1.15 (t, J = 6.9 Hz, 3H), 1.00 (t, J = 7.5 Hz, 6H) ppm.

### [Example 42] Synthesis of YMSA-1062

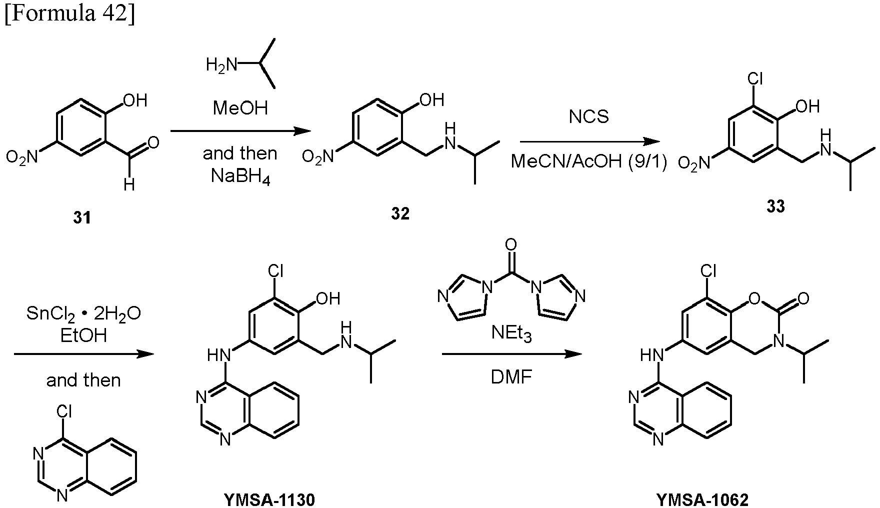

### 2-((Isopropylamino)methyl)-4-nitrophenol (32)

Compound 31 (0.84 g, 5.00 mmol) was dissolved in 25 mL of methanol, and isopropylamine (0.51 mL, 5.95 mmol) was added, followed by stirring at room temperature for 1 hour. Under cooling with ice, sodium borohydride (0.23 g, 6.04 mmol) was added to the reaction solution, which was further stirred for 1 hour. After confirming the completion of the reaction, the solvent was distilled off under reduced pressure. The resulting mixture was purified by silica gel column chromatography (ethyl acetate:methanol = 5:1), thus isolating Compound 32 (1.00 g, 95%) as a yellow solid. M.p. 182.5-184.4°C. IR(ATR): v = 2948, 2718, 2662, 2545, 2491, 1590, 1623, 1590, 1564, 1477, 1443, 1432, 1375, 1255, 1168, 1149, 1123, 1089, 994, 938, 919, 898, 841, 821, 775, 761, 733, 645, 611, 558, 540, 497, 477, 456, 422 cm⁻¹. ¹H NMR (300 MHz, CDCl₃/TMS): δ = 8.07 (dd, J = 8.9, 3.0 Hz, 1H), 7.94 (d, J = 3.0, 1H), 6.84 (d, J = 9.0, 1H), 4.10 (s, 2H), 2.96-2.86 (m, 1H), 1.18 (d, J = 6.0, 6H) ppm. ¹³C NMR (100 MHz, CDCl₃/TMS): δ = 165.4, 140.0, 125.3, 124.3, 122.6, 116.9, 49.7, 48.5, 22.3 ppm. [M+H]⁺, C₁₀H₁₅N₂O₃: 211.1077; found 211.1083.

### 2-Chloro-6-((isopropylamino)methyl)-4-nitrophenol (33)

N-chlorosuccinimide (0.59 g, 4.46 mmol) was added to a solution of Compound 32 (0.85 g, 4.04 mmol) dissolved in 22.8 mL of acetonitrile and 2.53 mL of acetic acid, followed by stirring at room temperature for 2 days. After the solvent was distilled off under reduced pressure, methanol was added to the resulting mixture. The generated precipitates were separated by filtration, and washed with methanol. Thereafter, Compound 33 (0.26 mg, 26%) was isolated as a yellow solid. M.p. 222.0-224.0°C. IR (ATR): v = 3148, 2975, 2295, 1586, 1563, 1480, 1456, 1435, 1391, 1348, 1298, 1280, 1209, 1167, 1131, 1089, 11077, 1018, 1004, 963, 923, 906, 883, 858, 849, 826, 782, 760, 746, 719, 657, 581, 535, 472, 446 cm⁻¹. ¹H NMR (300 MHz, CD₃OD/TMS): δ = 8.18 (d, J = 3.0 Hz 1H), 8.06 (d, J = 3.0, 1H), 4.13 (s, 2H), 3.42-3.294 (m, 1H), 1.37 (d, J = 6.6, 6H) ppm. ¹³C NMR (100 MHz, DMSO/TMS): δ = 172.3, 128.5, 127.2, 126.4, 123.2, 120.4, 49.7, 45.9, 19.2 ppm. HRMS (ESI (Positive mode)): calcd for [M+H]⁺, C₁₀H₁₄³⁵ClN₂O₃: 245.0687; found 245.0686.

### YMSA-1130 (TY-3)

Tin chloride dihydrate (46 mg, 0.20 mmol) was added to a solution of Compound 33 (10 mg, 0.04 mmol) dissolved in 3 mL of ethanol, followed by stirring under reflux conditions for 4 hours. After the reaction solution was cooled to room temperature, 4-chloroquinazoline (14 mg, 0.08 mmol) was added to the reaction solution, which was again stirred under reflux conditions for 2 hours. After confirming the completion of the reaction, saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (chloroform:methanol = 5:1), thus isolating the compound YMSA-1130 (16 mg, quant.) as a yellow solid. M.p. 107.1-110.2°C. IR (ATR): v = 3184, 2975, 1621, 1601, 1576, 1530, 1498, 1465, 1393, 1367, 1321, 1287, 1252, 1230, 1154, 1136, 1125, 1078, 1014, 977, 948, 926, 906, 866, 850, 820, 808, 756, 746, 675, 592, 551, 516, 485, 471, 427 cm⁻¹. ¹H NMR (300 MHz, CDCl₃/TMS): δ = 8.73 (s, 1H), 7.93-7.78 (m, 3H), 7.58-7.53 (m, 2H), 7.62-7.54 (m, 2H), 7.35 (d, J= 2.4 Hz 1H), 4.06 (s, 2H), 2.99-2.93 (m, 1H), 2.01(s br, 1H), 1.21 (d, 6H) ppm. ¹³C NMR (100 MHz, CHCl₃/TMS): δ = 158.0, 155.0, 152.1, 149.9, 133.1, 129.2, 128.8, 126.7, 124.1, 123.9, 121.9, 121.2, 115.0, 50.0, 48.6, 22.3 ppm. HRMS (ESI (Positive mode)): calcd for [M+H]⁺, C₁₈H₂₀³⁵ClN₄O: 343.1320; found 343.1420.

### YMSA-1062 (TY-4)

Carbonyldiimidazole (0.25 g, 0.15 mmol) and triethylamine (25.3 µL, 0.18 mmol) were added to YMSA-1130 (0.510 g, 0.15 mmol) dissolved in 1 mL of dimethylformamide, followed by stirring at 80°C for 20 hours. After confirming the completion of the reaction, water was added, followed by extraction with diethyl ether. The organic phase was washed with saturated saline water, and dried over sodium sulfate. Solids were removed by filtration, and the solvent was distilled off under reduced pressure. The resulting mixture was purified by silica gel column chromatography (chloroform:methanol = 10:1), thus isolating the compound YMSA-1062 (12.4 mg, 23%) as a yellow solid. M.p. 261.2-269.1°C. IR (ATR): v = 3388, 3119, 2968, 1698, 1625, 1609, 1569, 1533, 1489, 1470, 1438, 1416, 1394, 1372, 1353, 1328, 1310, 1280, 1251, 1227, 1210, 1192, 1141, 1132, 1073, 1047, 990, 968, 917, 896, 874, 864, 820, 792, 765, 737, 705, 677, 662, 615, 596, 583, 539, 511, 502, 482, 468, 438, 403 cm⁻¹. ¹H NMR (300 MHz, CDCl₃/TMS): δ = 8.80 (s, 1H), 7.98-7.95 (m, 1H), 7.88-7.80 (m, 2H), 7.64-7.56 (m, 2H), 7.36 (s, 1H), 4.73-4.68 (m, 1H), 4.42 (s, 2H), 1.30 (d, J = 6.7 Hz 6H) ppm. ¹³C NMR (100 MHz, CHCl₃/TMS): δ = 157.7, 154.9, 150.3, 150.0, 142.6, 135.0, 133.5, 129.4, 127.3, 123.0, 121.6, 120.6, 119.9, 117.9, 115.3, 48.4, 41.4, 19.3 ppm. HRMS (ESI (Positive mode)): calcd for [M+H]⁺, C₁₉H₁₈^{3s}ClN₄O₂: 369.1113; found 369.1109.

### [Example 43] Synthesis of YMSA-1055

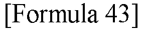

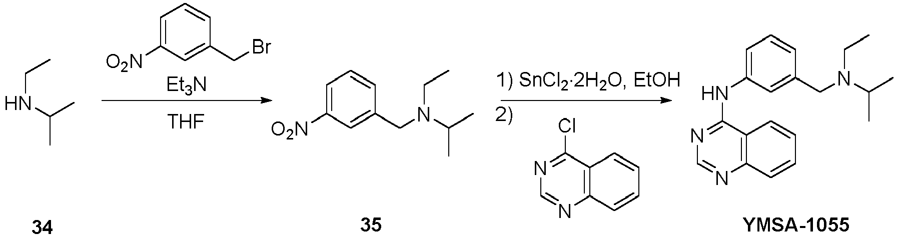

### N-ethyl-N-(3-nitrobenzyl)propan-2-amine (35)

Compound 34 (145 µL, 1.2 mmol) and triethylamine (306 µL, 2.2 mmol) were added to a solution of 3-nitrobenzyl bromide (216 mg, 1.0 mmol) dissolved in 2 mL of tetrahydrofuran, followed by stirring under reflux conditions for 1 hour. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating Compound 35 (120 mg, 54%) as a light yellow oil.

### YMSA-1055

Tin chloride dihydrate (0.49 g, 2.15 mmol) was added to a solution of Compound 35(120 mg, 0.54 mmol) dissolved in 5 mL of ethanol, followed by stirring under reflux conditions for 2 hours. After the reaction solution was cooled to room temperature, 4-chloroquinazoline (178 mg, 1.1 mmol) was added to the reaction solution, which was again stirred under reflux conditions for 2 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 1:1), thus isolating the compound YMSA-1055 (135 mg, 78%) as a yellow solid. ¹H NMR (300 MHz, CDCl₃/TMS): δ = 8.78 (s, 1H), 7.91 (t, J = 9.6 Hz, 2H), 7.84-7.76 (m, 2H), 7.61-7.55 (m, 2H), 7.44 (s, 1H), 7.36 (t, J = 7.5 Hz, 1H), 7.16 (d, J = 8.1 Hz, 1H), 3.60 (s, 1H), 3.03 (sept, J = 6.6 Hz, 1H), 2.51 (q, J = 6.6 Hz, 2H), 1.06-1.00 (m, 9H).¹³CNMR (100MHz, CDCl₃/TMS): δ= 158.0, 154.7, 149.9, 142.1, 139.1, 133.1, 128.2, 128.0, 126.4, 123.8, 123.2, 122.3, 121.0, 115.4, 53.1, 49.2, 43.3, 18.1, 14.2 ppm.

### [Example 44] Synthesis of YMSA-1066

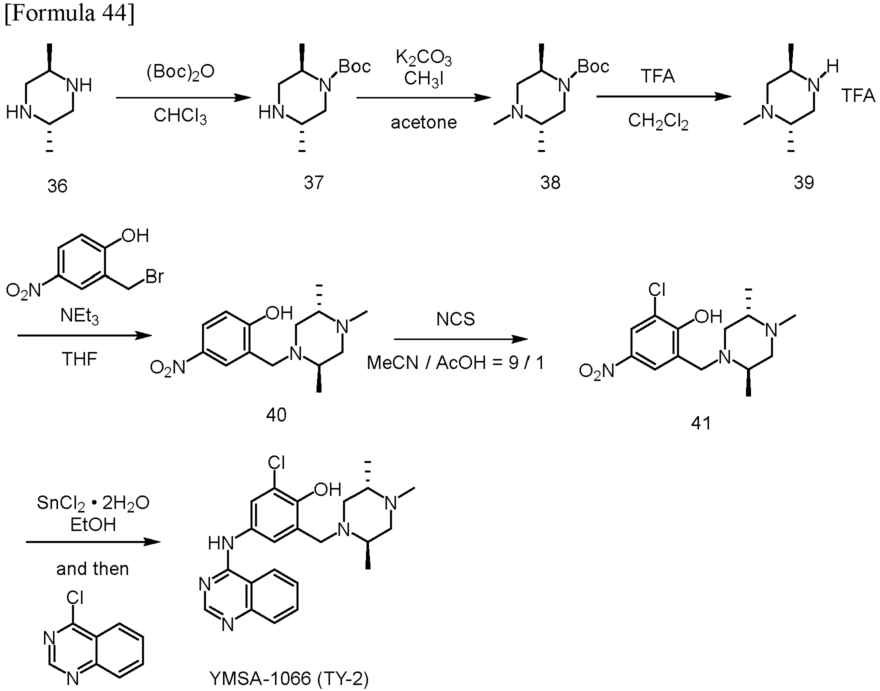

tert-Butyl (±)-2,5-dimethylpiperazine-1-carboxylate (37)

(Boc)₂O (0.53 g, 2.4 mmol) solution in chloroform (200 mL) was added dropwise to Compound 36 (0.50 g, 4.5 mmol) solution in chloroform (250 mL) under cooling with ice, followed by stirring for 23 hours. After confirming the completion of the reaction, water was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (chloroform:methanol =25:1 to 3:1), thus isolating Compound 37 (0.32 g, 62%) as a yellow solid. IR (ATR): v = 3333, 2972, 2932, 1679, 1456, 1414, 1364, 1339, 1317, 1247, 1149, 1119, 1099, 1059, 1037, 936, 866, 815, 752, 665, 592, 522, 455, 408 cm⁻¹. ¹H NMR (300 MHz, CDCl₃/TMS): δ = 4.16-4.06 (m, 1H), 3.55 (dd, J = 15, 2.4 Hz, 1H), 3.24-3.08 (m, 3H), 2.48 (dd, J = 13, 3, 1H), 1.46 (s, 9H), 1.21 (d, J = 6.6, 3H), 1.17 (d, J = 6.9, 3H) ppm. ¹³C NMR(100 MHz, CDCl₃/TMS): δ = 155.4, 79.3, 47.2, 46.8, 44.0, 43.5, 28.4, 17.1, 15.3 ppm. HRMS (ESI (Positive mode)): calcd for [M+H]⁺, C₁₁H₂₃N₂O₂: 215.1753; found 215.1754.

### tert-Butyl (±)-2,4,5-trimethylpiperazine-1-carboxylate (38)

Compound 37 (0.20 g, 0.93 mmol) was dissolved in 15. 5 mL of acetone, and methyl iodide (0.11 mL, 1.8 mmol) and potassium carbonate (0.14 mg, 1.0 mmol) were added, followed by stirring at room temperature for 1 hour. After confirming the completion of the reaction, saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 4:1), thus isolating Compound 38 (0.15 g, 73%) as a yellow oil. ¹H NMR (300 MHz, CDCl₃/TMS): δ = 4.22-4.18 (m, 1H), 8.10-8.04 (m, 1H), 3.63 (dd, J = 13, 1.2, 1H), 3.23 (dd, J = 13, 3.6, 1H), 2.83-2.79 (m, 1H), 2.71-2.63 (m, 1H), 2.27 (s, 3H), 2.22-2.18 (m, 1H), 1.46 (s, 9H), 1.25 (d, J = 6.9, 3H), 0.93 (d, J = 6.6, 3H) ppm. ¹³C NMR (100 MHz, CDCl₃/TMS): δ = 155.4, 79.2, 53.4, 52.1, 46.8, 44.4, 42.8, 28.4, 16.4, 7.38 ppm. HRMS (ESI (Positive mode)): calcd for [M+H]⁺, C₁₂H₂₅N₂O₂: 229.1911; found 229.1911.

### (±)-1,2,5 -Trimethylpiperazine (39)

Compound 38 (0.12 g, 0.52 mmol) was dissolved in 6 mL of dichloromethane, trifluoroacetic acid (6 mL) was added, followed by stirring at room temperature for 1 hour. After confirming the completion of the reaction, the solvent was distilled off, thus isolating Compound 39 (0.28 g, quant.) as a colorless solid. ¹H NMR (300 MHz, CD₃OD/TMS): δ = 3.79-3.66 (m, 3H), 3.54-3.44 (m, 1H), 3.28-3.11 (m, 1H), 2.97 (s, 3H), 1.45 (d, 6.3, 1H), 1.40 (d, 6.3, 3H) ppm. HRMS (ESI (Positive mode)): calcd for [M+H]⁺, C₇H₁₇N₂: 129.1386; found 129.1386.

### 4-Nitro-2-(((±)-2,4,5-trimethylpiperazin-1-yl)methyl)phenol (40)

39 (0.20 g, 0.84 mmol) and triethylamine (240 µL, 1.68 mmol) were added to a solution of 2-hydroxy-5-nitrobenzyl bromide (0.13 g, 0.56 mmol) dissolved in 1 mL of tetrahydrofuran, followed by stirring under reflux conditions for 1 hour. After confirming the completion of the reaction, the solvent was distilled off under reduced pressure, and saturated sodium hydrogen carbonate aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate), thus isolating Compound 40 (81.6 mg, 52%) as a yellow solid. ¹H NMR (300 MHz, CDCl₃/TMS): δ = 8.09 (dd, J = 9.0, 2.7 Hz, 1H), 7.93 (d, J = 2.7, 1H), 6.83 (d, J = 8.7, 1H), 4.43 (d, J = 15, 1H), 3.33 (d, J = 14, 1H), 2.83 (dd, J= 12, 2.7, 1H), 2.74 (dd, J = 11, 1.8, 1H) 2.65-2.61 (m, 1H), 2.28 (s, 3H), 2.15-2.03 (m, 3H), 1.21 (d, J = 6.3, 3H), 1.01 (d, J = 6.0, 3H) ppm. HRMS (ESI (Positive mode)): calcd for [M+H]⁺, C₁₄H₂₂N₃O₃: 280.1656; found 280.1659.

### 2-Chloro-4-nitro-6-(((±)-2,4,5-trimethylpiperazin-1-yl)methyl)phenol (41)

N-chlorosuccinimide (62 mg, 0.46 mmol) was added to a solution of Compound 40 (59 mg, 0.21 mmol) dissolved in 2.07 mL of acetonitrile and 0.23 mL of acetic acid, followed by stirring at room temperature for 6 days. The solvent was distilled off under reduced pressure, and 10% sodium thiosulfate aqueous solution was added, followed by extraction with ethyl acetate. The organic phase was washed with saturated sodium hydrogen carbonate aqueous solution and saturated saline water, and dried over sodium sulfate. Solids were removed by filtration, and the solvent was distilled off under reduced pressure. The resulting mixture was purified by silica gel column chromatography (hexane:ethyl acetate = 10:1 to 4:1), thus isolating Compound 41 (14.8 mg, 22%) as a yellow solid. ¹H NMR (300 MHz, CDCl₃/TMS): δ = 8.22 (d, J = 2.7 Hz 1H), 7.85 (d, J = 2.4, 1H), 4.46 (d, J= 15, 1H), 3.42 (d, J = 14, 1H), 2.86 (dd, J = 12, 3.3, 1H), 2.80-2.73 (m, 2H), 2.29 (s, 3H), 2.24-2.07 (m, 3H), 1.24 (d, J = 6.3, 3H), 1.04 (d, J = 6.0, 3H) ppm. HRMS (ESI (Positive mode)): calcd for [M+H]⁺, C₁₄H₂₁³⁵ClN₃O₃: 314.1266; found 314.1267.

### YMSA-1066 (TY-2)

Tin chloride dihydrate (27 mg, 0.12 mmol) was added to a solution of Compound 41 (12 mg, 0.04 mmol) dissolved in 2 mL of ethanol, followed by stirring under reflux conditions for 1.5 hours. After the reaction solution was cooled to room temperature, 4-chloroquinazoline (7.4 mg, 0.05 mmol) was added to the reaction solution, which was again stirred under reflux conditions for 3 hours. After confirming the completion of the reaction, the solvent was distilled off under reduced pressure, and 1N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting mixture was purified by silica gel column chromatography (ethyl acetate:methanol = 10:1 to 5:1), thus isolating the compound YMSA-1066 (4.7 mg, 29%) as a yellow solid. ¹H NMR (300 MHz, CDCl₃/TMS): δ = 8.75 (s, 1H), 7.93 (d, J = 2.1 Hz, 1H), 7.84-7.79 (m, 2H), 7.62-7.54 (m, 2H), 7.31 (d, J = 2.7, 1H), 4.47 (d, J = 14, 1H), 3.26 (d, J = 14, 1H), 2.87-2.83 (m, 2H), 2.64 (s br, 1H), 2.28-2.04 (m, 6H), 1.23 (d, J = 6.0, 3H), 1.03 (d, J = 6.3, 3H) ppm. HRMS (ESI (Positive mode)): calcd for [M+H]⁺, C₂₂H₂₇³⁵ClN₅O: 412.1899; found 412.1900.

### [Example 45] Synthesis of YMSA-1111

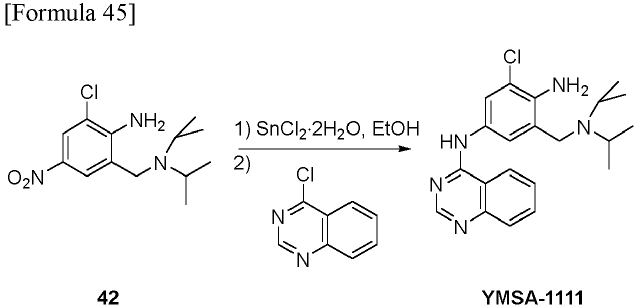

### YMSA-1111

Tin chloride dihydrate (31 mg, 0.14 mmol) was added to a solution of Compound 42 (11.5 mg, 0.035 mmol) dissolved in 1 mL of ethanol, followed by stirring under reflux conditions for 1 hour. After the reaction solution was cooled to room temperature, 4-chloroquinazoline (12 mg, 0.07 mmol) was added to the reaction solution, which was again stirred under reflux conditions for 1.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (ethyl acetate), thus isolating the compound YMSA-1111 (15.4 mg, quant) as a light yellow solid. ¹H NMR (300 MHz, DMSO-d₆/TMS): δ = 9.60 (s, 1H), 8.50 (s, 1H), 8.46 (d, J= 7.8 Hz, 1H), 7.84-7.79 (m, 1H), 7.74-7.72 (m, 2H), 7.61-7.56 (m, 1H), 7.40 (d, J = 2.4 Hz, 1H), 5.52 (s, 2H), 3.67 (s, 2H), 3.01 (sept, J = 6.6 Hz, 2H), 1.03 (d, J = 6.6 Hz, 12H), ¹³CNMR (100MHz, CDCl₃/TMS): δ= 157.9, 155.2, 149.9, 141.5, 132.8, 128.9, 127.6, 126.4, 125.2, 124.0, 122.9, 120.3, 119.2, 115.0, 49.2, 47.1, 20.1 ppm. HRMS (FAB⁺): calcd for [M+H]⁺, C₂₁H₂₇ClN₅: 384.1955; found 384.1949.

### [Example 46] Synthesis of YMSA-1112

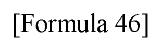

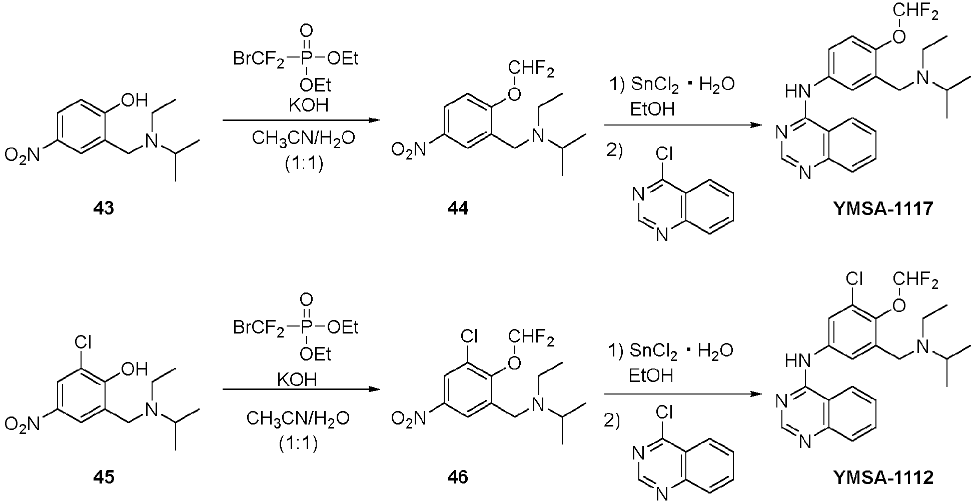

### N-(2-(Difluoromethoxy)-5-nitrobenzyl)-N-ethyl-N-propan-2-amine (44)

Under -78°C, potassium hydroxide (2.1 g, 3.79 mmol) and BrCF₂PO(OEt)₂ were added to a solution of Compound 43 (451 mg, 1.89 mmol) dissolved in 10 mL of 50% acetonitrile aqueous solution (1¹H NMR (300 MHz, CDCl₃/TMS): δ= 8.57 (d, J = 3.0 Hz, 1H), 8.10 (dd, J = 3.0, 9.0 Hz, 1H), 6.66 (t, J = 72.6 Hz, 1H), 3.64 (s, 2H), 2.99 (sept, J = 6.6 Hz, 1H), 2.53 (q, J = 6.9 Hz, 2H), 1.06-0.98 (m, 9H) ppm.

### YMSA-1117

Tin chloride dihydrate (181 mg, 0.8 mmol) was added to a solution of Compound 44 (58 mg, 0.2 mmol) dissolved in 3 mL of ethanol, followed by stirring under reflux conditions for 1 hour. After the reaction solution was cooled to room temperature, 4-chloroquinazoline (66 mg, 0.4 mmol) was added to the reaction solution, which was again stirred under reflux conditions for 2.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (ethyl acetate), thus isolating the compound YMSA-1117 (31 mg, 41%) as a colorless solid. ¹H NMR (300 MHz, CDCl₃/TMS): δ= 8.76 (s, 1H), 7.92 (t, J = 8.4 Hz, 1H), 7.89-7.76 (m, 3H), 7.62-7.57 (m, 1H), 7.15 (d, J = 8.7 Hz, 1H), 6.58 (t, J = 74.7 Hz, 1H), 3.64 (s, 2H), 3.02 (sept, J = 6.6 Hz, 1H), 2.54 (q, J = 7.5 Hz, 2H), 1.07-1.01 (m, 9H) ppm. ¹³CNMR (100MHz, CDCl₃/TMS): δ= 154.9, 132.9, 129.1, 126.7, 123.9, 121.3, 120.5, 120.3, 50.0, 47.5, 44.0, 18.1, 13.9 ppm. HRMS (ESI⁺): calcd for [M+H]⁺, C₂₁H₂₅F₂N₄O: 387.1996; found 387.1984.

### N-(3-Chloro-2-(difluoromethoxy)-5-nitrobenzyl)-N-ethyl-N-propan-2-amine (46)

Under -78°C, potassium hydroxide (1.1 g, 20 mmol) and BrCF2 PO(OEt)₂ (356 µL, 2 mmol) were added to a solution of Compound 45 (273 mg, 1 mmol) dissolved in 10 mL of 50% acetonitrile aqueous solution, followed by stirring at room temperature for 1 hour. After confirming the completion of the reaction, H₂O was added to the reaction mixture, which was extracted with ethyl acetate. The organic phase was washed with saturated saline water, and dried over sodium sulfate. Solids were removed by filtration, and the solvent was distilled off under reduced pressure. The resulting mixture was purified by silica gel column chromatography (hexane:ethyl acetate = 10:1), thus isolating Compound 46 (129 mg, 40%) as a colorless oil. ¹H NMR (300 MHz, CDCl₃/TMS): δ= 8.54 (d, J = 2.7 Hz, 1H), 8.19 (d, J = 2.7 Hz, 1H), 6.70 (t, J = 74.1 Hz, 1H), 3.71 (s, 2H), 2.97 (sept, J = 6.6 Hz, 1H), 2.52 (q, J = 6.9 Hz, 2H), 1.05-0.98 (m, 9H) ppm.

### YMSA-1112

Tin chloride dihydrate (362 mg, 1.6 mmol) was added to a solution of Compound 46 (129 mg, 0.40 mmol) dissolved in 6 mL of ethanol, followed by stirring under reflux conditions for 1.5 hours. After the reaction solution was cooled to room temperature, 4-chloroquinazoline (132 mg, 0.8 mmol) was added to the reaction solution, which was again stirred under reflux conditions for 1.5 hours. After confirming the completion of the reaction, the solvent was distilled off under reduced pressure, 3N sodium hydroxide aqueous solution was added, followed by extraction with ethyl acetate. The organic phase was washed with saturated saline water, and dried over sodium sulfate. Solids were removed by filtration, and the solvent was distilled off under reduced pressure. The resulting residue was purified by silica gel column chromatography (ethyl acetate), thus isolating the compound YMSA-1112 (150 mg, 89%) as a light yellow solid. IR(ATR): v =3112, 2970, 1626, 1609, 1574, 1535, 1503, 1462, 1418, 1381, 1354, 1322, 1207, 1179, 1102, 1050, 927, 915, 880, 865, 841, 819, 794, 761, 720, 676, 635, 609, 573, 531, 509, 486, 458, 433, 406 ppm. ¹H NMR (300 MHz, CDCl₃/TMS): δ= 8.81 (s, 1H), 8.19 (d, J = 2.7 Hz, 1H), 7.98-7.82 (m, 3H), 7.70 (d, J = 2.4 Hz, 1H), 7.64-7.58 (m, 1H), 7.47 (s, 1H), 6.64 (t, J = 75.3 Hz, 1H), 3.70 (s, 2H), 3.00 (sept, J = 6.6 Hz, 1H), 2.54 (q, J = 6.9 Hz, 2H), 1.07-1.01 (m, 9H) ppm. ¹³CNMR (100MHz, CDCl₃/TMS): δ= 157.6, 154.2, 149.7, 140.2, 138.0, 137.3, 133.2, 127.9, 126.4, 126.0, 123.1, 122.1, 121.4, 117.9, 115.1, 49.8, 47.3, 43.8, 17.9(2C), 13.9(2C) ppm. HRMS (ESI⁺): calcd for [M+H]⁺, C₂₁H₂₄ClF₂N₄O: 421.1607; found 421.1600.

### [Example 47] Synthesis of YMSA-1033

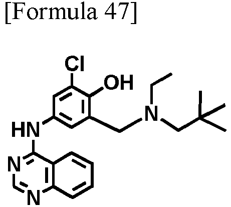

YMSA-1033 was obtained by the same method as that in Example 21.

All the publications, patents, and patent applications cited in this specification are incorporated herein in their entirety by reference.

## Claims

1. A compound represented by the following formula (I), a salt thereof, a solvate thereof, or a prodrug thereof 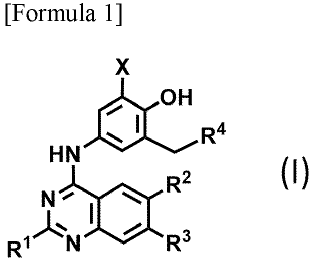 wherein R¹, R², and R³ are the same or different, and each of them is a hydrogen atom, a halogen atom, a substituted or unsubstituted C₁₋₆-alkyl group, a substituted or unsubstituted C₁₋₆-alkoxy group, or -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
R⁴ is -N(R^{a})(R^{b}) (wherein R^{a} and R^{b} are the same or different, and each of them is a substituted or unsubstituted C₁₋₁₀-alkyl group or a substituted or unsubstituted aryl group; or R^{a} and R^{b}, together with an adjacent nitrogen atom, may form a substituted or unsubstituted 5 to 7-membered ring),
X is a hydrogen atom, a fluorine atom, a chlorine atom, a bromine atom, or an iodine atom, and
the hydroxyl group in the formula may be substituted with a protecting group.

2. The compound, a salt thereof, a solvate thereof, or a prodrug thereof according to claim 1, wherein in the formula (I), R⁴ is an ethyl (isopropyl)amino group, a diisopropylamino group, a cyclohexyl(ethyl)amino group, an ethyl(pentane-3-yl)amino group, a tert-butyl(ethyl)amino group, or a 3,5-dimethylpiperidino group.

3. The compound, a salt thereof, a solvate thereof, or a prodrug thereof according to claim 1 or 2, wherein in the formula (I), R¹, R², and R³ are the same or different, and each of them is a hydrogen atom, a halogen atom, a C₁₋₆-alkoxy group, or a dimethylamino group.

4. The compound, a salt thereof, a solvate thereof, or a prodrug thereof according to any one of claims 1 to 3, wherein in the formula (I), X is a hydrogen atom or a chlorine atom.

5. An anti-SARS-CoV-2 drug comprising the compound, a salt thereof, a solvate thereof, or a prodrug thereof according to any one of claims 1 to 4.
